# EUROPEAN PATENT SPECIFICATION

(11) **EP 4 085 057 B1**
(45) Date of publication and mention of the grant of the patent: **08.10.2025**
(21) Application number: 20841958.0
(22) Date of filing: 30.12.2020
(51) Int. Cl.: C07D 417/14, C07D 401/14, A61P 31/04, A61K 31/4439

(54) **PYRIDINE AND PYRIMIDINE DERIVATIVES USEFUL AS PQSR INVERSE AGONISTS**
PYRIDIN- UND PYRIMIDIN-DERIVATE ZUR VERWENDUNG ALS PQSR-INVERSE AGONISTEN
DÉRIVÉS DE PYRIDINE ET PYRIMIDINE UTILES EN TANT QU'AGONISTES INVERSES DE PQSR

(30) Priority: 02.01.2020 EP 20150119
(43) Date of publication of application: 09.11.2022
(73) Proprietor: Helmholtz-Zentrum für Infektionsforschung GmbH, 38124 Braunschweig (DE)
(72) Inventor: HAMED, Mostafa, 38124 Braunschweig (DE); AHMED, Ahmed S. A., 38124 Braunschweig (DE); EMPTING, Martin, 38124 Braunschweig (DE); SCHÜTZ, Christian, 38124 Braunschweig (DE); HARTMANN, Rolf W., 38124 Braunschweig (DE); RÖHRIG, Teresa, 38124 Braunschweig (DE); KANY, Andreas M., 38124 Braunschweig (DE); HIRSCH, Anna K. H., 38124 Braunschweig (DE)
(74) Representative: Forstmeyer, Dietmar
(86) International application number: PCT/EP2020/088029
(87) International publication number: WO 2021/136805

(56) References cited:
- WO-A1-2020/007938
- CN-A- 108 003 151
- XIN CHEN ET AL: "Quorum sensing inhibitors: a patent review (2014-2018)", EXPERT OPINION ON THERAPEUTIC PATENTS, vol. 28, no. 12, 31 October 2018 (2018-10-31), GB, pages 849 - 865, XP055694628, ISSN: 1354-3776, DOI: 10.1080/13543776.2018.1541174

## Description

### Field of the invention

The present invention relates to a compound according to general formula (I), which acts as an inverse agonist of PqsR (the currently only known receptor for the *Pseudomonas* Quinolone Signal (PQS), sometimes also referred to as MvfR); to a pharmaceutical composition containing one or more of the compound(s) of the invention; to a combination preparation containing at least one compound of the invention and at least one further active pharmaceutical ingredient; and to said compound(s) for use as a medicament, e.g. for use in the treatment or prophylaxis of a bacterial infection, especially a *Pseudomonas aeruginosa* or *Burkholderia* infection.

### Background of the invention

In view of the rapid decline in the effectiveness of antibiotics due to the emergence of resistance, there is a need for a constant supply of new compounds for effective treatment of infections. The development of antimicrobial resistance is mainly attributed to the overuse of antibiotics interfering with essential metabolic processes in bacteria. Moreover, bacteria living in biofilms, sessile cell communities embedded in a matrix of extracellular polymeric substances showing reduced metabolic activity and growth rate, exhibit up to 1000-fold higher resistance against antibiotics than free-living bacteria. Thus, novel therapeutic approaches that aim at reducing bacterial pathogenicity by interfering with bacterial virulence and biofilm formation instead of their metabolic activity are considered as highly favorable and are urgently needed.

The opportunistic pathogen *P. aeruginosa* causes severe and fatal infections including such of the urinary tract, of the gastrointestinal tract, of chronic and burnt wounds, of the eyes, of the ears, and of the lungs. Its pathogenicity is strongly related to the expression of virulence factors causing progressive tissue damage and biofilm formation hindering a successful drug therapy. The regulation of pathogenicity is based on a cell-density dependent intercellular communication system known as *quorum sensing* (QS).

*P. aeruginosa* uses as signal molecules *N*-acyl-L-homoserine lactones (AHLs) for the *las* and *rhl* QS systems and 2-alkyl-4-(1H)-quinolones (AQs) for the *pqs* QS system. The latter is restricted to *Pseudomonas* and *Burkholderia* species allowing for selective therapy with *pqs* QS inhibitors. While *Pseudomonas* and *Burkholderia* both produce 2-heptyl-4-hydroxyquinoline (HHQ), *Pseudomonas* uniquely uses the *Pseudomonas* quinolone signal (PQS) as signal molecule. PQS and its biosynthetic precursor HHQ serve as the natural ligands and agonists of the key DNA-binding receptor PqsR. This transcriptional regulator fine-tunes a large set of genes, notably such involved in the biosynthesis of HHQ and in the production of virulence factors such as pyocyanin and lectins. Regarding biofilms, the production of extracellular DNA (eDNA) and lectins, both main biofilm matrix components, is controlled by the *pqs* QS system. A *pqsR* mutant of *P. aeruginosa* is *pqs* QS-deficient, does not produce any pyocyanin or lectin A, shows reduced eDNA production, and displays reduced pathogenicity in mice.

To date, a number of compounds have been discovered that target QS in *P. aeruginosa.* The majority of these compounds has been reported to interfere with the AHL-based QS systems in *Pseudomonas* either via direct interaction with the receptors LasR [1, 2] or RhlR [3], at the post-transcriptional level [4-6], or at superior regulatory systems [7]. However, except an extract from *Allium sativum* (garlic), that exhibited no significant improvement of lung function in a clinical trial, these QS inhibitors have been only used in preclinical studies. Whereas AHL-mediated QS is widespread among Gram-negative bacteria, interference with *pqs* QS allows for selective therapy avoiding adverse effects on beneficial bacterial consortia present in the host. A few *pqs* QS inhibitors have been described acting as blockers of the signal molecule biosynthesis [8-16] or as antagonists of the receptor PqsR [17-21]. A QS inhibitor based on anthranilate structure, methyl anthranilate, was shown to inhibit PQS formation and the production of the virulence factor elastase at millimolar concentrations [12]. QS inhibitors targeting the enzyme PqsA were able to reduce the production of signal molecules HHQ and PQS (IC₅₀: ~100 µM for 6FABA) [14, 16, 22] and enhanced the survival rate of *Pseudomonas*-infected mice in a thermal injury mice model [14]. However, high concentrations were necessary to obtain an *in cellulo* or *in vivo* effect. Inhibitors of the enzyme PqsD were able to reduce the biovolume of a *P. aeruginosa* biofilm [15], however, did not exhibit any anti-virulence properties (no effect on virulence factor pyocyanin, no effect on the survival of *Pseudomonas*-infected *Galleria mellonella* larvae; unpublished data). Zender [18] and Klein [17] reported PqsR antagonists affecting the production of virulence factor pyocyanin, however with moderate potency (IC₅₀ values in the double-digit micromolar range). Furthermore, these compounds did not inhibit biofilm formation (unpublished data). Quinazoline-based PqsR antagonists developed in the group of Paul Williams [21] were reported to exhibit antivirulence activity. However, the most promising compound was only moderately active in reducing pyocyanin production (IC₅₀ ~ 50 µM in a less PQS- and pyocyanin-producing *P. aeruginosa* strain). A reduction in biomass of a *P. aeruginosa* biofilm by this compound was observed, however at an unknown concentration. Another potent antagonist of PqsR is 2-heptyl-6-nitro-4-oxo-1,4-dihydroquinoline-3-carboxamide and was developed in the group of Anke Steinbach and Rolf W. Hartmann[20]. 2-heptyl-6-nitro-4-oxo-1,4-dihydroquinoline-3-carboxamide is highly affine to PqsR (IC₅₀ = 35 nM in *E.coli* reporter gene assay, IC₅₀ = 400 nM in *P. aeruginosa* reporter gene assay), strongly reduces signal molecule production (HHQ production by 54% and PQS by 37% at 15 µM), and shows excellent anti-virulence potency *in cellulo* (inhibition of virulence factor pyocyanin production: IC₅₀ = 2 µM) and in *in vivo* animal infection models. Further reported PqsR-targeting compounds have been described by the working group of Laurence Rahme (DOI: 10.1371/journal.ppat.1004321; WO2012116010) and in follow-up patents by Spero Therapeutics (WO2014176258, WO2016040764, WO2016007837, WO20161 12088). These compound classes show activity against pyocyanin and alkylquinolone production in the nanomolar range. The compounds commonly referred to as M64 and SPR-00305 (205 in WO2016112088) suffer from pharmakokinetic drawbacks (*e.g*., low metabolic stability).

All in all, the hitherto known compounds show a number of deficiencies hampering their utility as a drug, including low metabolic stability, low bioavailability/solubility, low selectivity and toxicity.

In view of the deficits of the prior art compounds and the severe conditions associated with antibiotic resistant microorganisms, both acute and chronic, there is a need for novel anti-pathogenic compounds there is a need for novel anti-pathogenic compounds that use a different mechanism of action than "classical" antibiotics.

### Summary and description of the invention

The present invention was made in view of the prior art and the needs described above, and, therefore, the object of the present invention is to provide novel PqsR inverse agonsists according to general formula (I) exhibiting both anti-virulence and anti-biofilm activity. Due to their inverse agonistic activity on PqsR, the compounds of the invention permit an effective prophylaxis or treatment of bacterial infections, especially infections caused by *Pseudomonas aeruginosa* or *Burkholderia.* The PqsR inverse agonists of the invention preferably have one or more improved properties, e.g. biofilm inhibition (biovolume reduction and/or eDNA suppression), high antivirulence activity, an improved pharmacokinetic and/or physiochemical property, including bioavailability, solubility, and metabolic stability. Other objects of the present invention are to provide a pharmaceutical composition comprising at least one PqsR inverse agonist as described herein; a combination preparation containing at least one compound of the invention and at least one further active pharmaceutical ingredient; and the compound(s) of the invention for use as a medicament, e.g. for use in the treatment or prophylaxis of a bacterial infection, especially *a Pseudomonas aeruginosa* or *Burkholderia* infection.

These objects are solved by the subject matter of the attached claims as will become apparent upon reference to the following description and definitions.

The present invention relates to:
[1] A compound having the formula (1): 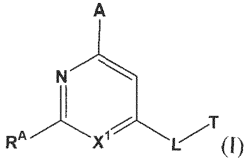 or a pharmacologically acceptable salt thereof,
   wherein
   A represents a 5- or 6-membered heteroaromatic group containing at least one nitrogen atom, which may optionally be substituted by one or more, identical or different, group(s) selected from (C₁-C₃)alkyl group, CN, a halogen atom, and OH;
   X¹ represents CH or N;
   R^{A} represents CF₃, Cl or CN;
   L is a group represented by formula (L-1) or (L-2): 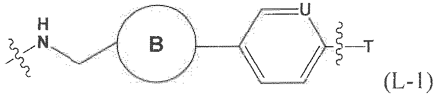 wherein
   ring B is 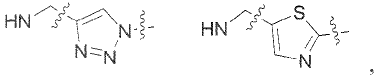 or a 6-membered heterocyclic ring represented by formula (B-1): 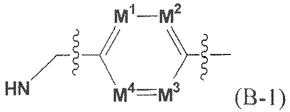
   wherein
   M¹, M², M³ and M⁴ each, independently of one another, represents CH or N; at least one of M¹, M², M³, and M⁴ being N;
   U represents N or C(R^{U});
      R^{U} represents a hydrogen atom, a halogen atom, NR^{U1}R^{U2}, OH, CN, CF₃, CH₂-OH, OCH₃, or OCF₃;
      R^{U1} and R^{U2} each, independently of one another, represents a hydrogen atom; or an akyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted by one or more, identical or different, group(s) selected from a halogen atom, OH, =O, and NH₂; 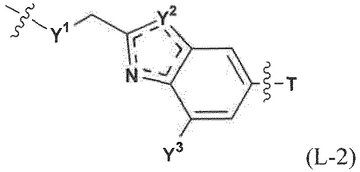
   wherein
      Y¹ is NH or S;
      Y² is N, NH, O, or S;
      Y³ represents a hydrogen atom, F, Cl, OH, CN, (C₁-C₃)alkyl, CF₃, CH₂-OH, OCH₃, or OCF₃;
   each " 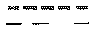 ", independently of one another, represents a single bond or a double bond, wherein at least one " ---" in the ring of formula (L-2) is a double bond;

   T is R or a group: -Z-R', wherein
   R represents a hydrogen atom, a halogen atom, CN, CF₃, CH₂-OH; NR^{T1}R^{T2}; or an akyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
   R^{T1} and R^{T2} each, independently of one another, represents a hydrogen atom or a (C₁-C₃)alkyl group, which may be substituted by one or more, identical or different, group(s) selected from a halogen atom, OH, =O, and NH₂;
   Z is -NH-, -O- or -S-; and
   R' is a hydrogen atom; or an akyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted.

Compounds are usually described herein using standard nomenclature or the definitions presented below. For compounds having asymmetric centers, it should be understood that, unless otherwise specified, all of the optical isomers and mixtures thereof are encompassed. Compounds with two or more asymmetric elements can also be present as mixtures of diastereomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms. It will be apparent that the compound of the invention may, but need not, be present as a hydrate, solvate or non-covalent complex. In addition, the various crystal forms and polymorphs are within the scope of the present invention, as are prodrugs of the compound of the invention. Recited compounds are further intended to encompass compounds in which one or more atoms are replaced with an isotope, *i.e.,* an atom having the same atomic number but a different mass number. By way of general example, and without limitation, isotopes of hydrogen include tritium and deuterium and isotopes of carbon include ¹¹C, ¹³C, and ¹⁴C.

Compounds according to the formulas provided herein, which have one or more stereogenic center(s), have an enantiomeric excess of at least 50%. For example, such compounds may have an enantiomeric excess of at least 60%, 70%, 80%, 85%, 90%, 95%, or 98%. Some embodiments of the compounds have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example a chiral HPLC column.

The compound according to the invention is described herein using a general formula that includes variables such as, *e.g.* A, L, T, M¹-M⁴, R, R', R^{A}, R^{U}, R^{U1}-R^{U2}, R^{T1}-R^{T2}, U, X¹, Y¹-Y³ and Z. Unless otherwise specified, each variable within such a formula is defined independently of any other variable, and any variable that occurs more than one time in a formula is defined independently at each occurrence. Thus, for example, if a group is shown to be substituted with 0-2 R*, the group may be unsubstituted, or substituted with 1 or 2 group(s) R*, wherein R* at each occurrence is selected independently from the corresponding definition of R*. Also, combinations of substituents and/or variables are permissible only if such combinations result in stable compounds, *i.e.,* compounds that can be isolated, characterized and tested for biological activity.

As used herein, a wording defining the limits of a range of length such as, e. g., "from 1 to 5" means any integer from 1 to 5, i. e. 1, 2, 3, 4 and 5. In other words, any range defined by two integers explicitly mentioned is meant to comprise and disclose any integer defining said limits and any integer comprised in said range. For example, the term "C₁-C₃" refers to 1 to 3, *i.e.* 1, 2 or 3, carbon atoms; and the term "C₁-C₆" refers to 1 to 6, *i.e.* 1, 2, 3, 4, 5 or 6, carbon atoms. Further, the prefix "(C_{x-y})" as used herein means that the chain, ring or combination of chain and ring structure as a whole, indicated in direct association of the prefix, may consist of a minimum of x and a maximum of y carbon atoms (*i.e.,* x < y), wherein x and y represent integers defining the limits of the length of the chain (number of carbon atoms) and/or the size of the ring (number of carbon ring atoms).

A "pharmacologically acceptable salt" of a compound disclosed herein is an acid or base salt that is generally considered in the art to be suitable for use in contact with the tissues of human beings or animals without excessive toxicity or carcinogenicity, and preferably without irritation, allergic response, or other problem or complication. Such pharmaceutical salts include mineral and organic acid salts of basic residues such as amines, as well as alkali or organic salts of acidic residues such as carboxylic acids.

Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)*ₙ*-COOH where *n* is any integer from 0 to 4 (*i.e*., 0, 1, 2, 3, or 4) and the like. Similarly, pharmaceutically acceptable cations include, but are not limited to sodium, potassium, calcium, aluminum, lithium and ammonium. Those of ordinary skill in the art will recognize further pharmacologically acceptable salts for the compounds provided herein. In general, a pharmacologically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of nonaqueous media, such as ether, ethyl acetate, ethanol, isopropanol or acetonitrile, is preferred.

A "substituent," as used herein, refers to a molecular moiety that is covalently bonded to an atom within a molecule of interest. For example, a substituent on a ring may be a moiety such as a halogen atom, an alkyl, haloalkyl, hydroxy, cyano, or amino group, or any other substituent described herein that is covalently bonded to an atom, preferably a carbon or nitrogen atom, that is a ring member.

The term "substituted," as used herein, means that any one or more hydrogen atom(s) on the designated atom or group (*e.g.,* alkyl, alkoxy, alkoxyalkyl, cycloalkyl, heterocycloalkyl, heteroaryl) is replaced with a selection from the indicated substituents, provided that the designated atom's normal valence or the group's number of possible sites for substitution is not exceeded, and that the substitution results in a stable compound, *i.e.,* a compound that can be isolated, characterized and tested for biological activity. When a substituent is oxo, *i.e.,* =O, then 2 hydrogens on the atom are replaced. An oxo group that is a substituent of an aromatic carbon atom results in a conversion of -CH- to -C(=O)- and may lead to a loss of aromaticity. For example, a pyridyl group substituted by oxo is a pyridone. The indication mono-, di-, tri or tetrasubstituted denotes groups having one (mono), two (di), three (tri) or four substituents, provided that the substitution does not exceeded the number of possible sites for substitution and results in a stable compound. For example, a monosubstituted imidazolyl group may be an (imidazolidin-2-on)yl group and a disubstituted isoxazolyl group may be a ((3,5-dimethyl)isoxazolyl) group.

As used herein, "comprising", "including", "containing", "characterized by", and grammatical equivalents thereof are inclusive or open-ended terms that do not exclude additional, unrecited elements or method steps. Yet, "Comprising", etc. is also to be interpreted as including the more restrictive terms "consisting essentially of" and "consisting of", respectively.

As used herein, "consisting of" excludes any element, step, or ingredient not specified in the claim.

When trade names are used herein, it is intended to independently include the trade name product formulation, the generic drug, and the active pharmaceutical ingredient(s) of the trade name product.

In general, unless defined otherwise, technical and scientific terms used herein have the same meaning as commonly understood by one of ordinary skill in the art to which this invention belongs, and are consistent with general textbooks and dictionaries.

The expression "optionally substituted" refers to groups in which one or more hydrogen atom(s), e.g. 1 to 6 hydrogen atoms, has/have been replaced, each independently of one another, by fluorine, chlorine, bromine or iodine atom(s); or by OH, =O, SH, =S, NH₂, =NH, CN or NO₂ group(s). This expression refers furthermore to groups in which one or more hydrogen atom(s) has/have been replaced, each independently of the others, by unsubstituted C₁-C₆alkyl, (C₁-C₆) haloalkyl (e.g. a fluoromethyl, trifluoromethyl, chloromethyl, (1- or 2-)haloethyl (e.g. (1- or 2-) chloroethyl), or (2- or 3-) halopropyl (e.g. (2- or 3-) fluoropropyl) group), (C₁-C₆) hydroxyalkyl (e.g. a hydroxymethyl, (1- or 2-)hydroxyethyl, or (2- or 3-) hydroxypropyl group), unsubstituted C₂-C₆alkenyl, unsubstituted C₂-C₆alkynyl, unsubstituted C₁-C₆heteroalkyl, unsubstituted C₃-C₁₀cycloalkyl, unsubstituted C₂-C₉heterocycloalkyl, unsubstituted C₆-C₁₀aryl, unsubstituted C₁-C₉heteroaryl, unsubstituted C₇-C₁₂aralkyl or unsubstituted C₂-C₁₁heteroaralkyl groups.

The expression alkyl or alkyl group denotes a saturated, straight-chain or branched hydrocarbon group that contains from 1 to 20 carbon atoms, preferably from 1 to 12 carbon atoms, more preferably from 1 to 6 carbon atoms, or the number of carbon atoms indicated in the prefix. If an alkyl is substituted, the substitution may take place, independently of one another, by mono-, di-, or tri-substitution of individual carbon atoms of the molecule, *e.g.,* 1, 2, 3, 4, 5, 6, or 7 hydrogen atom(s) may, at each occasion independently, be replaced by a selection from the indicated substituents. The foregoing also applies if the alkyl group forms a part of a group, e.g., haloalkyl, hydroxyalkyl, alkylamino, alkoxy, or alkoxyalkyl. Examples of an alkyl group include methyl, ethyl, propyl, iso-propyl, n-butyl, iso-butyl, sec-butyl, *tert*-butyl, n-pentyl, iso-pentyl, n-hexyl, 2,2-dimethylbutyl, or n-octyl, and examples of a substituted alkyl group or a group where the alkyl forms a part of a group, include haloalkyl, *e.g.,* a trifluoromethyl or a difluoromethyl group; hydroxyalkyl, *e.g*., hydroxymethyl or 2-hydroxyethyl group, and a methoxymethyl group. The term "(C₁₋₆) alkyl" includes, for example, H₃C-, H₃C-CH₂-, H₃C-CH₂-CH₂-, H₃C-CH(CH₃)-, H₃C-CH₂-CH₂-CH₂-, H₃C-CH₂-CH(CH₃)-, H₃C-CH(CH₃)-CH₂, H₃C-C(CH₃)₂-, H₃C-CH₂-CH₂-CH₂-CH₂-, H₃C-CH₂-CH₂-CH(CH₃)-, H₃C-CH₂-CH(CH₃)-CH₂-, H₃C-CH(CH₃)-CH₂-CH₂-, H₃C-CH₂-C(CH₃)₂-, H₃C-C(CH₃)₂-CH₂-, H₃C-CH(CH₃)-CH(CH₃)-, H₃C-CH₂-CH(CH₂CH₃)-, - CH₂CH₂CH₂CH₂CH₂CH₃, -CH(CH₃)CH₂CH₂CH₂CH₃, (H₃CH₂C)CH(CH₂CH₂CH₃)-, - C(CH₃)₂(CH₂CH₂CH₃), -CH(CH₃)CH(CH₃)CH₂CH₃, and -CH(CH₃)CH₂CH(CH₃)₂.

The expression alkenyl refers to an at least partially unsaturated, straight-chain or branched hydrocarbon group that contains one or more double bond(s) and from 2 to 20 carbon atoms, preferably from 2 to 12 carbon atoms, more preferably from 2 to 6 carbon atoms, for example an ethenyl (vinyl), propenyl (allyl), iso-propenyl, butenyl, isoprenyl or hex-2-enyl group. Preferably, an alkenyl group has one or two, especially one, double bond(s).

The expression alkynyl refers to an at least partially unsaturated, straight-chain or branched hydrocarbon group that contains one or more triple bond(s) and from 2 to 20 carbon atoms, preferably from 2 to 12 carbon atoms, more preferably from 2 to 6, *e.g.,* 2, 3 or 4, carbon atoms, for example an ethynyl (acetylenyl), propynyl, butynyl or propargyl group. Preferably, an alkynyl group has one or two, especially one, triple bond(s).

The expression alkoxy or alkoxy group refers to an alkyl group singular bonded to oxygen, *i.e., -* O-alkyl. The term "(C₁-C₆) alkoxy" includes, for example, methoxy, ethoxy, *n*-propoxy, isopropoxy, *n*-butoxy, *sec*-butoxy, isobutoxy, *tert*-butoxy, *n*-pentyloxy , *tert*-amyloxy- or *n-*hexyloxy, and accordingly (C₁-C₃)alkoxy includes methoxy, ethoxy, *n*-propoxy, or isopropoxy.

The expression alkoxyalkyl or alkoxyalkyl group refers to an alkyl group singular bonded to one or more alkoxy group(s), *e.g*., -alkyl-O-alkyl or -alkyl-O-alkyl-O-alkyl. The term "(C₂-C₅) alkoxyalkyl" includes, for example, methoxymethyl, methoxyethoxymethyl, and 1-ethoxyethyl.

The expression haloalkyl or haloalkyl group refers to an alkyl group in which one, two, three or more hydrogen atoms have been replaced independently of each other by a halogen atom. The term "(C₁-C₃) haloalkyl" includes, for example, fluoromethyl, difluoromethyl, trifluoromethyl, chloromethyl, dichloromethyl, bromomethyl, dibromomethyl, iodomethyl, (1- or 2-)haloethyl (*e.g.,* (1- or 2-)fluoroethyl or (1- or 2-)chloroethyl), (2- or 3-) halopropyl (*e.g.,* (2- or 3-) fluoropropyl or (2- or 3-) chloropropyl).

The expression hydroxyalkyl or hydroxyalkyl group refers to an alkyl group in which one, two, three or more hydrogen atoms have been replaced independently of each other by a hydroxy (OH) group. The term "(C₁-C₄) hydroxyalkyl" includes, for example, hydroxymethyl, hydroxyethyl, hydroxypropyl and hydroxybutyl.

As used herein, the expression heteroalkyl or heteroalkyl group refers to an alkyl group, straight chain or branched as defined above, in which one or more, preferably 1, 2, 3 or 4, carbon atom(s) has/have been replaced, each independently of one another, by an oxygen, nitrogen, selenium, silicon or sulfur atom, preferably by an oxygen, sulfur or nitrogen atom, C(O), OC(O), C(O)O, C(O)NH, NH, SO, SO₂ or by a CH=CH group, wherein said heteroalkyl group may be substituted. For example, a "(C₁₋C₄)heteroalkyl group" contains from 1 to 4, *e.g.* 1, 2, 3 or 4, carbon atoms and 1, 2, 3 or 4, preferably 1, 2 or 3, heteroatoms selected from oxygen, nitrogen and sulfur (especially oxygen and nitrogen). Examples of an heteroalkyl group include alkylamino, dialkylamino, alkylaminoalkyl, dialkylaminoalkyl, acyl, acylalkyl, alkoxycarbonyl, acyloxy, acyloxyalkyl, carboxyalkylamide, alkoxycarbonyloxy, alkylcarbamoyl, alkylamido, alkylcarbamoylalkyl, alkylamidoalkyl, alkylcarbamoyloxyalkyl, alkylureidoalkyl, alkoxy, alkoxyalkyl, or alkylthio group. The expression alkylthio or alkylthio group refers to an alkyl group, in which one or more non-adjacent CH₂ group(s) are replaced by sulfur, wherein the alkyl moiety of the alkylthio group may be substituted. Examples of heteroalkyl groups are groups of formulae: R^{a}-O-Y^{a}-, R^{a}-S-Y^{a}-, R^{a}-SO-Y^{a}-, R^{a}-SO₂-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂₋C₆ alkenyl or a C₂-C₆ alkynyl group and Y^{a} being a direct bond, a C₁-C₆ alkylene, a C₂-C₆ alkenylene or a C₂-C₆ alkynylene group, wherein each heteroalkyl group contains at least one *carbon* atom and, optionally, one or more hydrogen atom(s) may be replaced by fluorine or chlorine atom. Specific examples of a heteroalkyl group include acyl, methoxy, trifluoromethoxy, ethoxy, n-propyloxy, isopropyloxy, *tert*-butyloxy, methoxymethyl, ethoxymethyl, methoxyethyl, methylamino, ethylamino, dimethylamino, diethylamino, isopropylethylamino, methylaminomethyl, ethylaminomethyl, diisopropylaminoethyl, dimethylaminomethyl, dimethylaminoethyl, acetyl, propionyl, butyryloxy, acetyloxy, methoxycarbonyl, ethoxycarbonyl, isobutyrylamino-methyl, *N*-ethyl-*N*-methylcarbamoyl, *N-*methylcarbamoyl, cyano, nitrile, isonitrile, thiocyanate, isocyanate, isothiocyanate and alkylnitrile.

The expression cycloalkyl or cycloalkyl group refers to a saturated carbocyclic ring group comprising one or more rings (preferably 1 or 2) and containing from 3 to 14 ring carbon atoms, preferably from 3 to 10 (more preferably 3, 4, 5, 6 or 7) ring carbon atoms; the cycloalkyl group may be substituted and can be bonded as a substituent via every suitable position of the ring system. Examples of cycloalkyl include monocyclic hydrocarbon rings, bicyclic hydrocarbon rings and spiro-hydrocarbon rings. In a bicyclic cycloalkyl group, two rings are joined together so that they have at least two carbon atoms in common. In a spiro-hydrocarbon ring, 2 or 3 rings are linked together by one common atom carbon atom (spiro-atom). If a cycloalkyl is substituted, the substitution may take place, independently of one another, by mono- or disubstitution of individual ring carbon atoms of the molecule, and the cycloalkyl group as a whole may carry 1, 2, 3, or 4 substituents from the indicated selection of substituents, *i.e.,* 1, 2, 3, or 4 hydrogen atom(s) of the carbon ring atoms may, at each occasion independently, be replaced by a substituent selected from the indicated list of substituents thereby resulting in a mono-, di-, tri-, or tetrasubstituted cycloalkyl group. Examples of cycloalkyl include cyclopropyl, cyclobutyl, cyclopentyl, cyclohexyl, cycloheptyl, bicyclo[2.2.0]hexyl, bicyclo[3.2.0]heptyl, bicyclo[3.2.1]octyl, bicyclo[2.2.2]octyl, bicyclo[4.3.0]nonyl (octahydroindenyl), bicyclo[4.4.0]decyl (decahydronaphthyl), bicyclo[2.2.1]heptyl (norbornyl), bicyclo[4.1.0]heptyl (norcaranyl), bicyclo[3.1.1]heptyl (pinanyl), spiro[2.5]octyl, and spiro[3.3]heptyl. If a cycloalkyl is partially unsaturated, the group contains one, two or more double bonds, such as, for example, a cycloalkenyl group, including cyclopropenyl, cyclobutenyl, cyclopentenyl, cyclohexenyl, cycloheptenyl, cyclobutadienyl, cyclopentadienyl, cyclohexadienyl, bicyclo[2.2.1]heptadienyl, and spiro[4,5]decenyl.

The expression heterocycloalkyl or heterocycloalkyl group refers to a cycloalkyl group, saturated or partially unsaturated, as defined above, in which one or more, preferably 1, 2 or 3, ring carbon atom(s) has/have been replaced each independently of one another by an oxygen, nitrogen or sulfur atom, preferably oxygen or nitrogen, or by NO, SO or SO₂; the heterocycloalkyl may be substituted and can be bonded as a substituent via every suitable position of the ring system; at least one carbon atom must be present between two oxygen atoms and between two sulfur atoms or between an oxygen and a sulfur atom; and the ring as a whole must have chemical stability. A heterocycloalkyl group has preferably 1 or 2 ring(s) containing from 3 to 10 (more preferably 3, 4, 5, 6 or 7, and most preferably 5, 6 or 7) ring atoms. Examples of heterocycloalkyl include aziridinyl, oxiranyl, thiiranyl, oxaziridinyl, dioxiranyl, azetidinyl, oxetanyl, thietanyl, diazetidinyl, dioxetanyl, dithietanyl, pyrrolidinyl, tetrahydrofuranyl, thiolanyl, azolyl, thiazolyl, isothiazolyl, imidazolidinyl, pyrazolidinyl, oxazolidinyl, isoxazolidinyl, thiazolidinyl, isothiazolidinyl, dioxolanyl, dithiolanyl, piperazinyl, morpholinyl, thiomorpholinyl, trioxanyl, azepanyl, oxepanyl, thiepanyl, homopiperazinyl, urotropinyl, oxazolidinonyl, dihydropyrazolyl, dihydropyrrolyl, dihydropyrazinyl, dihydropyridyl, dihydropyrimidinyl, dihydrofuryl, dihydropyranyl, and examples of substituted heterocycloalkyl include lactam, lactone and cyclic imide ring systems.

The expression alkylcycloalkyl refers to a group containing both cycloalkyl and also an alkyl, alkenyl or alkynyl group in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two ring systems having from 3 to 10 (preferably 3, 4, 5, 6 or 7) carbon atoms, and one or two alkyl, alkenyl or alkynyl groups having 1 or 2 to 6 carbon atoms, the cyclic groups being optionally substituted.

The expression heteroalkylcycloalkyl refers to alkylcycloalkyl groups as defined above in which one or more (preferably 1, 2 or 3) carbon atoms have been replaced each independently of the others by an oxygen, nitrogen, silicon, selenium, phosphorus or sulfur atom (preferably oxygen, sulfur or nitrogen). A heteroalkylcycloalkyl group preferably contains 1 or 2 ring systems having from 3 to 10 (preferably 3, 4, 5, 6 or 7) ring atoms, and one or two alkyl, alkenyl, alkynyl or heteroalkyl groups having from 1 or 2 to 6 carbon atoms. Examples of such groups are alkylhetero-cycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being optionally substituted and saturated or mono-, di- or tri-unsaturated.

The expressions aryl, Ar or aryl group refer to an aromatic group that contains one or more aromatic rings containing from 6 to 14 ring carbon atoms (C₆-C₁₄), preferably from 6 to 10 (C₆-C₁₀), more preferably 6 ring carbon atoms; the aryl may be substituted and can be bonded as a substituent via every suitable position of the ring system. Examples of aryl include phenyl, naphthyl, bi-phenyl, indanyl, indenyl, anthracenyl, phenanthrenyl, tetrahydronaphthyl and fluorenyl.

The expression heteroaryl or heteroaryl group refers to an aromatic group that contains one or more aromatic rings containing from 5 to 14 ring atoms, preferably from 5 to 10 (more preferably 5 or 6) ring atoms, and contains one or more (preferably 1, 2, 3 or 4) oxygen, nitrogen, phosphorus or sulfur ring atoms (preferably O, S or N); the heteroaryl may be substituted and can be bonded as a substituent via every suitable position of the ring system. Examples of an unsubstituted heteroaryl group include 2-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl.

The expression aralkyl refers to a group containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcyclo-alkenyl groups. Specific examples of aralkyls are toluene, xylene, mesitylene, styrene, benzyl chloride, o-fluorotoluene, 1H-indene, tetralin, dihydronaphthalene, indanone, phenylcyclopentyl, cumene, cyclohexylphenyl, fluorene and indan. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to an aralkyl group as defined above in which one or more (preferably 1, 2, 3 or 4) carbon atoms have been replaced each independently of the others by an oxygen, nitrogen, silicon, selenium, phosphorus, boron or sulfur atom (preferably oxygen, sulfur or nitrogen), that is to say to groups containing both aryl or heteroaryl and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced each independently of the others by oxygen, sulfur or nitrogen atoms. Examples of heteroaralkyl groups are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroaryl-alkylcycloalkyl, heteroarylalkylheterocycloalkenyl, heteroarylheteroalkylcycloalkyl, heteroaryl-heteroalkylcycloalkenyl, heteroalkylheteroarylalkyl and heteroarylheteroalkylheterocycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

The expression heterocycle denotes ring systems, which include the above defined heterocycloalkyl and heteroaryl ring systems, e.g. a partially unsaturated heterocycle is synonymous with a partially unsaturated heterocycloalkyl and an aromatic heterocycle, e.g. a 6-membered heteroaromatic group, is synonymous with a heteroaryl. The heterocycle may be substituted and can be bonded as a substituent via every suitable position of the ring system. Examples of a partially unsaturated or aromatic heterocycle include oxetenyl, thietenyl, azetinyl, 2,3-dihydrofuranyl, 2,5-dihydrofuranyl, 2,5-dihydrothiophenyl, 2,5-dihydro-1H-pyrrolyl, furanyl, thiophenyl, pyrrolyl, benzo[b]furanyl, benzo[b]thiophenyl, indolyl, benzo[c]pyrrolyl, benzo[a]pyrrolyl, imidazolyl, pyrazolyl, oxazolyl, isoxazolyl, thiazolyl, isothiazolyl, 1,2,3-triazolyl, 1,2,4-triazolyl, tetrazolyl, dihydropyridinyl, oxazinyl, pyridinyl, dihydropyranyl, azepinyl, tetrahydropyranyl, dihydrothiopyranyl, quinolinyl, isoquinolinyl, quinazolinyl, pyridazinyl, pyrimidinyl, pyrazinyl, purinyl, and pteridinyl.

The general term ring as used herein, unless defined otherwise, includes the cyclic groups defined herein above, *e.g.,* a cycloalkyl group, heterocycloalkyl group, aryl group, heteroaryl group, and heterocycle.

The expression halogen or halogen atom as used herein means fluorine, chlorine, bromine, or iodine, among which fluorine and chlorine are usually preferred.

The expression heteroatom as used herein, preferably denotes an oxygen, nitrogen or sulfur atom, more preferably a nitrogen or oxygen atom unless specified otherwise.

The expression "alkylene" (or alkanediyl functional group) refers to an unsubstituted, saturated, straight chain hydrocarbon group that contains the indicated number of carbon atoms (in the form of methylene (CH₂) groups) and has the free valencies at the terminal methylene groups, for example a butylene -(CH₂)₄-, n-pentylene -(CH₂)₅-, n-hexylene -(CH₂)₆-, or n-octylene -(CH₂)₈-group.

The expression "alkenylene" refers to an at least partially unsaturated alkanediyl functional group as defined above that contains one or more double bond(s) (i.e. the methylene groups of the alkanediyl functional group are interrupted by -CH=CH- and/or terminated by -CH₂-CH=).

The expression "alkynylene" refers to an at least partially unsaturated alkanediyl functional group as defined above that contains one or more triple bond(s) (i.e. the methylene groups of the alkanediyl functional group are interrupted by -CH≡CH- and/ or terminated by -CH₂-C≡).

The term "PqsR inverse agonist", as used herein, refers to a compound of general formula (I) provided herein, as well as to salts and preferably pharmaceutically acceptable salts thereof. It will be apparent that such compounds may be further substituted as indicated.

The activity and more specifically the bioactivity of the compounds according to the present invention can be assessed using appropriate assays known to those skilled in the art, e.g. *in vitro* or *in vivo* assays. For instance, the PqsR inverse agonistic activity may be determined by *E. coli-*based β-galactosidase reporter gene assay, or evaluated in PQS assay, pyocyanin virulence assay and biofilm assay, as known in the art (see, *e.g*., references cited or PCT/EP2019/067899) and/or provided in the Examples below.

Preferably, the present invention relates to one or more of the following:
[2] the compound according to [1] above, or a pharmacologically acceptable salt thereof, wherein X² is CH;
[3] the compound according to [1], or a pharmacologically acceptable salt thereof, wherein X¹ is N;
[4] the compound according to any one of [1] to [3], or a pharmacologically acceptable salt thereof, wherein A represents a 5-membered heteroaromatic group containing at least one nitrogen atom, which may optionally be substituted by a group selected from a (C₁-C₃)alkyl group, CN, a halogen atom, and OH;
[5] the compound according to [4], or a pharmacologically acceptable salt thereof, wherein A is a group selected from the groups: 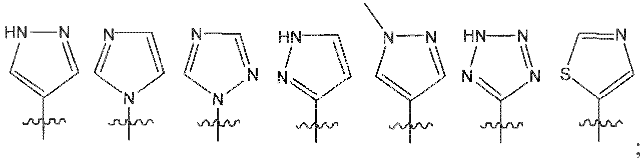
[6] the compound according to any one of [1] to [3], or a pharmacologically acceptable salt thereof, wherein A is a 6-membered heteroaromatic group containing at least one nitrogen atom, which may optionally be substituted by a group selected from (C₁-C₃)alkyl group, CN, halogen atom, and OH;
[7] the compound according to [6], or a pharmacologically acceptable salt thereof, wherein A is group selected from the groups: 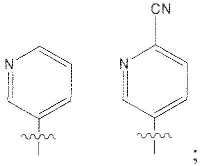
[8] the compound according to any one of [1] to [7], or a pharmacologically acceptable salt thereof, wherein R^{A} represents CF₃;
[9] the compound according to any one of [1] to [8], or a pharmacologically acceptable salt thereof, wherein L is a group of formula (L-1);
[10] the compound according to [9], or a pharmacologically acceptable salt thereof, wherein ring B is 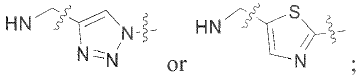
[11] the compound according to [9], or a pharmacologically acceptable salt thereof, wherein ring B is a 6-membered heterocyclic ring represented by formula (B-1), which is selected from the groups: 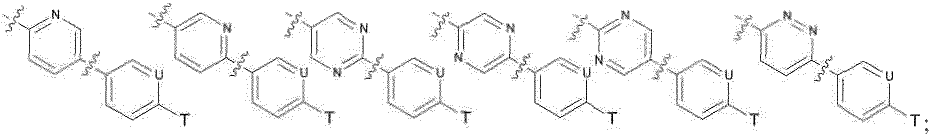
[12] the compound according to any one of [1] to [11], or a pharmacologically acceptable salt thereof, wherein R^{U} represents a hydrogen atom, Cl, F, CN; or a group selected from the groups: 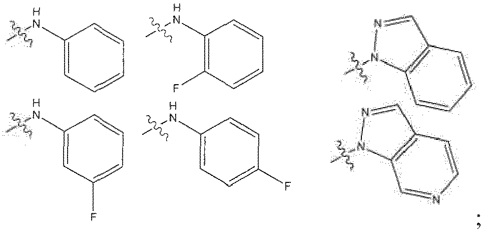
[13] the compound according to [9], [10] or [12], or a pharmacologically acceptable salt thereof, wherein the group represented by formula (L-1) contains one of the following groups as a partial structure: 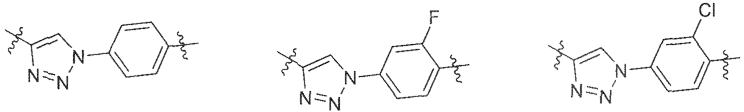 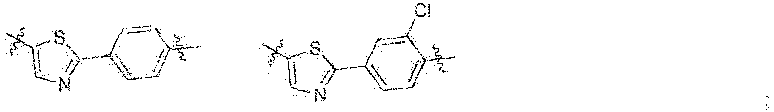
[14] the compound according to [9], [11] or [12], or a pharmacologically acceptable salt thereof, wherein the group represented by formula (L-1) contains one of the following groups as a partial structure: 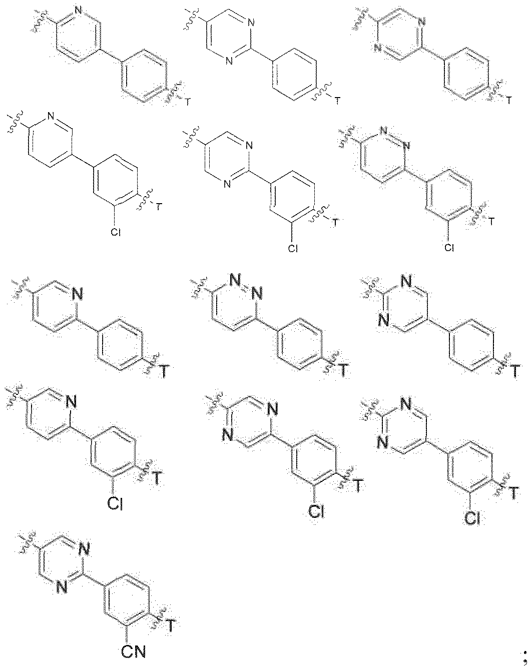
[15] the compound according to any one of [1] to [8], or a pharmacologically acceptable salt thereof, wherein L is a group of formula (L-2);
[16] the compound according to [15], or a pharmacologically acceptable salt thereof, wherein Y¹ is NH;
[17] the compound according to [15], or a pharmacologically acceptable salt thereof, wherein Y¹ is S;
[18] the compound according to any one of [15] to [17], or a pharmacologically acceptable salt thereof, wherein the group represented by formula (L-2) is a group selected from: 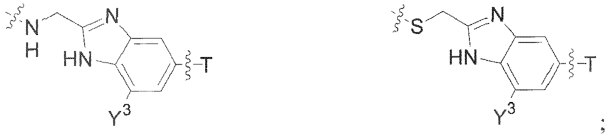
[19] the compound according to any one of [15] to [18], or a pharmacologically acceptable salt thereof, wherein Y³ represents a hydrogen atom, F or CH₃;
[20] the compound according to any one of [1] to [19], or a pharmacologically acceptable salt thereof, wherein T is a hydrogen atom, Cl, F; or a group selected from the groups: 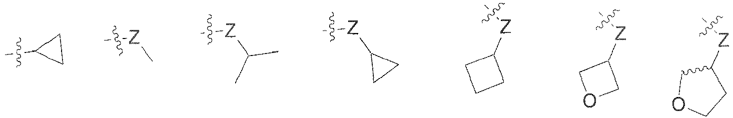 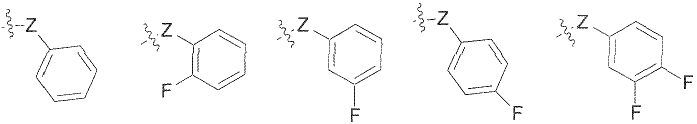 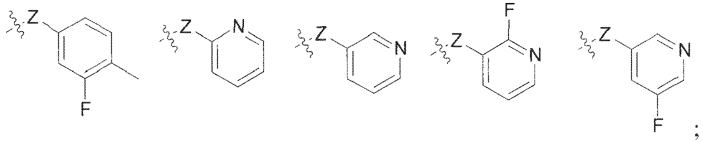
[21] the compound according to [20], or a pharmacologically acceptable salt thereof, wherein Z is NH or O;
[22] the compound according to [21], or a pharmacologically acceptable salt thereof, wherein Z is NH;
[23] the compound according to [21], or a pharmacologically acceptable salt thereof, wherein Z is O;
[24] the compound according to any one of [1] to [23], or a pharmacologically acceptable salt thereof, wherein the compound is selected from the group consisting of:

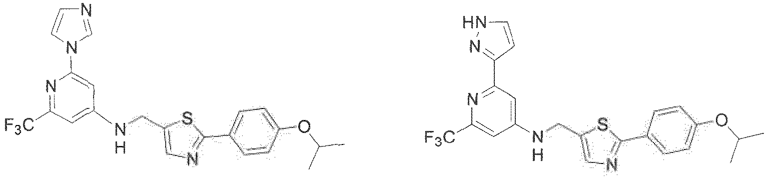

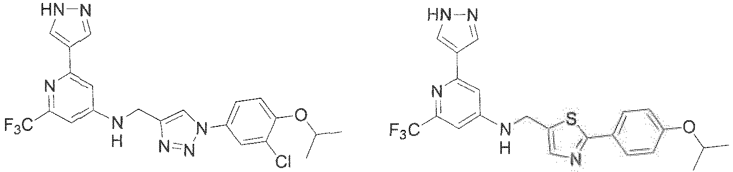

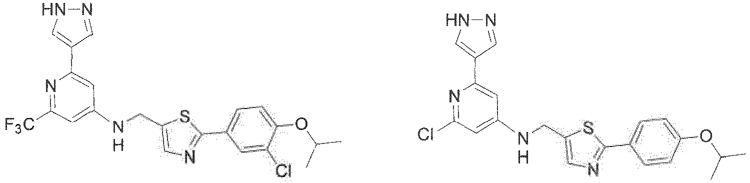

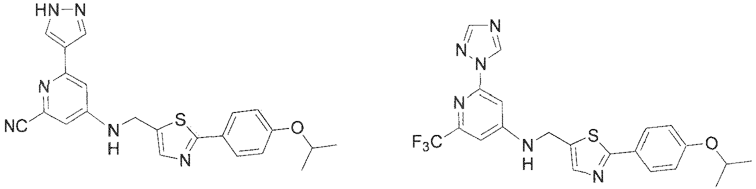

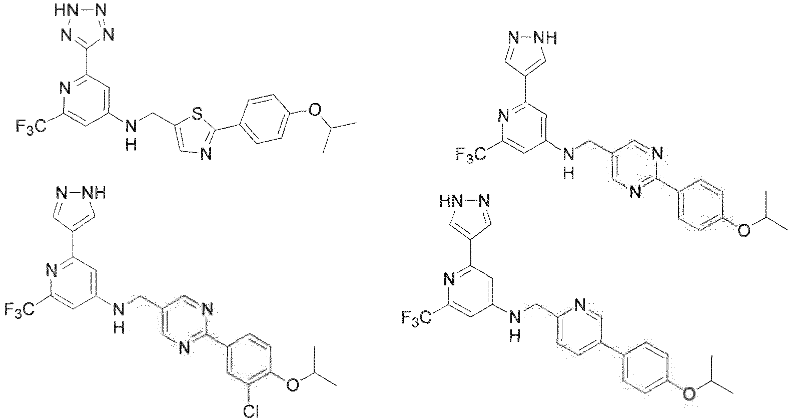

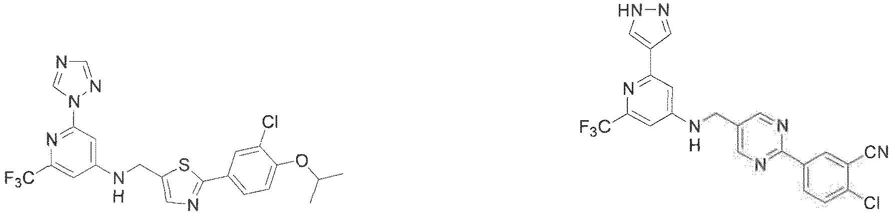

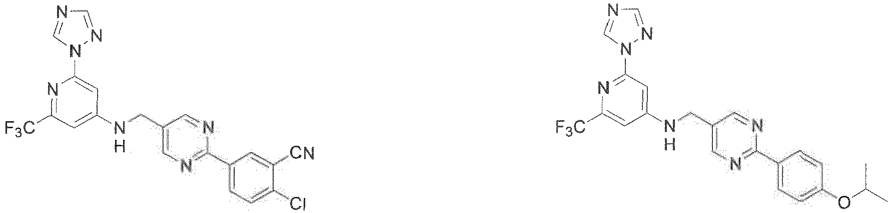

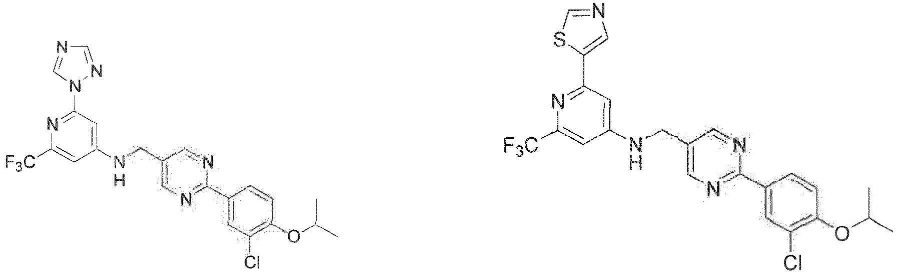

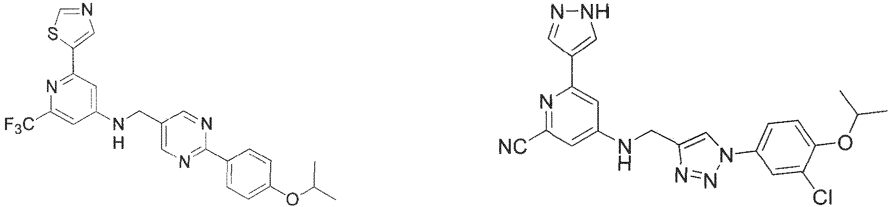

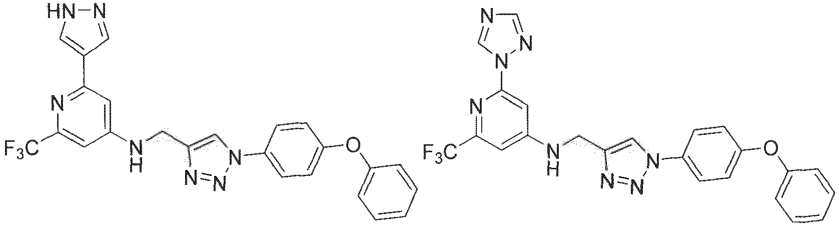

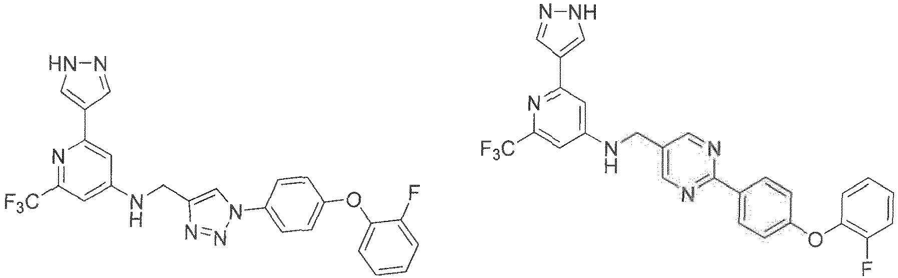

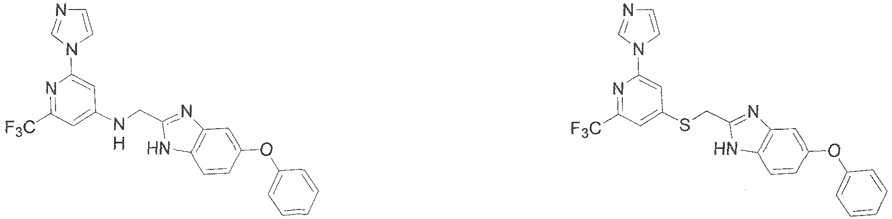

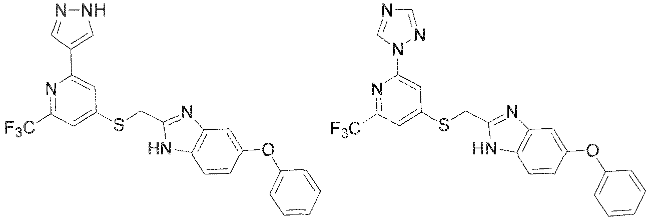

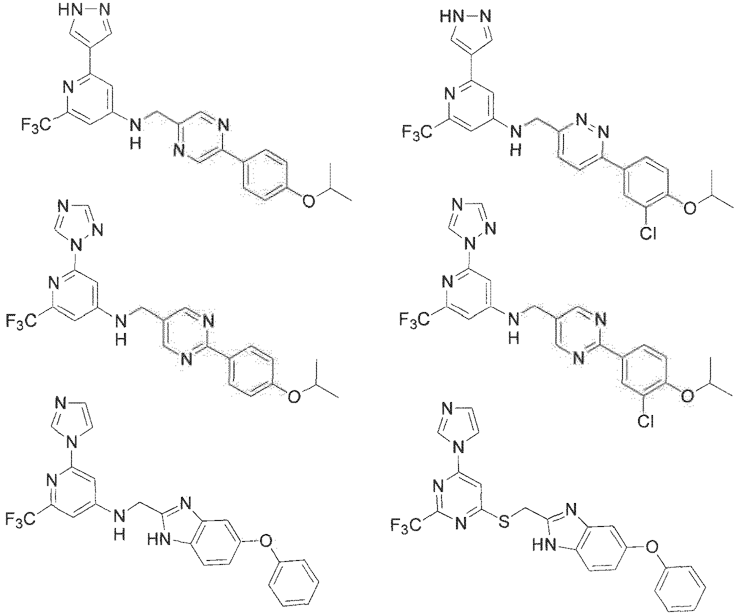

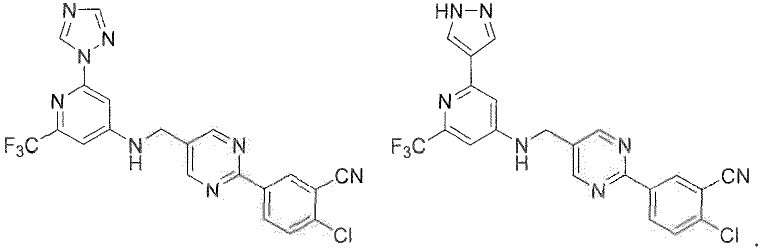

Compounds including suitable combinations of preferred embodiments, *i.e.,* [2] to [23] or [24], of the compound according to general formula (I), or a salt thereof, are particularly preferred; *e.g.,* a compound or salt thereof including a combination of [1], [5], [13] and [20]; [1], [5], [14] and [20] or [1], [5], [18] and [20]; as disclosed herein. In other words, the present invention specifically encompasses all possible combinations of [1] to [23] as indicated above, which result in a stable compound.

A compound of formula (I) can be obtained by chemical synthesis in a number of ways well known to one skilled in the art of organic synthesis using usual chemical reaction and synthesis methods. For example, intermediates of the compounds according to the invention can be prepared as described in PCT/EP2019/067899, published as WO2020/007938, which intermediates can then be converted to claimed compounds based on the materials and the reaction conditions for the preparation of compounds of formula (I) provided in the examples below.

The compound of formula (I), its pharmacologically acceptable salts, solvates or hydrates and also formulations and pharmaceutical compositions which contain the same for therapeutic use are within the scope of the present invention. Accordingly, the present invention relates to a compound or a pharmaceutical composition of the invention for use as a medicament. The present invention also relates to the use of those compounds of formula (I) as active ingredients in the preparation or manufacture of a medicament.

A pharmaceutical composition according to the present invention comprises at least one compound of formula (I) and, optionally, one or more carrier substance(s), excipient(s) and/or adjuvant(s). Pharmaceutical compositions may additionally comprise, for example, one or more of water, buffers (*e.g.,* neutral buffered saline or phosphate buffered saline), ethanol, mineral oil, vegetable oil, dimethylsulfoxide, carbohydrates (*e.g.,* glucose, mannose, sucrose or dextrans), mannitol, proteins, adjuvants, polypeptides or amino acids such as glycine, antioxidants, chelating agents such as EDTA or glutathione and/or preservatives. Furthermore, one or more other active ingredients may (but need not) be included in the pharmaceutical compositions provided herein. For instance, the compounds of the invention may advantageously be employed in combination with another antibiotic or antifungal agent, an anti-viral agent, an anti-histamine, a non-steroidal anti-inflammatory drug, a disease modifying anti-rheumatic drug, another cytostatic drug, a drug with smooth muscle activity modulatory activity or mixtures of the aforementioned.

Pharmaceutical compositions may be formulated for any appropriate route of administration, including, for example, topical such as, *e.g.,* transdermal or ocular, oral, buccal, nasal, vaginal, rectal or parenteral administration. The term parenteral as used herein includes subcutaneous, intradermal, intravascular such as, *e.g.,* intravenous, intramuscular, spinal, intracranial, intrathecal, intraocular, periocular, intraorbital, intrasynovial and intraperitoneal injection, as well as any similar injection or infusion technique. Within the invention, compositions provided herein may be formulated as a lyophilizate. Formulation for topical administration may be preferred for certain conditions such as, *e.g.,* in the treatment of skin conditions such as burns or itch.

Carrier substances are, for example, cyclodextrins such as hydroxypropyl β-cyclodextrin, micelles, liposomes, nanoparticles such as solid lipid nanoparticles, excipients and/or adjuvants. Customary excipients include, for example, inert diluents such as, e.g., calcium carbonate, sodium carbonate, lactose, calcium phosphate or sodium phosphate, granulating and disintegrating agents such as, *e.g.,* corn starch or alginic acid, binding agents such as, *e.g.,* starch, gelatin or acacia, and lubricating agents such as, *e.g.,* magnesium stearate or stearic acid. Examples of adjuvants are aluminum hydroxide, aluminum phosphate, calcium phosphate hydroxide, paraffin oil, squalene, thimerosal, detergents, Freund's complete adjuvant, or Freund's incomplete adjuvant.

For the prevention and/or treatment of bacterial infections, especially *P. aeruginosa* or *Burkholderia* infections, the dose of the biologically active compound according to the invention may vary within wide limits and may be adjusted to individual requirements. Active compounds according to the present invention are generally administered in a therapeutically effective amount. The expression "therapeutically effective amount" denotes a quantity of the compound(s) that produces a result that in and of itself helps to ameliorate, heal, or cure the respective condition or disease. Preferred doses range from about 0.1 mg to about 140 mg per kilogram of body weight per day (about 0.5 mg to about 7 g per patient per day). The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, and rate of excretion, drug combination (*i.e*., other drugs being used to treat the patient) and the severity of the particular disease undergoing therapy.

The invention further relates to a combination preparation containing at least one compound according to the invention and at least one further active pharmaceutical ingredient. The combination preparation of the invention for use as a medicament, in particular for use in the treatment or prophylaxis of bacterial infections, such as a *P. aeruginosa* or *Burkholderia* infection.

Preferably, in the combination preparation of the invention the further active pharmaceutical ingredient is another antibiotic, including classical antibiotics and anti-virulence compounds such as quorum sensing and adhesion inhibitors. The other antibiotic can be selected from the group consisting of (a) β-lactam antibiotics, including penams, carbapenams, oxapenams, penems, carbapenems, monobactams, cephems, carbacephems, oxacephems, and monobactams; (b) aminoglycoside antibiotics, including Amikacin, Arbekacin, Astromicin, Bekanamycin, Dibekacin, Framycetin, Gentamicin, Hygromycin B, Isepamicin, Kanamycin, Neomycin, Netilmicin, Paromomycin, Paromomycin sulfate, Ribostamycin, Sisomicin, Spectinomycin, Streptomycin, Tobramycin, and Verdamicin; (c) quinolone antibiotics, including Ciprofloxacin, Enoxacin, Gatifloxacin, Grepafloxacin, Levofloxacin, Lomefloxacin, Moxifloxacin, Nalidixic acid, Norfloxacin, Ofloxacin, Sparfloxacin, Temafloxacin, and Trovafloxacin; and (d) cationic peptidic antibiotics, including Polymyxins (*e.g*., Collistin), LL-37, and POL7080.

Preferred compounds of the invention will have certain pharmacological properties. Such properties include, but are not limited to oral bioavailability, such that the preferred oral dosage forms discussed above can provide therapeutically effective levels of the compound *in vivo.*

The compound according to the invention as well as the pharmaceutical composition according to the invention can be used as a medicament, which can be administered to a patient (e.g. parenterally to a human or another mammal), and will be present within at least one body fluid or tissue of the patient. As used herein, the term "treatment" encompasses both disease-modifying treatment and symptomatic treatment, either of which may be prophylactic, i.e., before the onset of symptoms, in order to prevent, delay or reduce the severity of symptoms, or therapeutic, i.e., after the onset of symptoms, in order to reduce the severity and/or duration of symptoms. In particular, the conditions or diseases that can be ameliorated, prevented or treated using a compound of formula (I) or a pharmaceutical composition according to the invention include bacterial infections, in particular antimicrobial activity against Gram-negative bacteria, especially infections with *Pseudomonas aeruginosa* strains (such as PAO1, PA14, MHH9639, MHH11444, and further clinical isolates exhibiting an intact *pqs* QS system) including such of the urinary tract, of the gastrointestinal tract, of chronic and burnt wounds, of the eyes, of the ears, and of the lungs or infections with *Burkholderia* species (such as *B. cenocepacia* and *B. pseudomallei*). Accordingly, the present invention also provides compounds for use in methods for treating a subject, e.g. patients, suffering from said diseases. Patients may include but are not limited to primates (especially humans), domesticated companion animals (such as dogs, cats, horses) and livestock (such as cattle, pigs, sheep, chicken), with dosages as described herein.

The present invention also enables prophylaxis or treatment of a respiratory condition, including cystic fibrosis (CF), non-cystic-fibrosis bronchiectasis (NCFB), chronic obstructive pulmonary disease (COPD) and primary ciliary dyskinesia. That is to say, the present invention also includes compounds for use in a method for preventing or treating a respiratory condition, including cystic fibrosis (CF), non-cystic-fibrosis bronchiectasis (NCFB), chronic obstructive pulmonary disease (COPD) and primary ciliary dyskinesia, in a subject, the method comprising:
administering to said subject an effective amount of the compound (i.e. a compound according to any one of [1] to [24]), of the pharmaceutical composition, or of the combination preparation according to the present invention,
thereby treating the respiratory condition.

The invention further relates to a coating for medicinal devices, e.g. catheters, implants, or tubings, containing at least one compound according to the present invention.

The present invention is now further illustrated by the following examples from which further features, embodiments and advantages of the present invention may be taken. However, the invention should not be construed to be limited to the examples, but encompasses the subject-matter as defined in the claims.

### EXAMPLES

### Materials and Methods

### A. Chemicals and analytical methods used for organic synthesis

¹H and ¹³C NMR spectra were recorded on a Bruker DRX-500 instrument. Chemical shifts are given in parts per million (ppm) with the solvent resonance as internal standard for spectra obtained in CDCl₃, MeOH-*d*₄ and DMSO-*d*₆. All coupling constants (*J*) are given in hertz. Mass spectrometry (LC/MS) was performed on a MSQ^{®} electro spray mass spectrometer (Thermo Fisher). The system was operated by the standard software Xcalibur^{®}. A RP C18 NUCLEODUR^{®} 100-5 (125 × 3 mm) column (Macherey-Nagel GmbH) was used as stationary phase with water/acetonitrile mixtures as eluent. All solvents were HPLC grade. Reagents were used as obtained from commercial suppliers without further purification. Flash chromatography was performed on silica gel 60, 70-230 mesh (Fluka) and the reaction progress was determined by thin-layer chromatography (TLC) analyses on silica gel 60, F₂₅₄ (Merck). Visualization was accomplished with UV light and staining with basic potassium permanganate (KMnO₄). The melting points were measured using melting point apparatus SMP3 (Stuart Scientific). The apparatus is uncorrected.

Specific examples for the preparation of compounds of formula (I) are provided in the following examples. Unless otherwise specified all starting materials and reagents are of standard commercial grade, and are used without further purification, or are readily prepared from such materials by routine methods or in accordance with procedures as described in PCT/EP2019/067899. Those skilled in the art of organic synthesis will recognize that starting materials and reaction conditions may be varied including additional steps employed to produce compounds encompassed by the present invention.

### B. Chemicals, bacterial strains and media used in biological experiments.

Yeast extract was purchased from Fluka (Neu-Ulm, Germany), peptone from casein from Merck (Darmstadt, Germany), and Bacto^{™} Tryptone from BD Biosciences (Heidelberg, Germany),. Salts and organic solvents of analytical grade were obtained from VWR (Darmstadt, Germany).

*P. aeruginosa* strain PA14 (PA14) was stored in glycerol stocks at - 80 °C.

The following media were used: Luria Bertani broth (LB) and PPGAS medium [23].

### Example 1. Compounds 01 to 60

### 1.1 Preparation of 2-Chloro-N-((1-(3-chloro-4-isopropoxyphenyl)-1H-1,2,3-triazol-4-yl)methyl)-6-(trifluoromethyl)pyridin-4-amine

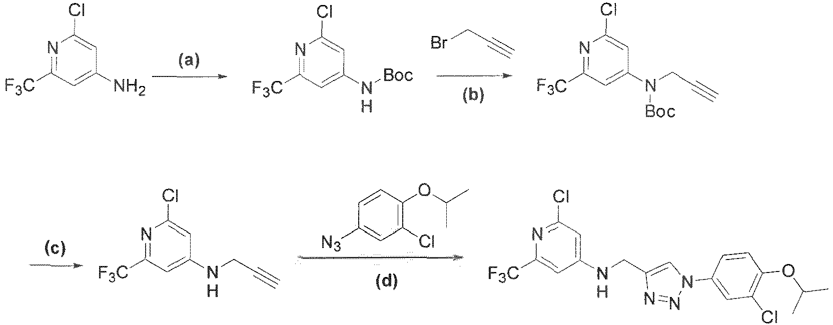

### Step (a) tert-Butyl (2-chloro-6-(trifluoromethyl)pyridin-4-yl)carbamate

2-Chloro-6-(trifluoromethyl)pyridin-4-amine (589.7 mg, 3 mmol), DMAP (73.3 mg, 0.6 mmol), Boc₂O (1.96 g, 9 mmol) and Et₃N (546 µl, 3.9 mmol) were suspended in anhydrous THF (10 mL) at rt, and stirred overnight under argon atmosphere. The reaction mixture was diluted with CH₂Cl₂ (40 mL) and washed with 0.1 N HCl (5 mL). The combined organic layers were dried over anhydrous MgSO₄ and evaporated under reduced pressure. The residue obtained was then dissolved in DCM (20 ml) and TFA (2 ml) was added. The reaction was stirred for 30 min at room temp until the starting material was fully consumed. Saturated NaHCO₃ was added followed by extraction with DCM. The organic solvent was then dried over MgSO₄ and evaporated under reduced pressure. Purification was done using automated column chromatography. LC-MS: m/z: 295 (M-H)⁻.

### Step (b) tert-Butyl (2-chloro-6-(trifluoromethyl)pyridin-4-yl)(prop-2-yn-1-yl)carbamate

To a stirred solution of *tert*-butyl (2-chloro-6-(trifluoromethyl)pyridin-4-yl)carbamate (593 mg, 2 mmol) in DMF (5 ml) at 0°C was added NaH (60wt%) (92.1 mg, 2.4 mmol) 3-bromoprop-1-yne (72.17 mg, 2.4 mmol). The reaction was stirred for 30 min and then propargylbromide (182 µL, 2.4 mmol) was added dropwise and stirred for 3 h at r.t. The reaction was then added on ice water followed by extraction with ethyl acetate. The organic solvent was then dried over MgSO₄ and evaporated under reduced pressure. The product was taken to the next step without further purification. LC-MS: m/z: 279 (M+H-*t*Bu)⁺

### Step (c) 2-Chloro-N-(prop-2-yn-1-yl)-6-(trifluoromethyl)pyridin-4-amine

To *tert*-butyl (2-chloro-6-(trifluoromethyl)pyridin-4-yl)(prop-2-yn-1-yl)carbamate (502 mg, 1.5 mmol) dissolved in DCM (10 mL) was added TFA (2 mL). After stirring at r.t. for 5 h the mixture was cooled to 0°C followed by the addition of saturated NaHCO₃ solution and extraction with DCM. The combined organic layers were dried over MgSO₄ and concentrated under reduced pressure. Purification was done using automated column chromatography. LC-MS: m/z: 235 (M+H)⁺

### Step (d) 2-chloro-N-((1-(3-chloro-4-isopropoxyphenyl)-1H-1,2,3-triazol-4-yl)methyl)-6-(trifluoromethyl)pyridin-4-amine

2-Chloro-*N*-(prop-2-yn-1-yl)-6-(trifluoromethyl)pyridin-4-amine (23.4 mg, 0.1 mmol) was dissolved in *t*BuOH/H₂O (2 mL) followed by the addition of 4-azido-2-chloro-1-isopropoxybenzene (21.1 mg, 0.1 mmol), Na-ascorbate (1.9 mg, 0.01 mmol) and copper (II) sulfate pentahydrate (2.5 mg, 0.01 mmol). The reaction was stirred for 15 h under Argon atmosphere. Excess solvent was evaporated under reduced pressure and to the remaining residue, water (20 ml) was added and then extraction with ethyl acetate. The combined organic layers were washed with Brine then dried over MgSO₄ and evaporated under reduced pressure. Purification was done by preparative HPLC.

LC-MS: m/z: 446 (M+H)⁺. **¹H-NMR** (300 MHz, CDCl₃): δ [ppm] = 1.43 (d, *J =* 6.1 Hz, 6 H), 4.59 (d, *J* = 5.3 Hz, 2 H), 4.64 (sept, *J* = 6.1 Hz, 1 H), 5.38 (bs, 1 H), 6.69 (d, *J* = 2.0 Hz, 1 H), 6.86 (d, *J =* 2.0 Hz, 1 H), 7.07 (d, *J* = 8.9 Hz, 1 H), 7.56 (dd, *J* = 8.9 Hz, *J =* 2.6 Hz, 1 H), 7.74 (d, *J =* 2.6 Hz, 1 H), 7.85 (s, 1 H).

### 1.2 General procedures for preparation of reactants, intermediates and compounds of the invention

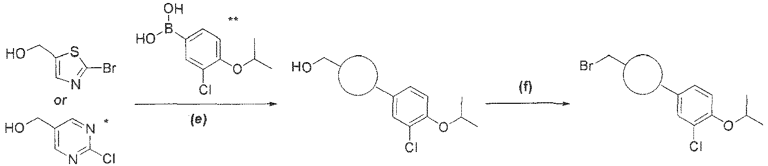

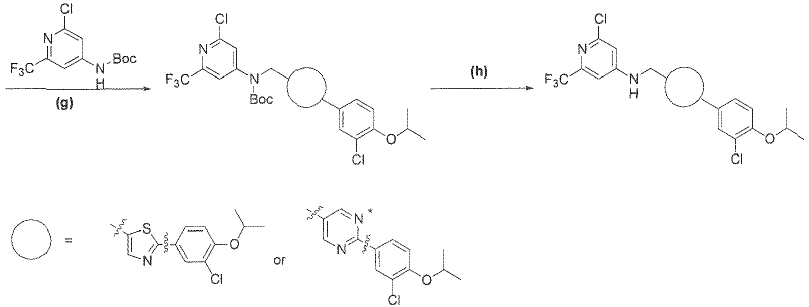

* example of 6-membered heterocyclic ring B
** example of boronic acid derivative

### General Step (e)

To 6-membered heterocyclic ring B reactant, e.g. (2-chloropyrimidin-5-yl)methanol, or (2-bromothiazol-5-yl)methanol (1 eq.); boronic acid derivative, e.g. (3-chloro-4-isopropoxyphenyl)boronic acid (1.2 eq.); Pd(dppf)Cl₂ (0.05 eq.) and Na₂CO₃ (4 eq.) was added 15 ml of the Dioxane/H₂O mixture. The reaction was refluxed at 110°C for 5 hours. Excess solvent was evaporated under reduced pressure and to the remaining residue, water (20 ml) was added and then extraction with ethyl acetate. The organic solvent was then dried over MgSO₄ and evaporated under reduced pressure. Purification was done using automated column chromatography.

### General Step (f)

To the desired methanol derivative (1 eq.) in DCM (20 ml) at 0_{°}C was added the Phosphorus tribromide (5 eq.). The reaction was stirred at room temp. for 18h. The reaction was then quenched by dropwise addition of NaHCO₃ at 0_{°}C followed by extraction with DCM. The organic solvent was then dried over MgSO₄ and concentrated under reduced pressure. Purification was done using automated column chromatography.

### General Step (g)

To *tert*-butyl (2-chloro-6-(trifluoromethyl)pyridin-4-yl)carbamate (1 eq.) in DMF (10 ml) at 0 °C was added NaH (60wt%) (1.2 eq.). The reaction was stirred for 30 min. followed by the addition of the desired bromomethyl derivative (1.2 eq.). The reaction mixture was stirred at room temp. for 2 hours. The reaction was then added to ice water followed by extraction with ethyl acetate. The combined organic layers were dried over MgSO₄ and under reduced pressure. Purification was done using automated column chromatography.

### General Step (h)

To the desired Boc-protected derivative (1 eq.) dissolved in DCM (10 mL) was added TFA (2 mL). After stirring at r.t. for 5 h the mixture was cooled to 0°C followed by the addition of saturated NaHCO₃ solution and extraction with DCM. The combined organic layers were dried over MgSO₄ and concentrated under reduced pressure. Purification was done using automated column chromatography.

### General Step (i)

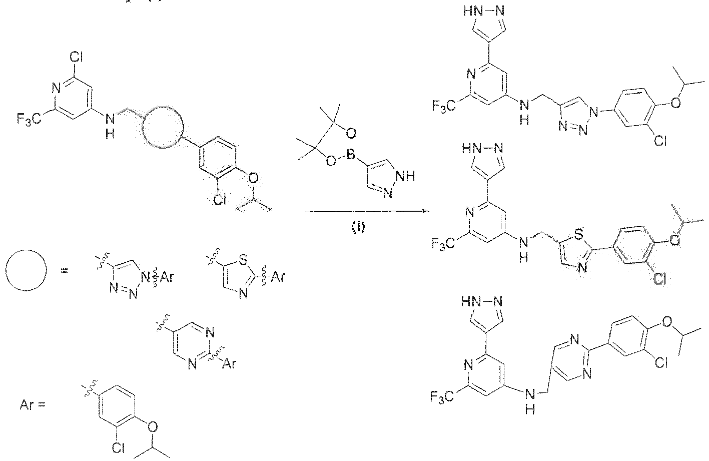

To the desired chloropyridine derivative (1 eq.), 4-(4,4,5,5-tetramethyl-1,3,2-dioxaborolan-2-yl)-1H-pyrazole (1.2 eq.) and Tetrakis (0.05 eq.) was added 15 ml of the dioxane/water mixture and Na₂CO₃ (4 eq.). The reaction was refluxed at 110°C for 5 hours during which excess 4-(4,4,5,5-tetramethyl-1,3,2-dioxaborolan-2-yl)-1H-pyrazole was added until the full reaction of the starting material. Excess solvent was evaporated under reduced pressure, then water (30 ml) was added and extraction with ethyl acetate. The organic solvent was then dried over MgSO₄ and concentrated in vacuo. Purification was done using preparative HPLC.

### General Step (j)

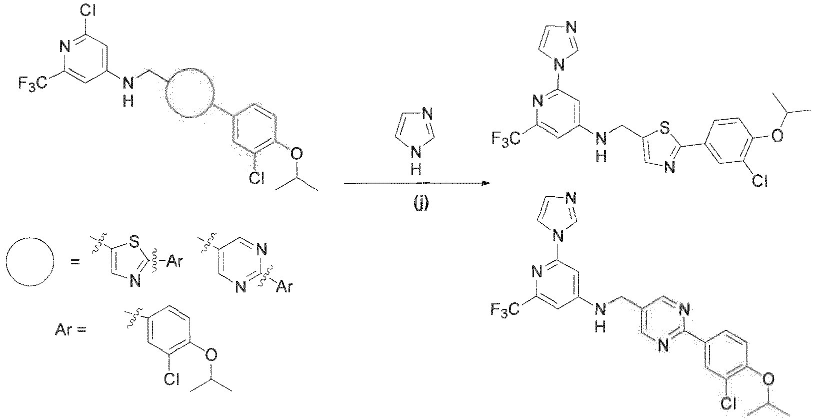

To a solution of the desired chloropyridine derivative (1 eq.), 1H-imidazole (1.5 eq.), cesium carbonate (2 eq.) in 1,4-dioxane (1 ml) was added Pd-175 (0.1 eq.). The reaction mixture was degassed and purged with N_{2(g)}(x3), after which the reaction mixture was stirred at 90°C for 1 h. The reaction mixture was filtered over celite and the filtrate was collected and concentrated in vacuo. The crude product was purified by preparative HPLC.

Compounds 01 to 60 described below are representative examples of compounds according to general formula (I) of the present invention which contain a group (L-1). These compounds have been synthesized based on the procedures described above.

### N-((1-(4-Phenoxyphenyl)-1H-1,2,3-triazol-4-yl)methyl)-2-(1H-pyrazol-4-yl)-6-(trifluoromethyl)pyridin-4-amine (01)

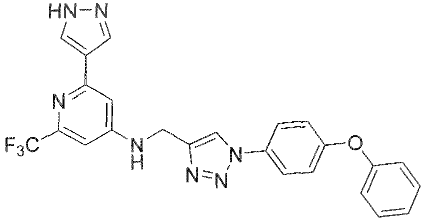

LC-MS: m/z: 478 (M+H)⁺. ¹H-NMR (500 MHz acetone-d₆): δ [ppm] = 4.71 (bs, 2 H), 6.70 (bs, 1H), 6.98 (d, *J* = 2.0 Hz, 1 H), 7.09 - 7.10 (m, 2 H), 7.16 - 7.18 (m, 2 H), 7.19 - 7.20 (m, 1 H), 7.22 -- 7.23 (m, 1 H), 7.42 - 7.45 (m, 2 H), 7.85 - 7.87 (m, 2 H), 8.15 (bs, 2 H), 8.53 (s, 1 H).

### 2-(1H-Imidazol-1-yl)-N-((1-(4-phenoxyphenyl)-1H-1,2,3-triazol-4-yl)methyl)-6-(trifluoromethyl)pyridin-4-amine (02)

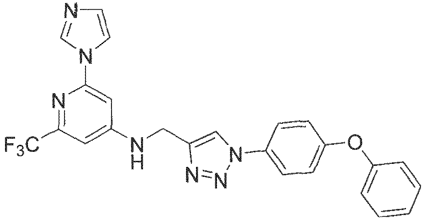

LC-MS: m/z: 478 (M+H)⁺. ¹H-NMR (500 MHz, DMSO-d₆): δ [ppm] = 4.79 (d, *J* = 5.7 Hz, 2 H), 7.09 (m, 1 H), 7.10 - 7.11 (m, 2 H), 7.15 (d, *J* = 1.7 Hz, 1 H), 7.16 - 7.19 (m, 2 H), 7.20 - 7.21 (m, 1 H), 7.23 - 7.25 (m, 2 H), 7.43 - 7.46 (m, 2 H), 7.83 (s, 1 H), 7.85 - 7.88 (m, 2 H), 8.40 (s, 1 H), 8.59 (s, 1 H).

### N-((1-(4-Phenoxyphenyl)-1H-1,2,3-triazol-4-yl)methyl)-2-(1H-1,2,4-triazol-1-yl)-6-(trifluoromethyl)pyridin-4-amine (03)

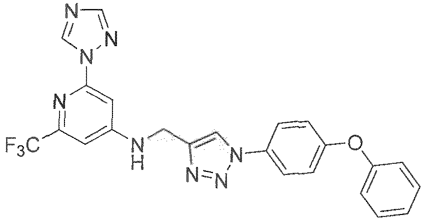

LC-MS: m/z: 479 (M+H)⁺. ¹H-NMR (500 MHz, DMSO-d₆): δ [ppm] = 4.79 (d, J= 5.7 Hz, 2 H), 7.08 - 7.09 (m, 1 H), 7.09 - 7.11 (m, 2 H), 7.16 - 7.19 (m, 2 H), 7.21 (s, 1 H), 7.22 (d, *J* = 2.1 Hz, 1 H), 7.35 (bs, 1 H), 7.42 - 7.46 (m, 2 H), 7.85 - 7.87 (m, 2 H), 8.13 (s, 1 H), 8.57 (s, 1 H), 9.15 (s, 1 H).

### N-((1-(4-(2-Fluorophenoxy)phenyl)-1H-1,2,3-triazol-4-yl)methyl)-2-(1H-pyrazol-4-yl)-6-(trifluoromethyl)pyridin-4-amine (04)

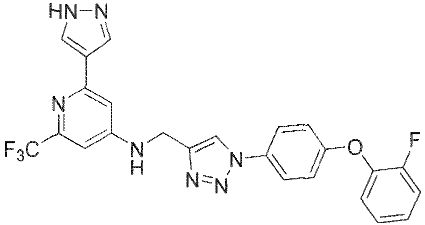

LC-MS: m/z: 496 (M+H)⁺. ¹H-NMR (300 MHz, CDCl₃): δ [ppm] = 4.59 (s, 2 H), 5.54 (bs, 1 H), 6.69 (s, 1 H), 6.87 (s, 1 H), 7.09 - 7.11 (m, 2 H), 7.15 - 7.25 (m, 5 H), 7.64 - 7.67 (m, 2 H), 7.88 (s, 1 H).

### N-((1-(4-Isopropoxyphenyl)-1H-1,2,3-triazol-4-yl)methyl)-2-(1H-pyrazol-4-yl)-6-(trifluoromethyl)pyridin-4-amine (05)

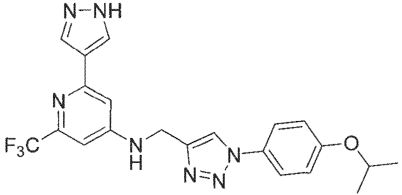

LC-MS: m/z: 444 (M+H)⁺. ¹H-NMR (500 MHz, CDCl₃): δ [ppm] = 1.36 (d, *J* = 6.0 Hz, 6 H), 4.61 (dq, *J* = 6.1 Hz, 1 H), 4.64 (d, *J* = 5.5 Hz, 2 H), 5.47 (bs, 1 H), 6.80 (d, *J* = 2.0 Hz, 1 H), 6.88 (s, 1 H), 6.98 - 7.00 (m, 2 H), 7.56 - 7.60 (m, 2 H), 7.88 (s, 1 H), 8.09 (s, 2 H).

### N-((1-(4-Isopropoxyphenyl)-1H-1,2,3-triazol-4-yl)methyl)-2-2,4-triazol-1-yl)-6-(trifluoromethyl)pyridin-4-amine (06)

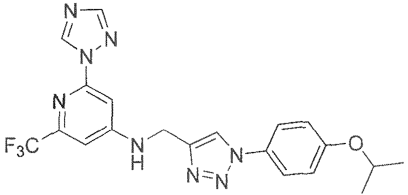

LC-MS: m/z: 445 (M+H)⁺. ¹H-NMR (500 MHz, DMSO-d₆): δ [ppm] = 1.32 (d, J= 6.0 Hz, 6 H), 4.70 (dq, *J* = 6.1 Hz, 1 H), 4.78 (d, *J* = 5.7 Hz, 2 H), 7.07 -7.11 (m, 2 H), 7.21 (d, *J =* 2.0 Hz, 1 H), 7.32 (bs, 1 H), 7.41 (d, *J* = 1.8 Hz, 1 H), 7.72 - 7.74 (m, 2 H), 8.13 (s, 1 H), 8.48 (s, 1 H), 9.15 (s, 1 H).

### N-((1-(3-Chloro-4-isopropoxyphenyl)-1H-1,2,3-triazol-4-yl)methyl)-2-(1H-pyrazol-4-yl)-6-(trifluoromethyl)pyridin-4-amine (07)

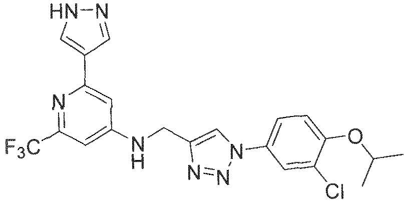

LC-MS: m/z: 478.07 (M+H)⁺. Mixture of tautomers by NMR, ratio = 1:0.15, shifts of major tautomer given:
¹H NMR (500 MHz, Acetone) δ 12.24 (s, 1H), 8.55 (s, 1H), 8.15 (s, 2H), 7.91 (d, *J* = 2.6 Hz, 1H), 7.77 (dd, *J* = 8.9, 2.6 Hz, 1H), 7.32 (d, *J* = 9.0 Hz, 1H), 7.22 (d, *J* = 1.7 Hz, 1H), 6.97 (d, *J* = 1.9 Hz, 1H), 6.73 (t, *J* = 5.4 Hz, 1H), 4.77 (hept, J= 6.0 Hz, 1H), 4.70 (d, *J* = 5.6 Hz, 2H), 1.37 (d, *J* = 6.*0* Hz, 6H).

### 2-Chloro-N-((1-(3-chloro-4-isopropoxyphenyl)-1H-1,2,3-triazol-4-yl)methyl)-6-(1H-pyrazol-4-yl)pyridin-4-amine (08)

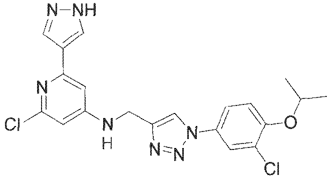

LC-MS: m/z: 444.2 (M+H)⁺. Mixture of tautomers by NMR, ratio = 1:0.15, shifts of major tautomer given:
¹H NMR (500 MHz, Acetone) δ 12.15 (s, 1H), 8.53 (s, 1H), 8.07 (s, 2H), 7.91 (d, *J* = 2.7 Hz, 1H), 7.77 (dd, *J* = 8.9, 2.7 Hz, 1H), 7.31 (d, *J* = 9.0 Hz, 1H), 7.01 (d, *J* = 1.8 Hz, 1H), 6.56 (d, *J =* 1.9 Hz, 2H), 4.77 (hept, *J* = 6.0 Hz, 1H), 4.63 (d, *J* = 5.7 Hz, 2H), 1.37 (d, *J* = 6.0 Hz, 6H).

### 4-(((1-(3-Chloro-4-isopropoxyphenyl)-1H-1,2,3-triazol-4-yl)methyl)amino)-6-(1H-pyrazol-4-yl)picolinonitrile (09)

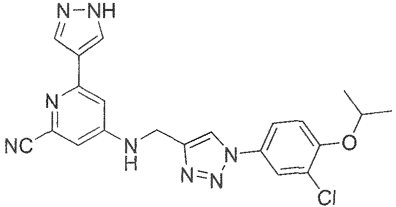

LC-MS: m/z: 435.2 (M+H)⁺. Mixture of tautomers by NMR, ratio = 1:0.15, shifts of major tautomer given:
¹H NMR (500 MHz, Acetone) δ 12.08 (s, 1H), 8.56 (s, 1H), 8.14 (s, 2H), 7.90 (d, *J* = 2.7 Hz, 1H), 7.77 (dd, *J* = 8.9, 2.7 Hz, 1H), 7.32 (d, *J* = 9.0 Hz, 1H), 7.25 (d, J= 2.2 Hz, 1H), 7.05 (d, *J* = 2.2 Hz, 1H), 6.81 (t, *J* = 5.5 Hz, 1H), 4.78 (hept, *J* = 6.0 Hz, 1H), 4.69 (d, *J* = 5.7 Hz, 2H), 1.37 (d, *J* = 6.0 Hz, 6H).

### 2-(1-Methyl-1H-pyrazol-4-yl)-N-((1-(4-phenoxyphenyl)-1H-1,2,3-triazol-4-yl)methyl)-6-(trifluoromethyl)pyridin-4-amine (10)

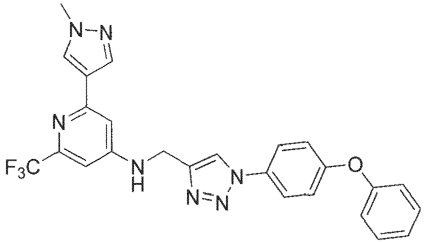

LC-MS: 492.12 (M+H)⁺.

### N-((2-(4-Isopropoxyphenyl)thiazol-5-yl)methyl)-2-(1H-pyrazol-4-yl)-6-(trifluoromethyl)pyridin-4-amine (11)

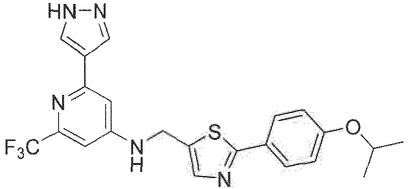

LC-MS: 460.3 (M+H)⁺. ¹H NMR (500 MHz, Acetone) δ 8.20 - 8.12 (m, 1H), 7.91 - 7.78 (m, 1H), 7.21 (d, *J* = 5.9 Hz, 1H), 7.04 - 6.91 (m, 1H), 6.85 (s, 1H), 4.85 (d, *J* = 5.9 Hz, 1H), 4.76 - 4.61 (m, 1H), 1.37 - 1.25 (m, 1H).

### 4-(((2-(4-Isopropoxyphenyl)thiazol-5-yl)methyl)amino)-6-(1H-pyrazol-4-yl)picolinonitrile (12)

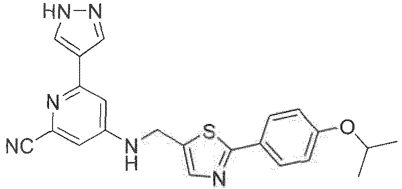

LC-MS: 417.3 (M+H)⁺. ¹H NMR (500 MHz, Acetone) δ 8.15 (s, 2H), 7.89 - 7.77 (m, 3H), 7.24 (dd, *J* = 4.4, 2.2 Hz, 1H), 7.04 (dd, *J* = 7.5, 2.2 Hz, 1H), 7.01 - 6.93 (m, 3H), 4.92 - 4.78 (m, 2H), 4.69 (dq, *J* = 12.0, 6.0 Hz, 1H), 1.32 (d, *J* = 6.0 Hz, 6H).

### N-((2-(4-Isopropoxyphenyl)thiazol-5-yl)methyl)-2-(1-methyl-1H-pyrazol-4-yl)-6-(trifluoromethyl)pyridin-4-amine (13)

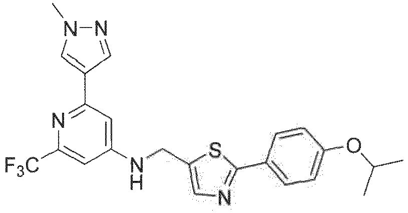

LC-MS: 474.2 (M+H)⁺. ¹H NMR (500 MHz, CDCl₃) δ 7.88 (s, 1H), 7.79 (s, 1H), 7.77 - 7.70 (m, 2H), 7.61 (s, 1H), 6.87 - 6.84 (m, 1H), 6.84 - 6.82 (m, 1H), 6.70 (d, *J* = 2.0 Hz, 1H), 6.66 (d, J= 2.1 Hz, 1H), 4.56 (d, J= 5.6 Hz, 2H), 4.52 (dt, *J* = 12.1, 6.1 Hz, 1H), 3.85 (s, 3H), 1.28 (d,*J* = 6.1 Hz, 6H).

### N-((2-(4-Isopropoxyphenyl)thiazol-5-yl)methyl)-2-(1H-pyrazol-3-yl)-6-(trifluoromethyl)pyridin-4-amine (14)

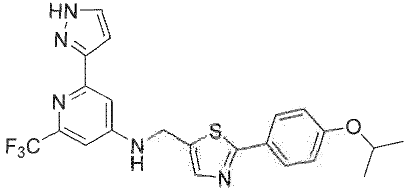

LC-MS: 460.2 (M+H)⁺. ¹H NMR (500 MHz, MeOD) δ 8.02 (s, br., 4H), 7.78 - 7.56 (m, 3H), 6.90 - 6.75 (m, 2H), 6.71 (s, 2H), 4.61 (s, 2H), 4.51 (dt, *J* = 12.1, 6.0 Hz, 1H), 1.19 (d, *J* = 6.0 Hz, 6H).

### 2-(1H-Imidazol-1-yl)-N-((2-(4-isopropoxyphenyl)thiazol-5-yl)methyl)-6-(trifluoromethyl)pyridin-4-amine (15)

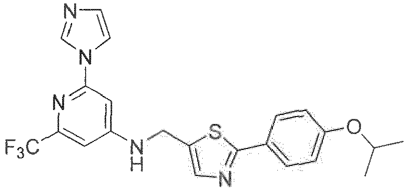

LC-MS: 460.2 (M+H)⁺. ¹H NMR (500 MHz, Acetone) δ 8.41 (s, 1H), 7.89 - 7.82 (m, 4H), 7.33 (s, 1H), 7.21 (s, 1H), 7.13 (d, *J* = 1.0 Hz, 1H), 7.04 - 6.93 (m, 2H), 4.93 (d, *J* = 5.7 Hz, 2H), 4.70 (dt, *J* = 12.1, 6.0 Hz, 1H), 1.32 (d, *J* = 6.0 Hz, 7H).

### N-((2-(3-Chloro-4-isopropoxyphenyl)thiazol-5-yl)methyl)-2-(1H-imidazol-1-yl)-6-(trifluoromethyl)pyridin-4-amine (16)

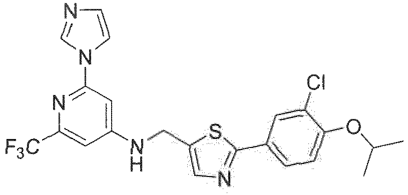

LC-MS: 494.0 (M+H)⁺. ¹H NMR (500 MHz, Acetone) δ 8.27 (s, 1H), 7.81 (d, *J =* 2.2 Hz, 1H), 7.74 (s, 1H), 7.68 (s, 1H), 7.65 (dd, *J* = 8.6, 2.2 Hz, 1H), 7.19 (t, *J* = 5.5 Hz, 1H), 7.05 (dd, J = 5.1, 3.5 Hz, 2H), 6.98 (d, *J* = 1.8 Hz, 1H), 6.95 (s, 1H), 4.80 (d, *J* = 5.8 Hz, 2H), 4.67 - 4.55 (m, 1H), 1.22 (d, *J* = 6.0 Hz, 6H).

### N-((2-(4-Isopropoxyphenyl)thiazol-5-yl)methyl)-2-(1H-1,2,4-triazol-1-yl)-6-(trifluoromethyl)pyridin-4-amine (17)

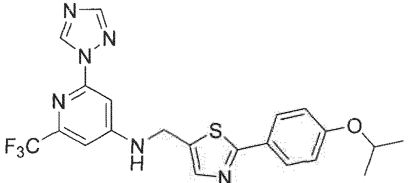

LC-MS: 461.2 (M+H)⁺. ¹H NMR (500 MHz, Acetone) δ 9.16 (s, 1H), 8.13 (s, 1H), 7.93 - 7.75 (m, 1H), 7.40 (d, *J* = 8.0 Hz, 1H), 7.19 (d, *J* = 1.9 Hz, 1H), 7.07 - 6.92 (m, 1H), 4.94 (d, *J* = 5.8 Hz, 1H), 4.78 - 4.60 (m, 1H), 1.32 (d, *J* = 6.0 Hz, 1H).

### N-((2-(3-Chloro-4-isopropoxyphenyl)thiazol-5-yl)methyl)-2-(1H-1,2,4-triazol-1-yl)-6-(trifluoromethyl)pyridin-4-amine (18)

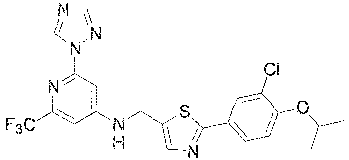

LC-MS: 495.2 (M+H)⁺. ¹H NMR (500 MHz, Acetone) δ 9.01 (s, 1H), 7.99 (s, 1H), 7.81 (d, *J* = 2.2 Hz, 1H), 7.73 (s, 1H), 7.64 (dd, *J* = 8.6, 2.2 Hz, 1H), 7.30 (t, *J* = 5.7 Hz, 1H), 7.24 (d, *J* = 1.8 Hz, 1H), 7.08 - 7.00 (m, 2H), 4.81 (d, *J* = 5.6 Hz, 2H), 4.61 (hept, *J* = 6.0 Hz, 1H), 1.22 (d, *J* = 6.0 Hz, 6H).

### N-((2-(4-Isopropoxyphenyl)thiazol-5-yl)methyl)-2-(2H-tetrazol-5-yl)-6-(trifluoromethyl)pyridin-4-amine (19)

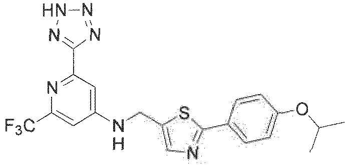

LC-MS: 462.2 (M+H)⁺. ¹H NMR (500 MHz, Acetone) δ 7.89 - 7.82 (m, 3H), 7.79 (d, *J* = 2.1 Hz, 1H), 7.43 (t, *J* = 5.5 Hz, 1H), 7.28 (d, *J* = 2.2 Hz, 1H), 7.03 - 6.94 (m, 2H), 4.96 (d, *J* = 5.8 Hz, 2H), 4.76 - 4.62 (m, 1H), 1.32 (d, *J=* 6.0 Hz, 6H).

### N-((5-(4-Isopropoxyphenyl)pyridin-2-yl)methyl)-2-(1H-pyrazol-4-yl)-6-(trifluoromethyl)pyridin-4-amine (20)

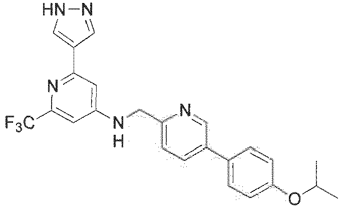

LC-MS: 454.4 (M+H)⁺. ¹H NMR (500 MHz, DMSO-d6) δ 13.03 (s, 1H), 8.83 (dd, J= 2.5, 0.8 Hz, 1H), 8.15 -8.04 (m, 2H), 8.02 (dd, J= 8.2, 2.4 Hz, 1H), 7.69 -7.59 (m, 3H), 7.45 (dd, J= 8.1, 0.8 Hz, 1H), 7.06 (d, J= 2.0 Hz, 1H), 7.04 -7.00 (m, 2H), 6.92 -6.88 (m, 1H), 4.67 (hept, J= 6.1 Hz, 1H), 4.60 (d, J= 5.9 Hz, 2H), 1.29 (d, J= 6.0 Hz, 6H).

### N-((6-(4-Isopropoxyphenyl)pyridin-3-yl)methyl)-2-(1H-pyrazol-4-yl)-6-(trifluoromethyl)pyridin-4-amine (21)

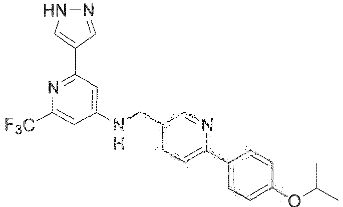

LC-MS: 454.6 (M+H)⁺. ¹H NMR (500 MHz, DMSO-d6) δ 13.05 (s, 1H), 8.66 (d, J= 2.4 Hz, 1H), 8.20 -8.03 (m, 2H), 8.03 -7.96 (m, 2H), 7.88 (dd, J= 8.2, 0.9 Hz, 1H), 7.82 (dd, J= 8.3, 2.3 Hz, 1H), 7.56 (t, J= 5.9 Hz, 1H), 7.05 (d, J= 2.0 Hz, 1H), 7.03 -6.97 (m, 2H), 6.86 (d, J= 2.0 Hz, 1H), 4.68 (hept, J= 6.0 Hz, 1H), 4.52 (d, J= 5.8 Hz, 2H), 1.29 (d, J= 6.1 Hz, 6H).

### N-((6-(4-Isopropoxyphenyl)pyridazin-3-yl)methyl)-2-(1H-pyrazol-4-yl)-6-(trifluoromethyl)pyridin-4-amine (22)

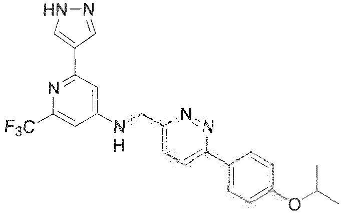

LC-MS: 455.3 (M+H)⁺. ¹H NMR (500 MHz, DMSO-d6) δ 13.06 (s, 1H), 8.33 -7.84 (m, 5H), 7.79 -7.60 (m, 2H), 7.17 -7.03 (m, 3H), 6.92 (d, J= 2.1 Hz, 1H), 4.81 (d, J= 6.1 Hz, 2H), 4.72 (hept, J= 6.1 Hz, 1H), 1.31 (d, J= 6.0 Hz, 6H).

### N-((6-(3-Chloro-4-isopropoxyphenyl)pyridin-3-yl)methyl)-2-(1H-pyrazol-4-yl)-6-(trifluoromethyl)pyridin-4-amine (23)

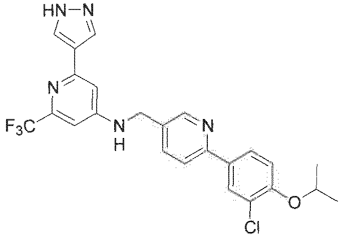

LC-MS: 488.3 (M+H)⁺. ¹H NMR (500 MHz, DMSO-d6) δ 13.06 (s, 1H), 8.68 (d, J= 2.3 Hz, 1H), 8.17 -8.04 (m, 3H), 8.00 (dd, J= 8.7, 2.3 Hz, 1H), 7.96 (dd, J= 8.2, 0.9 Hz, 1H), 7.85 (dd, J= 8.3, 2.3 Hz, 1H), 7.57 (t, J= 6.0 Hz, 1H), 7.26 (d, J= 8.9 Hz, 1H), 7.05 (d, J= 2.0 Hz, 1H), 6.86 (d, J= 2.0 Hz, 1H), 4.75 (hept, J= 6.0 Hz, 1H), 4.54 (d, J= 5.9 Hz, 2H), 1.33 (d, J= 6.0 Hz, 6H).

### N-((5-(3-Chloro-4-isopropoxyphenyl)pyridin-2-yl)methyl)-2-(1H-pyrazol-4-yl)-6-(trifluoromethyl)pyridin-4-amine (24)

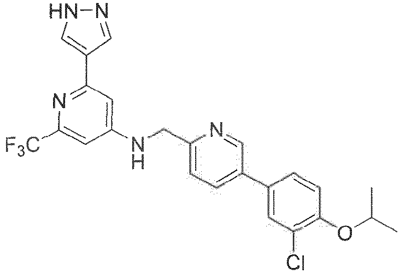

LC-MS: 489.3 (M+H)⁺. ¹H NMR (500 MHz, DMSO-d6) δ 13.05 (s, 1H), 8.87 (d, J= 2.4 Hz, 1H), 8.26 -8.19 (m, 1H), 8.07 (dd, J*=* 8.2, 2.5 Hz, 1H), 8.01 -7.90 (m, 1H), 7.81 (d, J= 2.4 Hz, 1H), 7.70 -7.60 (m, 2H), 7.46 (d, J= 8.2 Hz, 1H), 7.28 (d, J= 8.7 Hz, 1H), 7.06 (s, 1H), 6.94 -6.86 (m, 1H), 4.74 (hept, J= 6.0 Hz, 1H), 4.61 (d, J= 5.4 Hz, 2H), 1.32 (d, J= 6.0 Hz, 6H).

### N-((5-(4-Isopropoxyphenyl)pyridin-2-yl)methyl)-2-(1H-1,2,4-triazol-1-yl)-6-(trifluoromethyl)pyridin-4-amine (25)

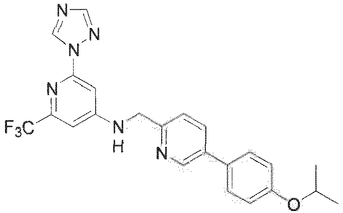

LC-MS: 455.4 (M+H)⁺. ¹H NMR (500 MHz, DMSO-d6) δ 9.20 (s, 1H), 8.84 (d, J= 2.4 Hz, 1H), 8.34 -8.25(m, 2H), 8.04 (dd, J= 8.2, 2.4 Hz, 1H), 7.71 -7.61 (m, 2H), 7.46 (d, J= 8.2 Hz, 1H), 7.17 (br s, 2H), 7.07 -6.99 (m, 2H), 4.72 -4.61 (m, 3H), 1.29 (d, J= 6.0 Hz, 6H).

### N-((5-(3-Chloro-4-isopropoxyphenyl)pyridin-2-yl)methyl)-2-(1H-1,2,4-triazol-1-yl)-6-(trifluoromethyl)pyridin-4-amine (26)

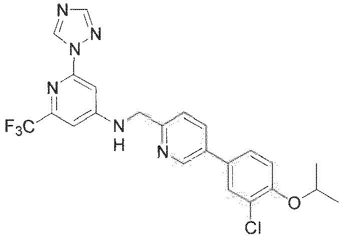

LC-MS: 489.4/491.4 (M+H)⁺. ¹H NMR (500 MHz, DMSO-d6) δ 9.20 (s, 1H), 8.88 (dd, J= 2.4, 0.8 Hz, 1H), 8.31 (t, J= 5.9 Hz, 1H), 8.27 (s, 1H), 8.09 (dd, J= 8.1, 2.4 Hz, 1H), 7.82 (d, J= 2.3 Hz, 1H), 7.66 (dd, J= 8.6, 2.4 Hz, 1H), 7.47 (dd, J= 8.2, 0.8 Hz, 1H), 7.28 (d, J= 8.8 Hz, 1H), 7.16 (br s, 2H), 4.74 (hept, J= 6.0 Hz, 1H), 4.69 -4.63 (m, 2H), 1.32 (d, J= 6.0 Hz, 6H).

### N-((5-(4-Isopropoxyphenyl)pyrazin-2-yl)methyl)-2-(1H-pyrazol-4-yl)-6-(trifluoromethyl)pyridin-4-amine (27)

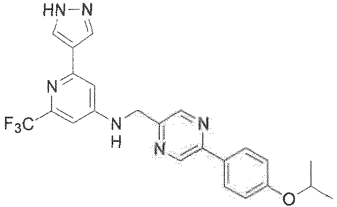

LC-MS: 455.4 (M+H)⁺. ¹H NMR (500 MHz, DMSO-d6) δ 13.06 (s, 1H), 9.16 (d, J= 1.5 Hz, 1H), 8.71 (d, J= 1.5 Hz, 1H), 8.31 -7.89 (m, 4H), 7.63 (t, J= 6.1 Hz, 1H), 7.10 (d, J= 2.1 Hz, 1H), 7.07 -7.02 (m, 2H), 6.91 (d, J= 2.0 Hz, 1H), 4.72 (hept, J= 6.0 Hz, 1H), 4.66 (d, J= 6.0 Hz, 2H), 1.30 (d, J= 6.0 Hz, 6H).

### N-((2-(4-Isopropoxyphenyl)pyrimidin-5-yl)methyl)-2-(1H-pyrazol-4-yl)-6-(trifluoromethyl)pyridin-4-amine (28)

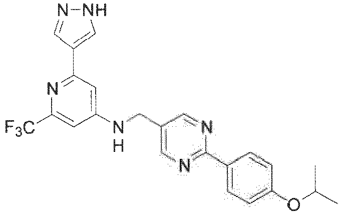

LC-MS: 455.2 (M+H)⁺. Mixture of tautomers by NMR, ratio = 1:0.15, shifts of major tautomer given:
¹H NMR (500 MHz, Acetone) δ 8.88 (s, 2H), 8.39 (d, *J* = 8.8 Hz, 2H), 8.14 (s, 2H), 7.17 (d, *J* = 1.9 Hz, 1H), 7.01 (d, *J* = 8.9 Hz, 2H), 6.94 (d, *J* = 2.0 Hz, 1H), 6.83 (t, *J* = 5.7 Hz, 1H), 4.74 (hept, *J* = 6.0 Hz, 1H), 4.68 (d, *J* = 5.9 Hz, 2H), 1.33 (d, *J* = 6.0 Hz, 6H).

### 2-(1H-Imidazol-1-yl)-N-((2-(4-isopropoxyphenyl)pyrimidin-5-yl)methyl)-6-(trifluoromethyl)pyridin-4-amine (29)

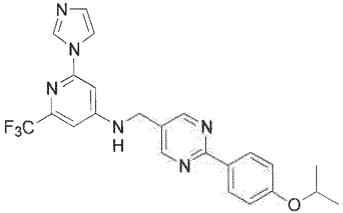

LC-MS: 455.4 (M+H)⁺. ¹H NMR (500 MHz, DMSO-d6) δ8.88 (s, 2H), 8.48 -4.45(m, 1H), 8.35 -8.27 (m, 2H), 7.98 (t, J= 5.8 Hz, 1H), 7.90 -7.87(m, 1H), 7.17 -7.09 (m, 2H), 7.08 -7.00 (m, 3H), 4.72 (hept, J= 6.0 Hz, 1H), 4.65 -4.56 (m, 2H), 1.30 (d, J= 6.0 Hz, 6H).

### N-((2-(3-Chloro-4-isopropoxyphenyl)pyrimidin-5-yl)methyl)-2-(1H-imidazol-1-yl)-6-(trifluoromethyl)pyridin-4-amine (30)

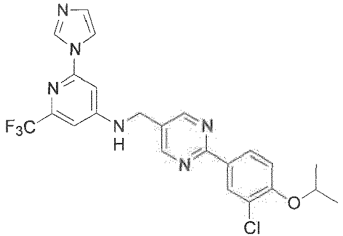

LC-MS: 489.3 (M+H)⁺. ¹H NMR (500 MHz, DMSO-d6) δ 8.91 (s, 2H), 8.49 -8.44(m, 1H), 8.36 (d, J= 2.2 Hz, 1H), 8.30 (dd, J= 8.7, 2.2 Hz, 1H), 8.00 (t, J= 5.8 Hz, 1H), 7.91 -7.86(m, 1H), 7.32 (d, J= 8.8 Hz, 1H), 7.11(d, J=1.6 Hz, 2H), 7.05 (d, J= 1.6 Hz, 1H),4.80 (hept, J=6.1 Hz, 1H), 4.68 -4.55 (m, 2H), 1.34 (d, J= 6.0 Hz, 6H).

### N-((2-(4-Isopropoxyphenyl)pyrimidin-5-yl)methyl)-2-(1H-pyrazol-3-yl)-6-(trifluoromethyl)pyridin-4-amine (31)

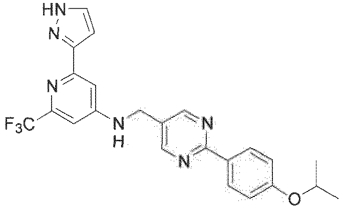

LC-MS: 455.2 (M+H)⁺. Mixture of tautomers by NMR, ratio = 1:0.2, shifts of major tautomer given:
¹H NMR (500 MHz, DMSO-d6) δ 13.04 (s, 1H), 8.86 (s, 2H), 8.31 (d, J= 9.0 Hz, 2H), 7.81 - 7.77(m, 1H), 7.66 (t, J= 5.8 Hz, 1H),7.40 -7.34(m, 1H), 7.09 -7.01 (m, 2H), 6.98 (d, J= 2.2 Hz, 1H), 6.77 -6.72(m, 1H), 4.72 (hept, J= 6.0 Hz, 1H), 4.54 (d, J= 5.5 Hz, 2H), 1.30 (d, J= 6.0 Hz, 6H).

### N-((2-(3-Chloro-4-isopropoxyphenyl)pyrimidin-5-yl)methyl)-2-(1H-pyrazol-3-yl)-6-(trifluoromethyl)pyridin-4-amine (32)

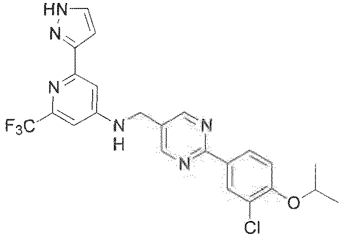

LC-MS: 490.3 (M+H)⁺. ¹H NMR (500 MHz, DMSO-d6) δ 13.04 (s, 1H), 8.89 (s, 2H), 8.36 (d, J= 2.2 Hz, 1H), 8.30 (dd,J= 8.7, 2.2 Hz, 1H), 7.83 -7.76(m, 1H), 7.72 -7.65(m, 1H), 7.40 - 7.35(m, 1H), 7.32 (d, J= 8.9 Hz, 1H), 7.01 -6.97(m, 1H), 6.75 -6.71 (m, 1H), 4.86 -4.74 (m, 1H), 4.56 (d, J= 5.5 *Hz,* 2H), 1.34 (d, J= 6.0 Hz, 6H).

### N-((2-(4-Chlorophenyl)pyrimidin-5-yl)methyl)-2-(1H-pyrazol-4-yl)-6-(trifluoromethyl)pyridin-4-amine (33)

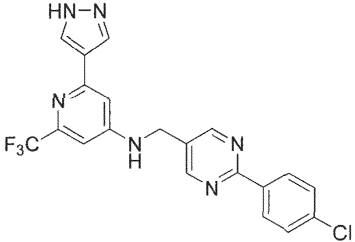

LC-MS: 431.4/433.4 (M+H)⁺. ¹H NMR (500 MHz, DMSO-d6) δ 13.07 (s, 1H), 8.95 (s, 2H), 8.43 -8.34 (m, 2H), 8.26 (s, 1H), 8.02 -7.94 (m, 1H), 7.63 -7.54 (m, 3H), 7.07 (d, J= 2.0 Hz, 1H), 6.88 (d, J= 2.0 Hz, 1H), 4.57 (d, J= 5.8 Hz, 2H).

### N-((2-(4-Chloro-3-fluorophenyl)pyrimidin-5-yl)methyl)-2-(1H-pyrazol-4-yl)-6-(trifluoromethyl)pyridin-4-amine (34)

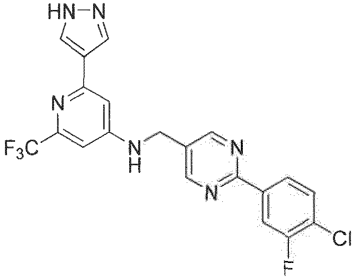

LC-MS: 449.4/451.4 (M+H)⁺. ¹H NMR (500 MHz, DMSO-d6) δ 13.07 (s, 1H), 8.98 (s, 2H),8.30 -8.18 (m, 3H),7.99 (s, 1H),7.76 (td, J= 8.0, 1.0 Hz, 1H),7.59 (t, J= 6.0 Hz, 1H),7.06 (d, J= 2.0 Hz, 1H),6.88 (d, J= 2.0 Hz, 1H),4.66 -4.52 (m, 2H)

### N-((2-(3,4-Dichlorophenyl)pyrimidin-5-yl)methyl)-2-(1H-pyrazol-4-yl)-6-(trifluoromethyl)pyridin-4-amine (35)

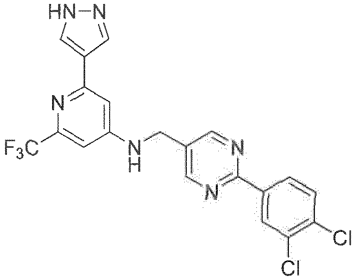

LC-MS: 465.3/467.3/469.3 (M+H)⁺. ¹H NMR (500 MHz, DMSO-d6) δ 13.07 (s, 1H), 8.98 (s, 2H),8.51 (d, J= 2.0 Hz, 1H), 8.33 (dd, J= 8.5, 2.0 Hz, 1H), 8.26 (s, 1H), 7.99 (s, 1H), 7.80 (d, J= 8.5 Hz, 1H), 7.60 (t, J= 6.0 Hz, 1H), 7.07 (d, J= 2.0 Hz, 1H), 6.88 (d, J= 2.0 Hz, 1H), 4.59 (d, J= 5.9 Hz, 2H).

### 3-(5-(((2-(1H-Pyrazol-4-yl)-6-(trifluoromethyl)pyridin-4-yl)amino)methyl)pyrimidin-2-yl)benzonitrile (36)

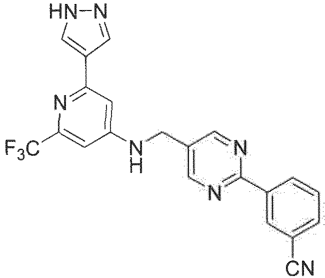

LC-MS: 422.4 (M+H)⁺. ¹H NMR (500 MHz, DMSO-d6) δ 13.07 (s, 1H), 9.00 (d, J= 1.4 Hz, 2H), 8.68 (dtd, J= 11.5, 3.4, 1.7 Hz, 2H), 8.26 (s, 1H), 8.01 (dtd, J= 6.8, 4.2, 3.6, 2.0 Hz, 2H), 7.81 -7.72 (m, 1H), 7.61 (t, J= 6.0 Hz, 1H), 7.07 (d, J= 2.0 Hz, 1H), 6.89 (d, J= 2.0 Hz, 1H), 4.61 (d, J= 5.7 Hz, 2H).

### 5-(5-(((2-(1H-Pyrazol-4-yl)-6-(trfluoromethyl)pyridin-4-yl)amino)methyl)pyrimidin-2-yl)-2-methylbenzonitrile (37)

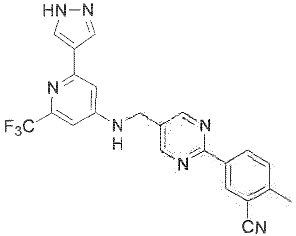

LC-MS: 436.4 (M+H)⁺. ¹H NMR (500 MHz, DMSO-d6) δ 13.07 (s, 1H), 8.97 (s, 2H), 8.62 (d, J = 1.9 Hz, 1H), 8.55 (dd, J= 8.1, 1.9 Hz, 1H), 8.26 (s, 1H), 7.99 (s, 1H), 7.64 (dt, J= 8.2, 0.7 Hz, 1H), 7.59 (t, J= 6.0 Hz, 1H), 7.07 (d, J= 2.0 Hz, 1H), 6.88 (d, J= 2.1 Hz, 1H), 4.59 (d, J= 5.9 Hz, 2H), 2.56 (s, 3H).

### 5-(5-(((2-(1H-Pyrazol-4-yl)-6-(trifluoromethyl)pyridin-4-yl)amino)methyl)pyrimidin-2-yl)-2-fluorobenzonitrile (38)

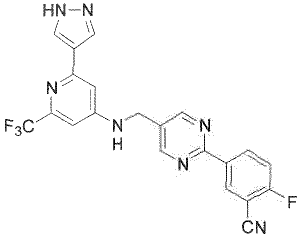

LC-MS: 440.4 (M+H)⁺. ¹H NMR (500 MHz, DMSO-d6) δ 13.07 (s, 1H), 8.99 (s, 2H), 8.83 - 8.66 (m, 2H), 8.26 (s, 1H), 7.99 (s, 1H), 7.70 (t, J= 9.0 Hz, 1H), 7.60 (t, J= 6.0 Hz, 1H), 7.07 (d, J= 2.0 Hz, 1H), 6.88 (d, J= 2.0 Hz, 1H), 4.69 -4.51 (m, 2H).

### 5-(5-(((2-(1H-1,2,4-Triazol-1-yl)-6-(trifluoromethyl)pyridin-4-yl)amino)methyl)pyrimidin-2-yl)-2-chlorobenzonitrile (39)

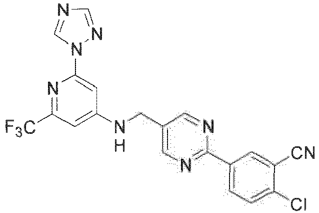

LC-MS: 457.4/459.4 (M+H)⁺. ¹H NMR (500 MHz, DMSO-d6) δ 9.22 (s, 1H), 9.00 (s, 2H), 8.79 (d, J= 2.1 Hz, 1H), 8.66 (dd, J= 8.6, 2.2 Hz, 1H), 8.29 (s, 1H), 8.21 (t, J= 5.8 Hz, 1H), 7.93 (d, J= 8.6 Hz, 1H), 7.25 (br s, 1H), 7.14 (d, J= 2.0 Hz, 1H), 4.74 -4.63 (m, 2H).

### N-((5-(4-Isopropoxyphenyl)pyrimidin-2-yl)methyl)-2-(1H-pyrazol-4-yl)-6-(trifluoromethyl)pyridin-4-amine (40)

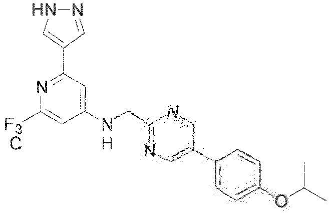

LC-MS: 455.5 (M+H)⁺. ¹H NMR (500 MHz, DMSO-d6) δ 13.04 (s, 1H), 9.09 (s, 2H), 8.22 (s, 1H), 7.95 (s, 1H), 7.79 -7.67 (m, 2H), 7.59 (t, J= 6.0 Hz, 1H), 7.14 -7.01 (m, 3H), 6.95 (s, 1H), 4.80 -4.64 (m, 3H), 1.29 (d, J= 6.0 Hz, 6H).

### 2-(1H-Imidazol-5-yl)-N-((2-(4-isopropoxyphenyl)pyrimidin-5-yl)methyl)-6-(trifluoromethyl)pyridin-4-amine (41)

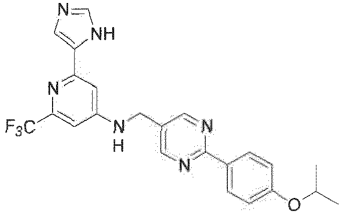

LC-MS: 455.4 (M+H)⁺. ¹H NMR (500 MHz, DMSO-d6) δ 12.32 (s, 1H), 8.85 (s, 2H), 8.38 - 8.25 (m, 2H), 7.71 (t, J= 1.2 Hz, 1H), 7.65 -7.55 (m,2H), 7.32 (d, J= 2.3 Hz, 1H), 7.07 -7.00 (m, 2H), 6.87 (d, J= 2.2 Hz, 1H), 4.71 (app pd, J= 6.0, 1.6 Hz, 1H), 4.52 (d, J= 5.5 Hz, 2H), 1.30 (dd, J= 6.0, 1.3 Hz, 6H).

### N-((5-(4-Isopropoxyphenyl)pyrimidin-2-yl)methyl)-2-(1H-1,2,4-triazol-1-yl)-6-(trifluoromethyl)pyridin-4-amine (42)

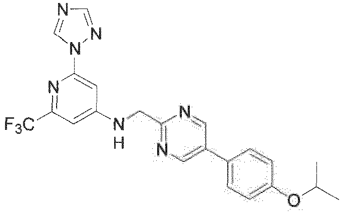

LC-MS: 456.7 (M+H)⁺. ¹H NMR (500 MHz, DMSO-d6) δ 9.21 (s, 1H), 9.10 (s, 2H), 8.33 (t, J= 6.2 Hz, 1H), 8.27 (s, 1H), 7.77 -7.69 (m, 2H), 7.39 -6.96 (m, 4H), 4.77 (d, J= 6.2 Hz, 2H), 4.70 (hept, J= 6.0 Hz, 1H), 1.29 (d, J= 6.0 Hz, 6H).

### N-((5-(3-Chloro-4-isopropoxyphenyl)pyrimidin-2-yl)methyl)-2-(1H-1,2,4-triazol-1-yl)-6-(trifluoromethyl)pyridin-4-amine (43)

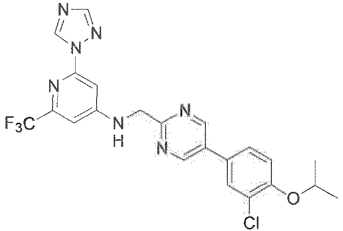

LC-MS: 490.4/492.4 (M+H)⁺. ¹H NMR (500 MHz, DMSO-d6) δ 9.21 (s, 1H), 9.14 (s, 2H), 8.34 (t, J= 6.2 Hz, 1H), 8.27 (s, 1H), 7.95 (d, J= 2.4 Hz, 1H), 7.75 (dd, J= 8.6, 2.3 Hz, 1H), 7.31 (d, J= 8.8 Hz, 2H), 7.19 (br s, 1H), 4.81 -4.72 (m, 3H), 1.32 (d, J= 6.0 Hz, 6H).

### N-((5-(4-Isopropoxyphenyl)pyrimidin-2-yl)methyl)-2-(1-methyl-1H-pyrazol-4-yl)-6-(trifluoromethyl)pyridin-4-amine (44)

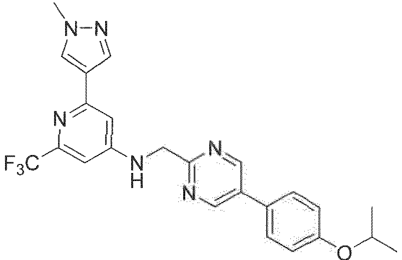

LC-MS: 469.5 (M+H)⁺. ¹H NMR (500 MHz, DMSO-d6) δ 8.87 (s, 2H), 8.33 -8.28 (m, 2H), 8.23 (s, 1H), 7.94 (s, 1H), 7.57 (t, J= 5.9 Hz, 1H), 7.06 -7.00 (m, 3H), 6.87 (d, J= 2.0 Hz, 1H), 4.71 (hept, J= 6.0 Hz, 1H), 4.53 (d, J= 5.7 Hz, 2H), 3.87 (s, 3H), 1.30 (d, J= 6.0 Hz, 6H).

### 2-(1H-Imidazol-1-yl)-N-((5-(4-isopropoxyphenyl)pyrimidin-2-yl)methyl)-6-(trifluoromethyl)pyridin-4-amine (45)

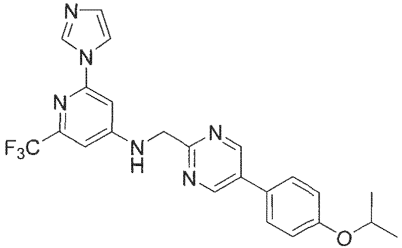

LC-MS: 456.5 (M+H)⁺. ¹H NMR (500 MHz, DMSO-d6) δ 9.10 (s, 2H), 8.43 (s, 1H), 8.02 (t, J= 6.0 Hz, 1H), 7.86 (s, 1H), 7.78 -7.67 (m, 2H), 7.26 -6.94 (m, 5H), 4.90 -4.76 (m, 2H), 4.70 (hept, J= 6.0 Hz, 1H), 1.29 (d, J= 6.0 Hz, 6H).

### N-((5-(3-Chloro-4-isopropoxyphenyl)pyrimidin-2-yl)methyl)-2-(1H-imidazol-1-yl)-6-(trifluoromethyl)pyridin-4-amine (46)

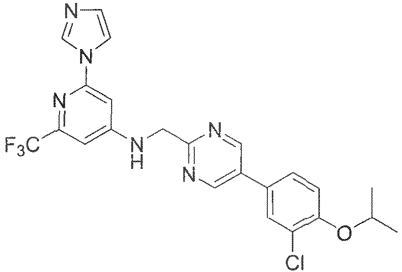

LC-MS: 489.8/491.5 (M+H)⁺. ¹H NMR (500 MHz, DMSO-d6) δ 9.14 (s, 2H), 8.43 (s, 1H), 8.03 (t, J= 5.9 Hz, 1H), 7.95 (d, J= 2.3 Hz, 1H), 7.86 (s, 1H), 7.75 (dd, J= 8.6, 2.3 Hz, 1H), 7.31 (d, J= 8.8 Hz, 1H), 7.24 -7.06 (m, 3H), 4.82 (d, J= 5.9 Hz, 2H), 4.76 (hept, J= 6.0 Hz, 1H), 1.32 (d, J= 6.0 Hz, 6H).

### N-((5-(3-Chloro-4-isopropoxyphenyl)pyrimidin-2-yl)methyl)-2-(1H-pyrazol-4-yl)-6-(trifluoromethyl)pyridin-4-amine (47)

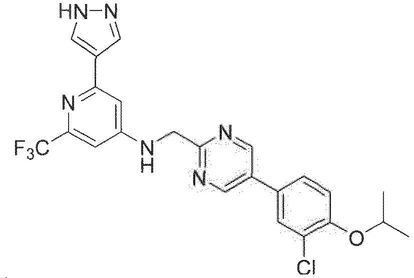

LC-MS: 489.4 (M+H)⁺. ¹H NMR (500 MHz, DMSO-d6) δ 13.04 (s, 1H), 9.14 (s, 2H), 8.22 (s, 1H), 7.94 (d, J= 2.4 Hz, 2H), 7.74 (dd, J= 8.6, 2.3 Hz, 1H), 7.60 (t, J= 6.1 Hz, 1H), 7.31 (d, J= 8.8 Hz, 1H), 7.09 (d, J= 2.1 Hz, 1H), 6.96 (s, 1H), 4.84 -4.69 (m, 3H), 1.32 (d, J= 6.0 Hz, 6H).

### N-((2-(3-Chloro-4-isopropoxyphenyl)pyrimidin-5-yl)methyl)-2-(1H-pyrazol-4-yl)-6-(trifluoromethyl)pyridin-4-amine (48)

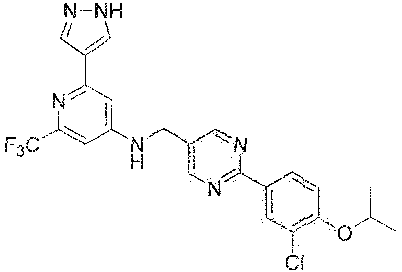

LC-MS: 489.4 (M+H)⁺. ¹H NMR (500 MHz, DMSO-d6) δ 13.07 (s, 1H), 8.90 (s, 2H), 8.36 (d, J = 2.1 Hz, 1H), 8.32 -8.23 (m, 2H), 8.04 -7.95 (m, 1H), 7.57 (t, J = 5.9 Hz, 1H), 7.31 (d, J = 8.9 Hz, 1H), 7.07 (d, J = 2.0 Hz, 1H), 6.87 (d, J = 2.0 Hz, 1H), 4.80 (hept, J = 6.0 Hz, 1H), 4.61 - 4.50 (m, 2H), 1.34 (d, J = 6.0Hz, 6H).

### N-((2-(3-Fluoro-4-isopropoxyphenyl)pyrimidin-5-yl)methyl)-2-(1H-pyrazol-4-yl)-6-(trifluoromethyl)pyridin-4-amine (49)

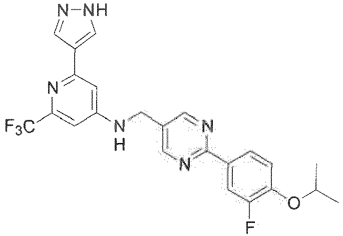

LC-MS: 473.4 (M+H)⁺. ¹H NMR (500 MHz, DMSO-d6) δ 13.07 (s, 1H), 8.90 (s, 2H), 8.29 - 8.24 (m, 1H), 8.17 (ddd, J= 8.7, 2.2, 1.1 Hz, 1H), 8.09 (dd, J= 12.7, 2.1 Hz, 1H), 8.02 -7.96 (m, 1H), 7.56 (t, J= 5.9 Hz, 1H), 7.34 -7.29 (m, 1H), 7.06 (d, J= 2.0 Hz, 1H), 6.87 (d, J= 2.0 Hz, 1H), 4.76 (hept, J= 6.1 Hz, 1H), 4.60 -4.50 (m, 2H), 1.33 (d, J= 6.0 Hz, 6H).

### 5-(5-(((2-(1H-Pyrazol-4-yl)-6-(trifluoromethyl)pyridin-4-yl)amino)methyl)pyrimidin-2-yl)-2-chlorobenzonitrile (50)

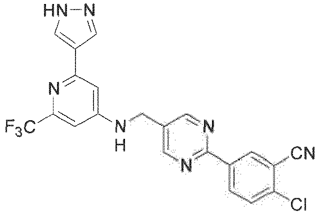

LC-MS: 456.4/458.4 (M+H)⁺. ¹H NMR (500 MHz, DMSO-d6) δ 13.07 (s, 1H), 9.01 (s, 2H), 8.78 (d, J= 2.1 Hz, 1H), 8.65 (dd, J= 8.6, 2.1 Hz, 1H), 8.25 (br s, 1H), 8.00 (br s, 1H), 7.92 (d, J= 8.6 Hz, 1H), 7.61 (t, J= 6.0 Hz, 1H), 7.07 (d, J= 2.0 Hz, 1H), 6.88 (d, J= 2.0 Hz, 1H), 4.61 (d, J= 5.9 Hz, 2H).

### N-((2-(4-(2-Fluorophenoxy)phenyl)pyrimidin-5-yl)methyl)-2-(1H-pyrazol-4-yl)-6-(trifluoromethyl)pyridin-4-amine (51)

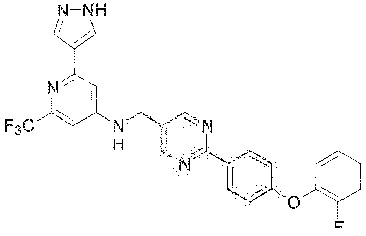

LC-MS: 507.1 (M+H)⁺. Mixture of tautomers by NMR, ratio = 1:0.15, shifts of major tautomer given: ¹H NMR (500 MHz, Acetone) δ 12.05 (s, 1H), 8.93 (s, 2H), 8.47 (d, *J* = 8.9 Hz, 2H), 8.14 (s, 2H), 7.34 - 7.26 (m, 4H), 7.17 (d, J= 1.9 Hz, 1H), 7.06 (d, *J* = 8.9 Hz, 2H), 6.95 (d, *J* = 2. 0 Hz, 1H), 6.84 (t, *J* = 5.7 Hz, 1H), 4.71 (d, *J* = 5.8 Hz, 2H).

### N-((2-(4-(3-Fluorophenoxy)phenyl)pyrimidin-5-yl)methyl)-2-(1H-pyrazol-4-yl)-6-(trifluoromethyl)pyridin-4-amine (52)

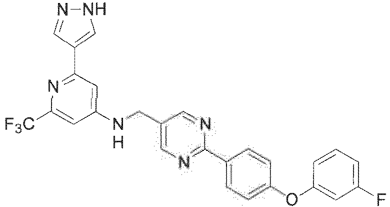

LC-MS: 507.4 (M+H)⁺. ¹H NMR (500 MHz, DMSO-d6) δ 13.07 (s, 1H), 8.91 (s, 2H), 8.45 - 8.34 (m, 2H), 8.28 -8.24 (m, 1H), 8.01 -7.97 (m, 1H), 7.57 (t, J= 5.9 Hz, 1H), 7.35 -7.24 (m, 2H), 7.23 -7.15 (m, 2H), 7.12 -7.03 (m, 3H), 6.87 (d, J= 2.0 Hz, 1H), 4.60 -4.49 (m, 2H).

### N-((2-(4-(4-Fluorophenoxy)phenyl)pyrimidin-5-yl)methyl)-2-(1H-pyrazol-4-yl)-6-(trifluoromethyl)pyridin-4-amine (53)

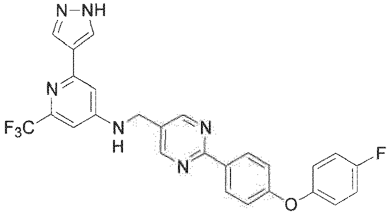

LC-MS: 507.4 (M+H)⁺. ¹H NMR (500 MHz, DMSO-d6) δ 13.07 (s, 1H), 8.93 (s, 2H), 8.47 - 8.39 (m, 2H), 8.29 -8.24 (m, 1H), 8.02 -7.97 (m, 1H), 7.57 (t, J= 5.9 Hz, 1H), 7.47 (td, J= 8.3, 6.9 Hz, 1H), 7.20 -7.14 (m, 2H), 7.08 -6.99 (m, 3H), 6.94 (ddd, J= 8.3, 2.3, 0.8 Hz, 1H),6.88 (d, J= 2.0 Hz, 1H), 4.56 (d, J= 5.8 Hz, 2H).

### N-(2-(4-(4-Chlorophenoxy)phenyl)pyrimidin-5-yl)methyl)-2-(1H-pyrazol-4-yl)-6-(trifluoromethyl)pyridin-4-amine (54)

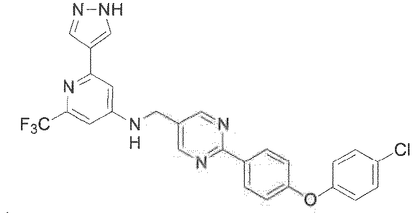

LC-MS: 523.4 (M+H)⁺. ¹H NMR (500 MHz, DMSO-d6) δ 13.07 (s, 1H), 8.92 (s, 2H), 8.46 - 8.36 (m, 2H), 8.30 -8.23 (m, 1H), 8.01 -7.97 (m, 1H), 7.57 (t, J= 5.9 Hz, 1H), 7.51 -7.47 (m, 2H), 7.18 -7.10 (m, 4H), 7.07 (d, J= 2.0 Hz, 1H), 6.88 (d, J= 2.0 Hz, 1H), 4.60 -4.50 (m, 2H).

### 2-(1H-Pyrazol-4-yl)-N-((2-(4-(trifluoromethoxy)phenyl)pyrimidin-5-yl)methyl)-6-(trifluoromethyl)pyridin-4-amine (55)

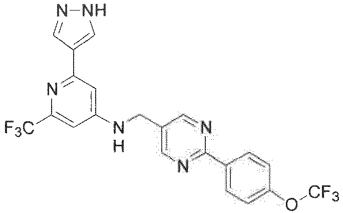

LC-MS: 481.4 (M+H)⁺. ¹H NMR (500 MHz, DMSO-d6) δ 13.07 (s, 1H), 8.97 (s, 2H), 8.59 - 8.43 (m, 2H), 8.28 -8.24 (m, 1H), 8.01 -7.97 (m, 1H), 7.59 (t, J= 5.9 Hz, 1H), 7.54 -7.48 (m, 2H), 7.07 (d, J= 2.0 Hz, 1H), 6.88 (d, J= 2.0 Hz, 1H), 4.64-4.55 (m, 2H).

### N-((2-(4-(4-Aminophenoxy)phenyl)pyrimidin-5-yl)methyl)-2-(1H-pyrazol-4-yl)-6-(trifluoromethyl)pyridin-4-amine (56)

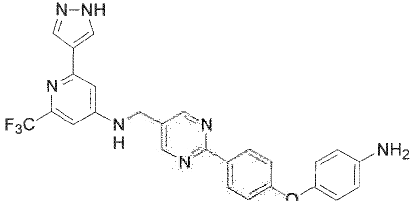

LC-MS: 504.4 (M+H)⁺. ¹H NMR (500 MHz, DMSO-d6) δ 13.07 (s, 1H), 8.88 (s, 2H), 8.36 - 8.29 (m, 2H), 8.28 -8.23 (m, 1H), 8.01 -7.97 (s, 1H), 7.55 (t, J= 5.9 Hz, 1H), 7.07 (d, J= 2.0 Hz, 1H), 7.01 -6.90 (m, 2H), 6.87 (d, J= 2.0 Hz, 1H), 6.85 -6.80 (m, 2H), 6.67-6.58 (m, 2H), 5.05 (s, 2H), 4.53 (d, J= 5.7 Hz, 2H).

### N-((5-(3-Chloro-4-isopropoxyphenyl)pyrazin-2-yl)methyl)-2-(1H-pyrazol-4-yl)-6-(trifluoromethyl)pyridin-4-amine (57)

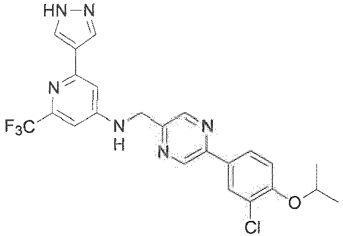

LC-MS: 489.3 (M+H)⁺. ¹H NMR (500 MHz, DMSO-d6) δ 13.06 (s, 1H), 9.23 (d, J= 1.5 Hz, 1H), 8.73 (d, J= 1.5 Hz, 1H), 8.34 -7.87 (m, 4H), 7.64 (t, J= 6.1 Hz, 1H), 7.31 (d, J= 8.9 Hz, 1H), 7.09 (d, J= 2.0 Hz, 1H), 6.92 (d, J= 2.0 Hz, 1H), 4.79 (hept, J= 6.0 Hz, 1H), 4.68 (d,J= 6.0 Hz, 2H), 1.33 (d, J= 6.0 Hz, 6H).

### N-((6-(3-Chloro-4-isopropoxyphenyl)pyridazin-3-yl)methyl)-2-(1H-pyrazol-4-yl)-6-(trifluoromethyl)pyridin-4-amine (58)

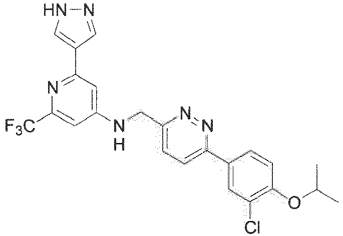

LC-MS: 489.3 (M+H)⁺. ¹H NMR (500 MHz, DMSO-d6) δ 13.06 (s, 1H), 8.31 -7.86 (m, 5H), 7.72 (dd, J = 7.5, 5.4 Hz, 2H), 7.35 (d, J = 8.8 Hz, 1H), 7.10 (d, J = 2.1 Hz, 1H), 6.92 (d, J = 2.0 Hz, 1H), 4.73 -4.90 (m, 3H), 1.34 (d, J = 6.0 Hz, 6H).

### N-((2-(4-Isopropoxyphenyl)pyrimidin-5-yl)methyl)-2-(1H-1,2,4-triazol-1-yl)-6-(trifluoromethyl)pyridin-4-amine (59)

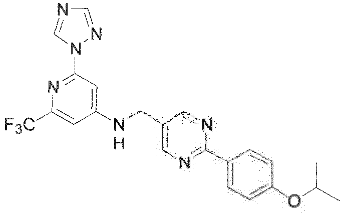

LC-MS: 456.4 (M+H)⁺. ¹H NMR (500 MHz, DMSO-d6) δ 9.22 (s, 1H), 8.87 (s, 2H), 8.37 -8.26 (m, 3H), 8.17 (t, J = 5.7 Hz, 1H), 7.27 -7.17(m, 1H), 7.13 (d, J = 2.0 Hz, 1H), 7.08 -6.98 (m, 2H), 4.72 (hept, J = 6.0 Hz, 1H), 4.65 -4.56 (m, 2H), 1.31 (d, J = 6.0 Hz, 6H).

### N-((2-(3-Chloro-4-isopropoxyphenyl)pyrimidin-5-yl)methyl)-2-(1H-1,2,4-triazol-1-yl)-6-(trifluoromethyl)pyridin-4-amine (60)

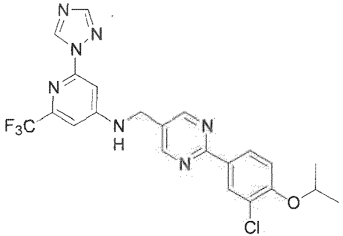

LC-MS: 490.3 (M+H)⁺. ¹H NMR (500 MHz, DMSO-d6) δ 9.22 (s, 1H), 8.90 (s, 2H), 8.41 - 8.24 (m, 3H), 8.18 (t, J= 5.7 Hz, 1H), 7.32 (d, J= 8.9 Hz, 1H), 7.28 -7.19 (m, 1H), 7.13 (d, J= 1.9 Hz, 1H), 4.80 (hept, J= 6.1 Hz, 1H), 4.67 -4.54 (m, 2H), 1.34 (d, J= 6.0 Hz, 6H).

### Example 2. Compounds 61 to 65

### 2.1 Preparation of 2-(1H-imidazol-1-yl)-N-((5-phenoxy-1H-benzo[d]imidazol-2-yl)methyl)-6-(trifluoromethyl)pyridin-4-amine (61)

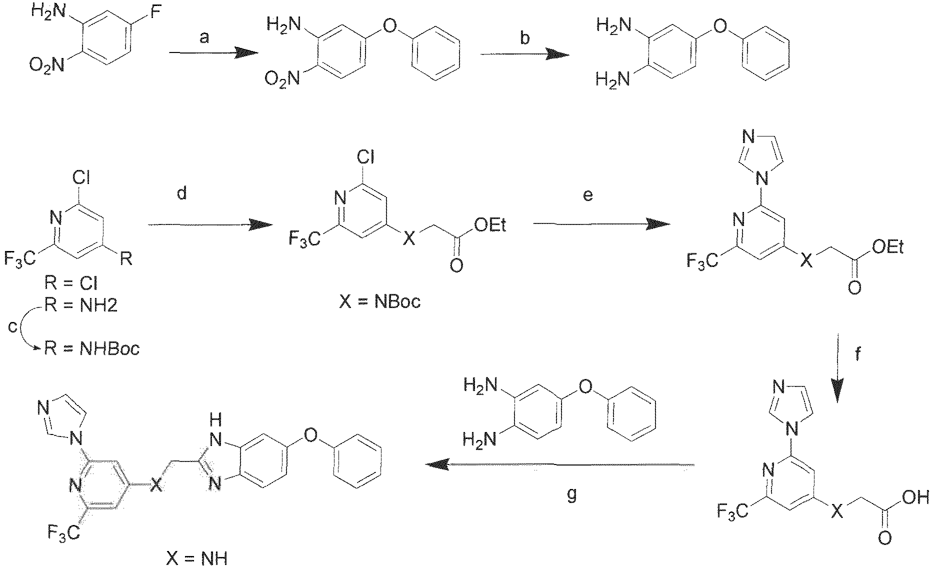

**Step a) 2-Nitro-5-phenoxyaniline.** To a mixture of 5-fluoro-2-nitroaniline (1560 mg, 10 mmol) in NMP (10 mL) were added phenol (1128 mg, 12 mmol) and K₂CO₃ (1656 mg, 12 mmol). The reaction mixture was stirred at 160 °C for 5 h. The reaction mixture was cooled to room temperature. The solid was collected by filtration, washed with cold water, and dried to obtain 2-nitro-5-phenoxyaniline (1.956 g, 7.56 mmol, 76 % yield) as a dark brown solid.

¹H NMR (500 MHz, DMSO-d6) δ 8.01 (d, J = 9.5 Hz, 1H), 7.55 - 7.45 (m, 4H), 7.34 - 7.25 (m, 1H), 7.22 - 7.14 (m, 2H), 6.37 (d, J = 2.6 Hz, 1H), 6.30 (dd, J = 9.5, 2.7 Hz, 1H); LC.MS: m/z 231.3 (M+H)⁺.

**Step b) 4-Phenoxybenzene-1,2-diamine.** A mixture of 2-nitro-5-phenoxyaniline 30a (1.956 g, 7.56 mmol), Fe (2.159 g, 38.7 mmol) and ammonium chloride (0.210 g, 3.93 mmol) was suspended in EtOH/H₂O (30 mL, 2:1, premixed). The reaction mixture was stirred at 100 °C for 2 h. EtOH/H₂O (15 mL, 2:1, premixed) were added and the reaction mixture stirred at 100 °C for 17 h. The hot reaction mixture was filtered, the solid rinsed with MeOH (2x 2 mL) and the alcoholic components of the filtrate obtained reduced in vacuo. The remaining black aqueous solution was diluted with water (50 mL) and extracted with EtOAc (3x 50 mL). The combined organics were washed with brine (50 mL), filtered, passed through a phase separation cartridge and reduced in vacuo. The black gum obtained (1.25 g) was dissolved in EtOAc (5 mL), passed through a plug of celite (3 g) and the plug rinsed with EtOAc (3x 20 mL). The combined organic extracts were reduced in vacuo to receive 4-phenoxybenzene-1,2-diamine (1.25 g, 5.62 mmol, 74.3 % yield) as a black oil.

¹H NMR (500 MHz, DMSO-d6) δ 7.36 - 7.23 (m, 2H), 6.98 (tt, J = 7.3, 1.1 Hz, 1H), 6.90 - 6.83 (m, 2H), 6.50 (d, J = 8.3 Hz, 1H), 6.25 (d, J = 2.6 Hz, 1H), 6.10 (dd, J = 8.2, 2.7 Hz, 1H), 4.64 (s, 2H), 4.32 (s, 2H). LC.MS: m/z 201.4 (M+H)⁺.

**Step c)** To a solution of 2-chloro-6-(trifluoromethyl)pyridin-4-amine (1.07 g, 5.44 mmol), DIPEA (2.092 mL, 11.98 mmol) and DMAP (0.200 g, 1.633 mmol) in DCM (20 mL) was added BOC-Anhydride (3.16 mL, 13.61 mmol) and the mixture was stirred at RT for 18 hours. Sat. aq. NH₄Cl (50 mL) was added, the mixture was stirred for 10 min then passed through a hydrophobic frit, washed with DCM (2 x 10 mL) and concentrated under reduced pressure to afford a viscous orange oil. DCM (30 mL) and TFA (3 mL) were added and the mixture was stirred at RT for 1 h. 10 wt% citric acid (50 mL) was added, the mixture was stirred for 10 min then passed through a hydrophobic frit, washed with DCM (2 x 10 mL) and concentrated under reduced pressure to afford a viscous orange oil. The crude product was purified by chromatography on silica gel (24 g cartridge, 0-50% EtOAc/isohexane) to afford Product 1 (1.79 g, 4.29 mmol, 79 % yield) as a colourless solid.

1H NMR (500 MHz, DMSO-d6) δ 10.52 (s, 1H), 7.89 (d, J = 1.7 Hz, 1H), 7.74 (d, J = 1.7 Hz, 1H), 1.51 (s, 9H). LC.MS: m/z 297 (M+H)⁺.

**Step d)** To a solution of tert-butyl (2-chloro-6-(trifluoromethyl)pyridin-4-yl)carbamate (500 mg, 1.685 mmol) and ethyl 2-bromoacetate (0.291 mL, 2.53 mmol) in DMF (5 mL, 64.6 mmol) cooled to 0 °C was added NaH (74.1 mg, 1.854 mmol) and the mixture was stirred for 4 h. The mixture was quenched by addition of sat. aq. NH₄Cl (20 mL) and extracted with TBME (2 x 20 mL). The combined organics were washed with water (2 x 20 mL), brine (20 mL), passed through a hydrophobic frit and concentrated under reduced pressure to afford a pale yellow oil. The crude product was purified by chromatography on silica gel (24 g cartridge, 0-50% EtOAc/isohexane) to afford ethyl 2-((tert-butoxycarbonyl)(2-chloro-6-(trifluoromethyl)pyridin-4-yl)amino)acetate (583 mg, 1.508 mmol, 89 % yield) as a colourless oil. LC.MS: m/z 383 (M+H)⁺.

Step e) To a solution of ethyl 2-((tert-butoxycarbonyl)(2-chloro-6-(trifluoromethyl)pyridin-4-yl)amino)acetate (150 mg, 0.392 mmol), 1H-imidazole (53.4 mg, 0.784 mmol) in dioxane (3 mL) was added caesium carbonate (383 mg, 1.176 mmol), the mixture was evacuated and purged with nitrogen 3 times then heated at 90 °C for 60 min under microwave irradiation. Pd175 (30.6 mg, 0.039 mmol) was added, the mixture was evacuated and purged with nitrogen 3 times then heated at 110°C for 60 min under microwave irradiation. The mixture was diluted with water (10 mL) and DCM (20 mL), passed through a hydrophobic frit and concentrated under reduced pressure. The crude product was purified by chromatography on silica gel (12 g cartridge, 0-50% EtOAc/isohexane) to afford ethyl 2-((2-(1H-imidazol-1-yl)-6-(trifluoromethyl)pyridin-4-yl)(tert-butoxycarbonyl)amino)acetate (66 mg, 0.158 mmol, 40.2 % yield) as a pale yellow solid. LC.MS: *m*/*z* 415 (M+H)⁺.

**Step f)** To a solution of ethyl 2-((2-(1H-imidazol-1-yl)-6-(trifluoromethyl)pyridin-4-yl)(tert-butoxycarbonyl)amino)acetate (66 mg, 0.159 mmol) in THF/H₂O (2.5 mL, 4:1) was added LiOH (19.07 mg, 0.796 mmol) and the mixture was stirred at RT for 90 min. The mixture was diluted with water (20 mL), DCM (10 mL) and passed through a hydrophobic frit. The aqueous phase was acidified to pH 1 by dropwise addition of 1 M aq. HCl then extracted with EtOAc (2 x 20 mL). The combined organics were washed with brine (2 x 20 mL), passed through a hydrophobic frit and concentrated under reduced pressure to afford 2-((2-(1H-imidazol-1-yl)-6-(trifluoromethyl)pyridin-4-yl)(tert-butoxycarbonyl)amino)acetic acid (22 mg, 0.048 mmol, 30.4 % yield) as a cream solid. LC.MS: m/z 387 (M+H)⁺.

**Step g)** To a solution of 2-((2-(1H-imidazol-1-yl)-6-(trifluoromethyl)pyridin-4-yl)(tert-butoxycarbonyl)amino)acetic acid (41 mg, 0.106 mmol) and 4-phenoxybenzene-1,2-diamine (25.5 mg, 0.127 mmol) in DCM (5 mL), cooled to 0 °C were added DIPEA (0.056 mL, 0.318 mmol) and T3P (50 wt% in EtOAc) (0.069 mL, 0.117 mmol) and the mixture was allowed to warm to RT and stirred for 18 h. The mixture was diluted with DCM (10 mL) and water (10 mL), stirred for 10 min, passed through a hydrophobic frit and concentrated under reduced pressre to afford a brown gum. AcOH (3 mL) was added and the mixture was heated at 90 °C for 3 h. The crude product was purified by preparative HPLC (Waters, Basic (0.1% Ammonium Bicarbonate), Basic, Waters X-Bridge Prep-C18, 5 µm, 19x50 mm column, 25-55% MeCN in Water) to afford 2-(1H-imidazol-1-yl)-N-((5-phenoxy-1H-benzo[d]imidazol-2-yl)methyl)-6-(tritluoromethyl)pyridin-4-amine (61) (10.6 mg, 0.023 mmol, 21.95 % yield) as a beige solid.

¹H NMR (500 MHz, DMSO-*d*₆) δ 12.51 (0.4H), 12.38 (s, 0.6H), 8.44 (s, 1H), 8.07 - 8.01 (m, 1H), 7.86 (s, 1H), 7.60 (d, *J* = 8.7 Hz, 0.6H), 7.49 (d, J = 8.7 Hz, 0.4H), 7.40 - 7.31 (m, 2H), 7.24 (m, 0.4H), 7.18 - 7.15 (m, 1H), 7.12 - 7.04 (m, 3.6H), 6.99 - 6.87 (m, 3H), 4.77 (s, 2H). LC.MS: m/z 451 (M+H)⁺.

Compounds 62 and 63 described below are further representative examples of compounds according to general formula (I) of the present invention which contain an imidazole group as group A and a group (L-2). These compounds have been synthesized based on the procedure described above.

### 2-(((2-(1H-Imidazol-1-yl)-6-(trifluoromethyl)pyridin-4-yl)thio)methyl)-5-phenoxy-1H-benzo[d]imidazole (62)

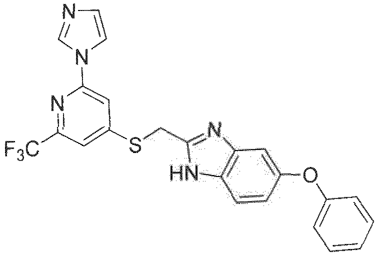

LC-MS: 468.3 (M+H)⁺. ¹H NMR (500 MHz, DMSO-*d*₆) δ 12.56 (s, 1H), 8.58 - 8.56 (m, 1H), 8.24 (d, *J* = 1.4 Hz, 1H), 7.99 - 7.96 (m, 2H), 7.55 (br s, 1H), 7.39 - 7.31 (m, 2H), 7.21 - 7.05 (m, 3H), 6.99 - 6.88 (m, 3H), 4.83 (s, 2H).

### 2-(((6-(1H-Imidazol-1-yl)-2-(trifluoromethyl)pyrimidin-4-yl)thio)methyl)-5-phenoxy-1H-benzo[d]imidazole (63)

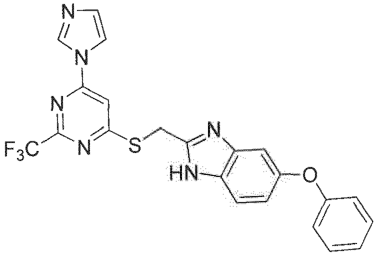

LC-MS: 469.2 (M+H)⁺. ¹H NMR (500 MHz, DMSO-*d*₆) δ 12.55 (s, 0.4H), 12.34 (s, 0.6H), 8.69 (app t, *J* = 1.1 Hz, 1H), 8.34 (s, 1H), 8.05 (app t, *J* = 1.5 Hz, 1H), 7.55 (br s, 1H), 7.35 (app t, *J* = 7.7 Hz, 2H), 7.24 (app t, *J* = 1.1 Hz, 2H), 7.09 (app t, *J =* 7.3 Hz, 1H), 7.00 - 6.88 (m, 3H), 4.85 (s, 2H).

### 2.2 Preparation of pyrazole-containing compound 64 - 2-(((2-(1H-pyrazol-4-yl)-6-(trifluoromethyl)pyridin-4-yl)thio)methyl)-5-phenoxy-1H-benzo[d]imidazole

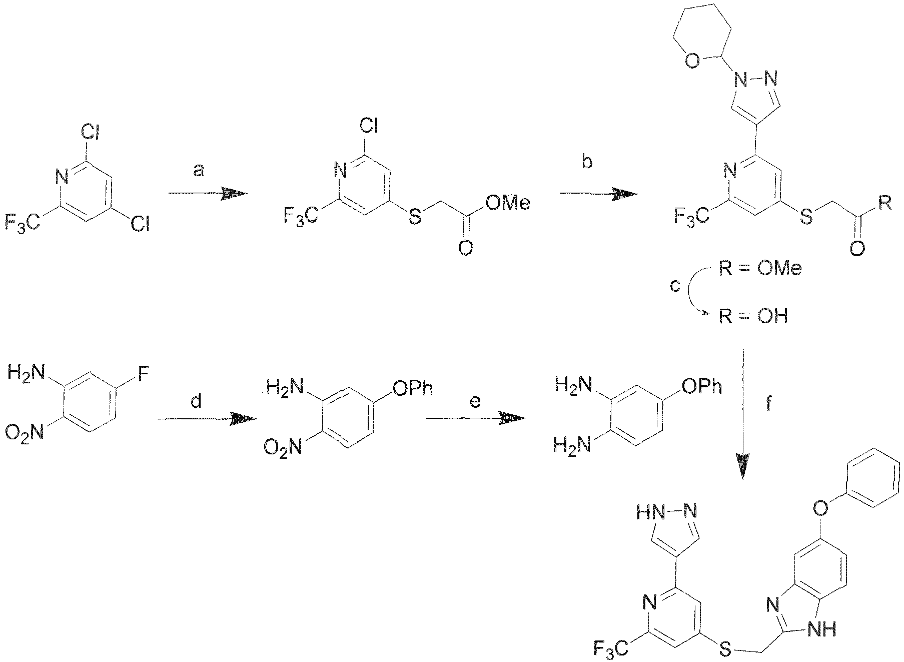

**Step a)** A mixture of 2,4-dichloro-6-(trifluoromethyl)pyridine (1.563 mL, 11.57 mmol), methyl 2-mercaptoacetate (1.035 mL, 11.57 mmol) and K2CO3 (1.600 g, 11.57 mmol) in ACETONITRILE (25 mL, 479 mmol) was heated at 65 °C (plate temperature) for 3 h then cooled to RT. The mixture was added slowly to 10 wt% aq. citric acid (100 mL) and extracted with TBME (3 x 50 mL). The combined organics were washed with water (100 mL), brine (50 mL), passed through a hydrophobic frit and concentrated under reduced pressure to give a colourless oil. The crude product was purified by chromatography on silica gel (40 g cartridge, 0-25% EtOAc/isohexane) to afford methyl 2-((2-chloro-6-(trifluoromethyl)pyridin-4-yl)thio)acetate (3.12 g, 10.92 mmol, 94 % yield) as a colourless oil. LC.MS: m/z 286 (M+H)⁺.

**Step b)** A mixture of methyl 2-((2-chloro-6-(trifluoromethyl)pyridin-4-yl)thio)acetate (50 mg, 0.175 mmol), 1-(tetrahydro-2H-pyran-2-yl)-4-(4,4,5,5-tetramethyl-1,3,2-dioxaborolan-2-yl)-1H-pyrazole (58.4 mg, 0.210 mmol) and SILVER CARBONATE (48.3 mg, 0.175 mmol) in 1-methyl-1H-pyrrole-2,5-dione (19.45 mg, 0.175 mmol) was evacuated and purged with nitrogen 3 times then heated under microwave irradiation at 130 °C for 10 min. The reaction was diluted with water (20 mL) and extracted with EtOAc (2 x 20 mL). The combined organics were washed with water (20 mL), brine (20 mL), passed through a hydrophobic frit and concentrated under reduced pressure. The crude product was combined with those from trial reactions 2505-14, 2505-16 & 2505-17, 2505-18, 2505-19,& 2505-20 and purified by chromatography on silica gel (24 g cartridge, 0-50% EtOAc/isohexane) to afford methyl 2-((2-(1-(tetrahydro-2H-pyran-2-yl)-1H-pyrazol-4-yl)-6-(trifluoromethyl)pyridin-4-yl)thio)acetate (206 mg, 0.498 mmol, 284 % yield) as a pale yellow oil. LC.MS: m/z 402 (M+H)⁺.

Step c) To a solution of methyl 2-((2-(1-(tetrahydro-2H-pyran-2-yl)-1H-pyrazol-4-yl)-6-(trifluoromethyl)pyridin-4-yl)thio)acetate (206 mg, 0.513 mmol) in THF-water (4 mL, 3:1) was added LiOH (61.5 mg, 2.57 mmol) and the mixture was stirred at RT for 3 h. The mixture was diluted with water (20 mL) and extracted with DCM (10 mL). The aqueous phase was acidified by addition of 10 wt% aq. citric acid and extracted with EtOAc (2 x 20 mL). The combined organics were washed with water (20 mL), brine (20 mL), passed through a hydrophobic frit and concentrated under reduced pressure to afford 2-((2-(1-(tetrahydro-2H-pyran-2-yl)-1H-pyrazol-4-yl)-6-(trifluoromethyl)pyridin-4-yl)thio)acetic acid (154 mg, 0.394 mmol, 77 % yield) as a cream solid. LC.MS: m/z 388 (M+H)⁺.

**Step d) 2-Nitro-5-phenoxyaniline.** To a mixture of 5-fluoro-2-nitroaniline (1560 mg, 10 mmol) in NMP (10 mL) were added phenol (1128 mg, 12 mmol) and K₂CO₃ (1656 mg, 12 mmol). The reaction mixture was stirred at 160 °C for 5 h. The reaction mixture was cooled to room temperature. The solid was collected by filtration, washed with cold water, and dried to obtain 2-nitro-5-phenoxyaniline (1.956 g, 7.56 mmol, 76 % yield) as a dark brown solid.

¹H NMR (500 MHz, DMSO-d6) δ 8.01 (d, J = 9.5 Hz, 1H), 7.55 - 7.45 (m, 4H), 7.34 - 7.25 (m, 1H), 7.22 - 7.14 (m, 2H), 6.37 (d, J = 2.6 Hz, 1H), 6.30 (dd, J = 9.5, 2.7 Hz, 1H); LC.MS: m/z 231.3 (M+H)⁺.

**Step e) 4-Phenoxybenzene-1,2-diamine.** A mixture of 2-nitro-5-phenoxyaniline 30a (1.956 g, 7.56 mmol), Fe (2.159 g, 38.7 mmol) and ammonium chloride (0.210 g, 3.93 mmol) was suspended in EtOH/H₂O (30 mL, 2:1, premixed). The reaction mixture was stirred at 100 °C for 2 h. EtOH/H₂O (15 mL, 2:1, premixed) were added and the reaction mixture stirred at 100 °C for 17 h. The hot reaction mixture was filtered, the solid rinsed with MeOH (2x 2 mL) and the alcoholic components of the filtrate obtained reduced in vacuo. The remaining black aqueous solution was diluted with water (50 mL) and extracted with EtOAc (3x 50 mL). The combined organics were washed with brine (50 mL), filtered, passed through a phase separation cartridge and reduced in vacuo. The black gum obtained (1.25 g) was dissolved in EtOAc (5 mL), passed through a plug of celite (3 g) and the plug rinsed with EtOAc (3x 20 mL). The combined organic extracts were reduced in vacuo to receive 4-phenoxybenzene-1,2-diamine (1.25 g, 5.62 mmol, 74.3 % yield) as a black oil.

¹H NMR (500 MHz, DMSO-d6) δ 7.36 - 7.23 (m, 2H), 6.98 (tt, J = 7.3, 1.1 Hz, 1H), 6.90 - 6.83 (m, 2H), 6.50 (d, *J* = 8.3 Hz, 1H), 6.25 (d, J = 2.6 Hz, 1H), 6.10 (dd, J = 8.2, 2.7 Hz, 1H), 4.64 (s, 2H), 4.32 (s, 2H). LC.MS: m/z 201.4 (M+H)⁺.

**Step f)** To a solution of 2-((2-(1-(tetrahydro-2H-pyran-2-yl)-1H-pyrazol-4-yl)-6-(trifluoromethyl)pyridin-4-yl)thio)acetic acid (75 mg, 0.194 mmol) and 4-phenoxybenzene-1,2-diamine (46.5 mg, 0.232 mmol) in DCM (5 mL), cooled to 0 °C were added DIPEA (0.101 mL, 0.581 mmol) and T3P (50 wt% in EtOAc) (0.127 mL, 0.213 mmol) and the mixture was allowed to warm to RT and stirred for 2 h. The mixture was diluted with DCM (10 mL) and water (10 mL), stirred for 10 min, passed through a hydrophobic frit and concentrated under reduced pressure to afford a brown gum. AcOH (3 mL) was added and the mixture was heated at 90 °C for 3 h. The crude product was purified by preparative HPLC (Waters, Basic (0.1% Ammonium Bicarbonate), Basic, Waters X-Bridge Prep-C18, 5 µm, 19x50 mm column, 25-55% MeCN in Water) to afford 2-(((2-(1H-pyrazol-4-yl)-6-(trifluoromethyl)pyridin-4-yl)thio)methyl)-5-phenoxy-1H-benzo[d]imidazole (37.6 mg, 0.080 mmol, 41.5 % yield) as a beige solid.

¹H NMR (500 MHz, DMSO-d6) δ 13.23 (s, 1H), 12.57 (s, 1H), 8.70-7.85 (m, 3H), 7.73 (d, J = 1.6 Hz, 1H), 7.53 (d, J = 8.5 Hz, 1H), 7.41 - 7.25 (m, 2H), 7.23 - 7.01 (m, 2H), 7.00 - 6.84 (m, 3H), 4.74 (s, 2H). LC.MS: m/z 468 (M+H)⁺.

Compound 65 described below is a further representative example of a compound according to general formula (I) of the present invention which contains a pyrazole group as group A and a group (L-2). This compound has been synthesized based on the procedure described above.

### 2-(((2-(1-Methyl-1H-pyrazol-4-yl)-6-(trifiuoromethyl)pyridin-4-yl)thio)methyl)-5-phenoxy-1H-benzo[d]imidazole (65)

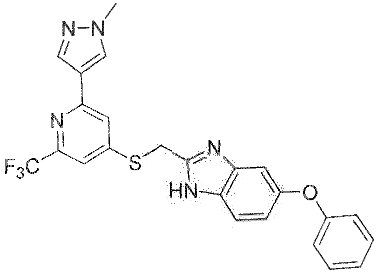

LC-MS: 482.5 (M+H)⁺. ¹H NMR (500 MHz, DMSO-*d*₆) δ 12.64 (s, 0.4H), 12.50 (s, 0.6H), 8.35 (s, 1H), 8.07 (s, 1H), 8.03 (s, 1H), 7.76 - 7.69 (m, 1H), 7.62 - 7.47 (m, 1H), 7.37 - 7.31 (m, 2H), 7.25 - 7.02 (m, 2H), 7.00 - 6.85 (m, 3H), 4.74 (s, 2H), 3.90 (s, 3H).

### Example 3: Compounds 66 to 70

Compound 66 to 70 described below are further representative examples of a compound according to general formula (I) of the present invention. These compounds have been synthesized based on the procedures described above.

### 2-(((2-(1H-1,2,4-Triazol-1-yl)-6-(trifluoromethyl)pyridin-4-yl)thio)methyl)-5-phenoxy-1H-benzo[d]imidazole. (66)

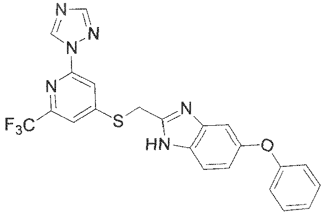

LC-MS: 469.2 (M+H)⁺. ¹H NMR (500 MHz, DMSO-*d*₆) δ 12.64 (s, 0.4H), 12.51 (s, 0.6H), 9.35 (s, 1H), 8.38 (s, 1H), 8.17 (d, *J* = 1.5 Hz, 1H), 8.12 (s, 1H), 7.58 - 7.48 (m, 1H), 7.39 - 7.32 (m, 2H), 7.21 - 7.03 (m, 2H), 6.99 - 6.85 (m, 3H), 4.81 (s, 2H).

### N-((1-(3-Chloro-4-isopropoxyphenyl)-1H-1,2,3-triazol-4-yl)methyl)-6-(trifluoromethyl)-[2,3'-bipyridin]-4-amine (67)

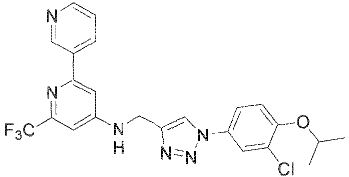

LC-MS: 489.1 (M+H)⁺. ¹H NMR (500 MHz, Acetone) δ 9.24 (s, 1H), 8.63 (s, 1H), 8.57 (s, 1H), 8.40 (d, *J* = 7.9 Hz, 1H), 7.90 (d, *J* = 2.6 Hz, 1H), 7.77 (dd, *J* = 8.9, 2.7 Hz, 1H), 7.53 (s, 1H), 7.48 (dd, J= 7.8, 4.6 Hz, 1H), 7.33 (d, *J* = 8.9 Hz, 1H), 7.17 (s, 1H), 6.97 (s, 1H), 4.79 (d, *J* = 5.7 Hz, 3H), 1.38 (d, *J* = 6.0 Hz, 6H).

### 4-(((2-(4-Isopropoxyphenyl)thiazol-5-yl)methyl)amino)-6-(trifluoromethyl)-[2,3'-bipyridine]-6'-carbonitrile (68)

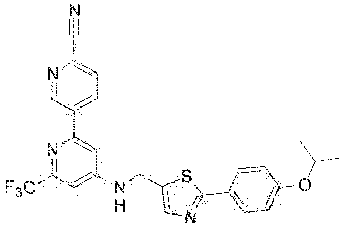

LC-MS: 496.2 (M+H)⁺. ¹H NMR (500 MHz, Acetone) δ 9.38 (d, *J* = 1.6 Hz, 1H), 8.66 (dd, J = 8.2, 2.2 Hz, 1H), 8.05 (dd, *J* = 8.2, 0.7 Hz, 1H), 7.88 - 7.82 (m, 3H), 7.64 (d, *J* = 1.9 Hz, 1H), 7.22 (d, *J* = 2.0 Hz, 1H), 7.18 (t, *J* = 5.8 Hz, 1H), 7.02 - 6.95 (m, 2H), 4.96 (d, *J* = 5.2 Hz, 2H), 4.76 - 4.63 (m, 1H), 1.32 (d, *J* = 6.0 Hz, 6H).

### N-((2-(3-Chloro-4-isopropoxyphenyl)pyrimidin-5-yl)methyl)-2-(thiazol-4-yl)-6-(trifluoromethyl)pyridin-4-amine (69)

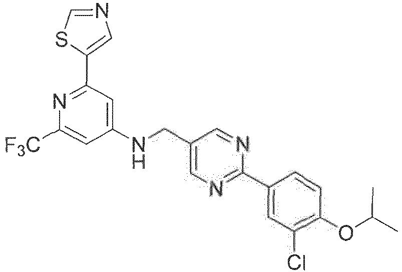

LC-MS: 506.3 (M+H)⁺. ¹H NMR (500 MHz, DMSO-d6) δ 9.15 (d,J = 0.6 Hz, 1H), 8.92 (s, 2H), 8.61 -8.57 (m, 1H), 8.36 (d, J= 2.2 Hz, 1H), 8.30 (dd, J= 8.8, 2.2 Hz, 1H),7.83 (t, J= 5.9 Hz, 1H), 7.40 -7.28 (m, 2H), 7.01 (d, J= 2.0 Hz, 1H), 4.80 (hept, J= 6.0 Hz, 1H), 4.60 (d, J= 5.8 Hz, 2H), 1.34 (d, J= 6.1 Hz, 6H).

### N-((2-(4-Isopropoxyphenyl)pyrimidin-5-yl)methyl)-2-(thiazol-5-yl)-6-(trifluoromethyl)pyridin-4-amine (70)

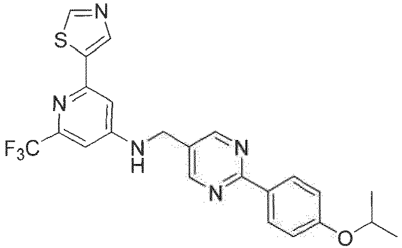

LC-MS: 472.4 (M+H)⁺. ¹H NMR (500 MHz, DMSO-d6) δ 9.15 (s, 1H), 8.88 (s, 2H), 8.59 (s, 1H), 8.35 -8.27 (m, 2H), 7.82 (t, J= 5.9 Hz, 1H), 7.38 (d, J= 2.0 Hz, 1H), 7.09 -6.98 (m, 3H), 4.72 (hept, J= 6.2 Hz, 1H), 4.58 (d, J= 5.4 Hz, 2H), 1.36 -1.25 (m, 6H).

### Example 4: PqsR inverse agonistic activity of test compounds (Reporter gene assay in E. coli)

Inhibition of pqsR-dependent gene expression of test compounds was determined in an *E. coli* based β-galactosidase reporter gene assay.[19] *Escherichia coli* DH5α was transfected with pEAL08-2 plasmid encoding pqsR under control of promoter tac and β-galactosidase reporter gene lacZ under control of promoter pqsA. Antagonistic effects of compounds were evaluated in the presence of/in competition to 50 nM PQS. A positive control was used to ensure the reliability of individual assays. First, PQS was diluted in methanol and added to the wells of a glass coated 96-deep-well plate, and the solvent was evaporated. Then, compounds were added in 5 µL DMSO to final concentrations of 0.001 - 10 µM. Overnight cultures of *E. coli* DH5a x pEAL08-2 were diluted 1:100 in LB medium with ampicillin (50 µg/ml) and incubated at 37°C with shaking until it reached an OD600 of 0.2. Finally, 995 µL of culture was added to each well and the β-galactosidase activity was determined by *ortho*-nitrophenyl-β-galactoside conversion in permeabilized cells after a 2.5 h incubation period (37°C, 180 rpm). OD600, OD420, and OD550 were measured using POLARstar Omega (BMG Labtech, Ortenberg, Germany), and the activity was expressed as ratio of the slope of β-galactosidase activity between basal control (no PQS) and solvent control. IC₅₀ values of antagonists were determined by variation of the concentration of the test compounds.

Results of measurements of example test compounds are summarized below.

The following Example Compounds showed an IC₅₀-value of equal to or below 50 nM towards PqsR-dependent transcription: 02, 03, 04, 07, 08, 09, 10, 11, 12, 15, 18, 48, 66.

The following Example Compounds showed an IC₅₀-value between 51 and 250 nM towards PqsR-dependent transcription: 01, 13, 14, 16, 17, 65, 67.

None of the tested compounds showed any bacteriostatic or bactericidal effects in the cell-based test system.

### Example 5: Pyocyanin assay

Inhibition of *P. aeruginosa* pyocyanin production by compounds was determined photometrically as described before.[20] In short, an overnight culture of PA14 in PPGAS medium was washed, diluted in medium to a start OD600 of 0.02, and incubated for 16 h (37°C, 200 rpm, relative humidity 75%) in the presence of test compounds. Then, cultures were extracted with chloroform and re-extracted with 0.2 M HCl. Protonated red colored pyocyanin was determined by measuring OD520 and normalizing to OD600.

Results of measurements of example test compounds are summarized below.

The following Example Compounds showed an IC₅₀-value of equal to or below 100 nM towards pyocyanin production: 07, 11, 12, 15, 17, 18, 28.

The following Example Compounds showed an IC₅₀-value between 101 and 200 nM towards pyocyanin production: 01, 04, 16, 20, 21, 25, 27, 29, 30, 35, 37, 38, 39, 48, 49, 50, 51, 52, 53, 56, 57, 58, 59, 60, 61, 61, 64, 66, 70.

The following Example Compounds showed an IC₅₀-value between 201 and 500 nM towards pyocyanin production: 02, 03, 08, 09, 10, 13, 14, 22, 23, 24, 26, 31, 32, 33, 34, 36, 40, 42, 44, 45, 46, 47, 55, 62, 68, 69.

### Example 6: Compound solublity

Kinetic Turbidimetric Aqueous Solubility at room temperature for 5 min. Test compound was diluted in PBS buffer to give a range of concentrations (typically 0.4, 2, 4, 20, 40, 100, and 200 µM, final DMSO concentration 2 %) and incubated at room temperature for 5 min. Absorbance was measured at a wavelength of 620 nm and the solubility was estimated from the concentration of test compound that produces an increase in absorbance above the vehicle control (i.e., 1 % DMSO in buffer). Data was analyzed by fitting a four parameter sigmoidal function to the measured and normalized absorbances and AUC calculation providing access to LogS (decadic logarithm of the solubility).

The following Example Compounds showed a Kinetic Turbidimetric Aqueous Solubility at room temperature for 5 min above or equal to 100 µM: 19.

The following Example Compounds showed a Kinetic Turbidimetric Aqueous Solubility at room temperature for 5 min above 50 µM: 17, 51.

The following Example Compounds showed a Kinetic Turbidimetric Aqueous Solubility at room temperature for 5 min above 25 µM: 01, 02, 03, 05, 07, 08, 10, 21, 22, 23, 27, 28, 36, 38, 48, 57, 58, 68.

The following Example Compounds showed a Kinetic Turbidimetric Aqueous Solubility at room temperature for 5 min above 10 µM: 04, 06, 09, 11, 12, 13, 14, 15, 20, 24, 30, 31, 33, 34, 35, 37, 39, 40, 41, 43, 47, 49, 54, 55, 56, 60, 61, 63, 64, 65, 66, 69.

### Example 7: Metabolic Stability Tests in Mouse Liver Microsomes (MLM).

For the evaluation of phase I metabolic stability, the compound (1 µM) was incubated with 0.5 mg/mL MLM (Corning) and 1 mM NADPH at 37 °C for 0, 5, 10, 15 and 30 min. The metabolic stability of Verapamil, Diphenhydramine and Benzydamine (1 µM each) were determined in parallel to confirm the enzymatic activity of the MLM. The incubation was stopped by addition of 2 volumes of acetonitrile containing internal standard (1 µM Leucine Enkephaline). Samples were centrifuged (15 min, 3,500 rpm). Concentration of the remaining test compound at the different time points was analyzed by HPLC-MS/MS and used to determine half-life (t_{1/2}).

Results of measurements of example test compounds are summarized below.

The following Example Compounds showed a half-life (t_{1/2}) above 100 min towards MLM: 14, 16, 18, 19, 20, 21, 23, 24, 25, 26, 27, 29, 30, 31, 34, 35, 39, 41, 42, 43, 44, 45, 48, 49, 51, 52, 53, 54, 55, 57, 58, 59, 68, 69, 70.

The following Example Compounds showed a half-life (t_{1/2}) between 61 min and 100 min towards MLM: 03, 06, 17, 28, 32, 33, 46, 56, 60.

The following Example Compounds showed a half-life (t_{1/2}) between 30 min and 60 min towards MLM: 04, 07, 10, 11, 12, 13, 15, 47, 61.

### References

1. Frei R, Breitbach AS, Blackwell HE (2012) Angew Chem Int Ed Engl 51:5226-5229
2. Yang L, Rybtke MT, Jakobsen TH, Hentzer M, Bjarnsholt T, Givskov M, Tolker-Nielsen T (2009) Antimicrob Agents Chemother 53:2432-2443
3. O'Loughlin CT, Miller LC, Siryaporn A, Drescher K, Semmelhack MF, Bassler BL (2013) Proc Natl Acad Sci U S A 110:17981-17986
4. Hentzer M, Wu H, Andersen JB, Riedel K, Rasmussen TB, Bagge N, Kumar N, Schembri MA, Song Z, Kristoffersen P, Manefield M, Costerton JW, Molin S, Eberl L, Steinberg P, Kjelleberg S, Hoiby N, Givskov M (2003) EMBO J 22:3803-3815
5. Hentzer M, Riedel K, Rasmussen TB, Heydorn A, Andersen JB, Parsek MR, Rice SA, Eberl L, Molin S, Hoiby N, Kjelleberg S, Givskov M (2002) Microbiology 148:87-102
6. Rasmussen TB, Skindersoe ME, Bjarnsholt T, Phipps RK, Christensen KB, Jensen PO, Andersen JB, Koch B, Larsen TO, Hentzer M, Eberl L, Hoiby N, Givskov M (2005) Microbiology 151:1325-1340
7. Jakobsen TH, van GM, Phipps RK, Shanmugham MS, Christensen LD, Alhede M, Skindersoe ME, Rasmussen TB, Friedrich K, Uthe F, Jensen PO, Moser C, Nielsen KF, Eberl L, Larsen TO, Tanner D, Hoiby N, Bjarnsholt T, Givskov M (2012) Antimicrob Agents Chemother 56:2314-2325
8. Hinsberger S, de Jong JC, Groh M, Haupenthal J, Hartmann RW (2014) Eur J Med Chem 76C:343-351
9. Storz MP, Brengel C, Weidel E, Hoffmann M, Hollemeyer K, Steinbach A, Muller R, Empting M, Hartmann RW (2013) ACS Chem Biol 8:2794-2801
10. Sahner JH, Brengel C, Storz MP, Groh M, Plaza A, Muller R, Hartmann RW (2013) J Med Chem 56:8656-8664
11. Weidel E, de Jong JC, Brengel C, Storz MP, Braunshausen A, Negri M, Plaza A, Steinbach A, Muller R, Hartmann RW (2013) J Med Chem 56:6146-6155
12. Calfee MW, Coleman JP, Pesci EC (2001) Proc Natl Acad Sci U S A 98:11633-1 1637
13. Pistorius D, Ullrich A, Lucas S, Hartmann RW, Kazmaier U, Muller R (2011) Chembiochem 12:850-853
14. Lesic B, Lepine F, Deziel E, Zhang J, Zhang Q, Padfield K, Castonguay MH, Milot S, Stachel S, Tzika AA, Tompkins RG, Rahme LG (2007) PLoS Pathog 3:1229-1239
15. Storz MP, Maurer CK, Zimmer C, Wagner N, Brengel C, de Jong JC, Lucas S, Musken M, Haussler S, Steinbach A, Hartmann RW (2012) J Am Chem Soc 134:16143-16146
16. Coleman JP, Hudson LL, McKnight SL, Farrow JM, III, Calfee MW, Lindsey CA, Pesci EC (2008) J Bacteriol 190:1247-1255
17. Klein T, Henn C, de Jong JC, Zimmer C, Kirsch B, Maurer CK, Pistorius D, Muller R, Steinbach A, Hartmann RW (2012) ACS Chem Biol 7:1496-1501
18. Zender M, Klein T, Henn C, Kirsch B, Maurer CK, Kail D, Ritter C, Dolezal O, Steinbach A, Hartmann RW (2013) J Med Chem 56:6761-6774
19. Lu C, Kirsch B, Zimmer C, de Jong JC, Henn C, Maurer CK, Musken M, Haussler S, Steinbach A, Hartmann RW (2012) Chem Biol 19:381-390
20. Lu C, Maurer CK, Kirsch B, Steinbach A, Hartmann RW (2014) Angew Chem Int Ed Engl 53:1109-1112
21. Ilangovan A, Fletcher M, Rampioni G, Pustelny C, Rumbaugh K, Heeb S, Camara M, Truman A, Chhabra SR, Emsley J, Williams P (2013) PLoS Pathog 9:e1003508
22. Maurer CK, Steinbach A, Hartmann RW (2013) J Pharm Biomed Anal 86C:127-134
23. Zhang Y, Miller RM (1992) Appl Environ Microbiol 58:3276-3282

Overall, the results presented herein show that compounds of the invention are anti-pathogenic compounds exhibiting both anti-virulence and anti-biofilm activity allowing for an effective treatment of bacterial infections and their application in *in vivo.*

## Claims

1. A compound of the formula (I): 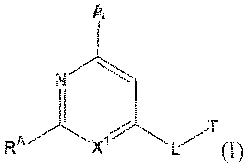 or a pharmacologically acceptable salt thereof,
wherein
A represents a 5- or 6-membered heteroaromatic group containing at least one nitrogen atom, which may optionally be substituted by one or more, identical or different, group(s) selected from (C₁-C₃)alkyl group, CN, a halogen atom, and OH;
X¹ represents CH or N;
R^{A} represents CF₃, Cl or CN;
L is a group represented by formula (L-1) or (L-2): 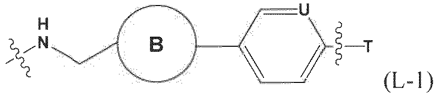
wherein
ring B is 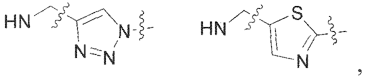 or a 6-membered heterocyclic ring represented by formula (B-1): 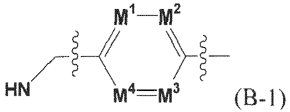
wherein
M¹, M², M³ and M⁴ each, independently of one another, represents CH or N; at least one of M¹, M², M³, and M⁴ being N;
wherein ring B is the ring shown between the wavy lines, and the connectivity to the groups attached thereto is as shown above;
U represents N or C(R^{U});
R^{U} represents a hydrogen atom, a halogen atom, NR^{U1}R^{U2}, OH, CN, CF₃, CH₂-OH, OCH₃, or OCF₃;
R^{U1} and R^{U2} each, independently of one another, represents a hydrogen atom; or an akyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted by one or more, identical or different, group(s) selected from a halogen atom, OH, =O, and NH₂; 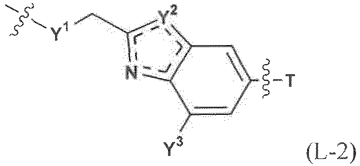
wherein
Y¹ is NH or S;
Y² is N, NH, O, or S;
Y³ represents a hydrogen atom, F, Cl, OH, CN, (C₁-C₃)alkyl, CF₃, CH₂-OH, OCH₃, or OCF₃;
each " 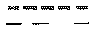 ", independently of one another, represents a single bond or a double bond, wherein at least one " 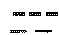 " in the ring of formula (L-2) is a double bond;
T is R or a group: -Z-R', wherein
R represents a hydrogen atom, a halogen atom, CN, CF₃, CH₂-OH; NR^{T1}R^{T2}; or an akyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
R^{T1} and R^{T2} each, independently of one another, represents a hydrogen atom or a (C₁-C₃)alkyl group, which may be substituted by one or more, identical or different, group(s) selected from a halogen atom, OH, =O, and NH₂;
Z is NH, -O- or -S-; and
R' is a hydrogen atom; or an akyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted.

2. The compound according to claim 1, or a pharmacologically acceptable salt thereof, wherein A is a group selected from the groups:

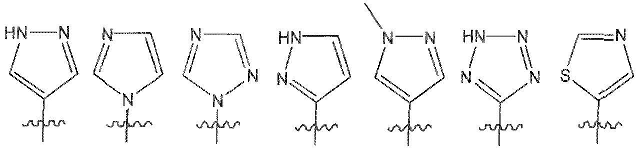

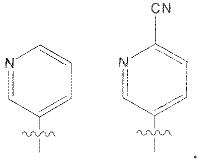

3. The compound according to claim 1 or 2, or a pharmacologically acceptable salt thereof, wherein L is a group of formula (L-1); and
wherein ring B is 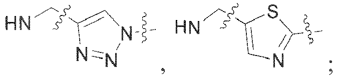 or
a 6-membered heterocyclic ring represented by formula (B-1), which is selected from the groups:

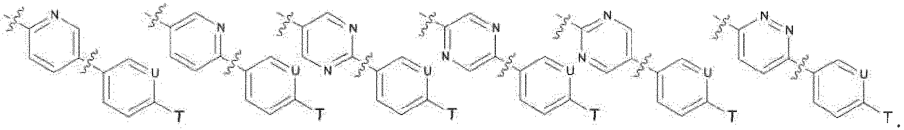

4. The compound according to any one of claims 1 to 3, or a pharmacologically acceptable salt thereof, wherein R^{U} represents a hydrogen atom, Cl, F, CN; or a group selected from the groups:

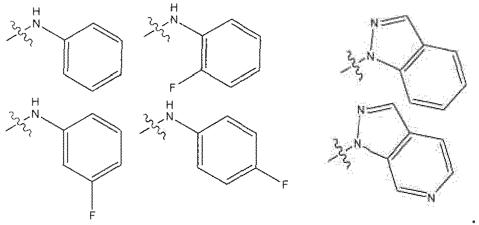

5. The compound according to any one of claims 1 to 4, or a pharmacologically acceptable salt thereof, wherein the group represented by formula (L-1) contains one of the following groups as a partial structure:

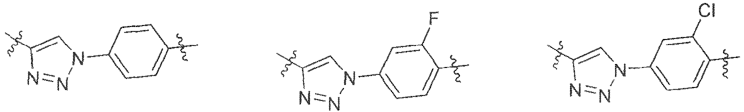

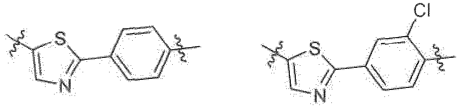

6. The compound according to any one of claims 1 to 4, or a pharmacologically acceptable salt thereof, wherein the group represented by formula (L-1) contains one of the following groups as a partial structure:

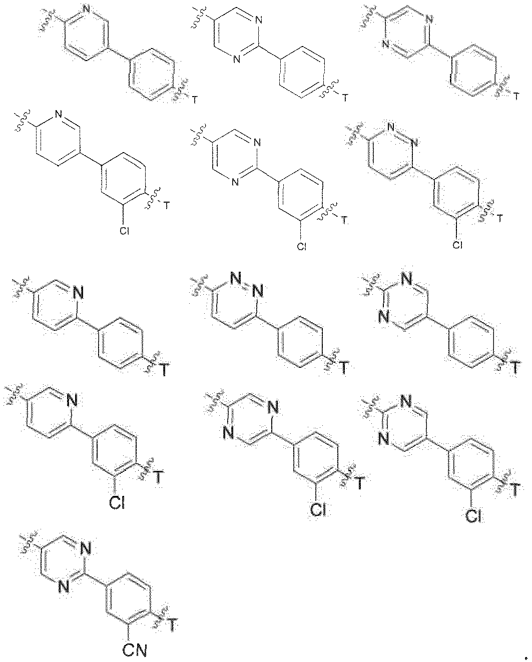

7. The compound according to claim 1 or 2, or a pharmacologically acceptable salt thereof,
wherein L is a group of formula (L-2); and
wherein the group represented by formula (L-2) is a group selected from:

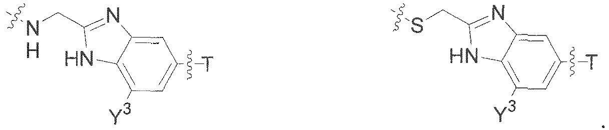

8. The compound according to any one of claims 1 to 7, or a pharmacologically acceptable salt thereof, wherein T is a hydrogen atom, Cl, F; or a group selected from the groups:

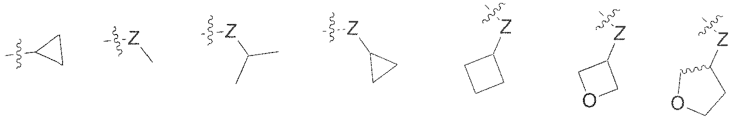

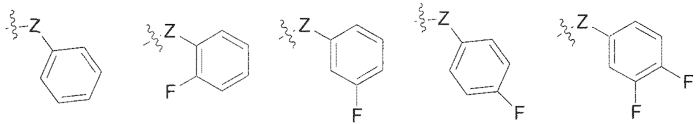

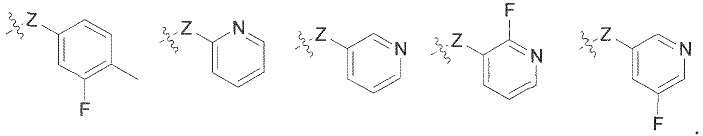

9. The compound according to claim 8, or a pharmacologically acceptable salt thereof, wherein Z is NH or O.

10. The compound according to any one of claims 1 to 9, or a pharmacologically acceptable salt thereof, wherein the compound is selected from the group consisting of:

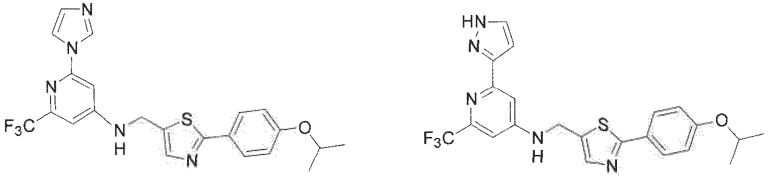

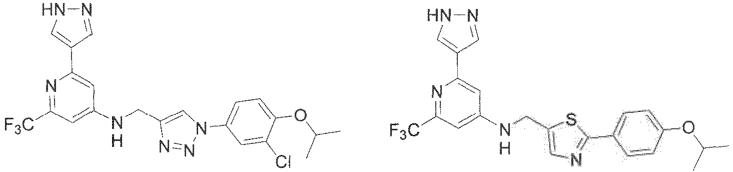

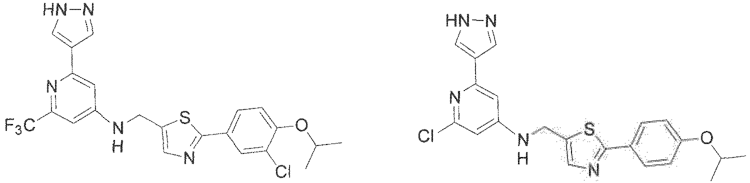

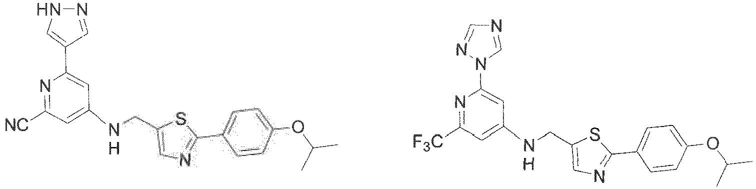

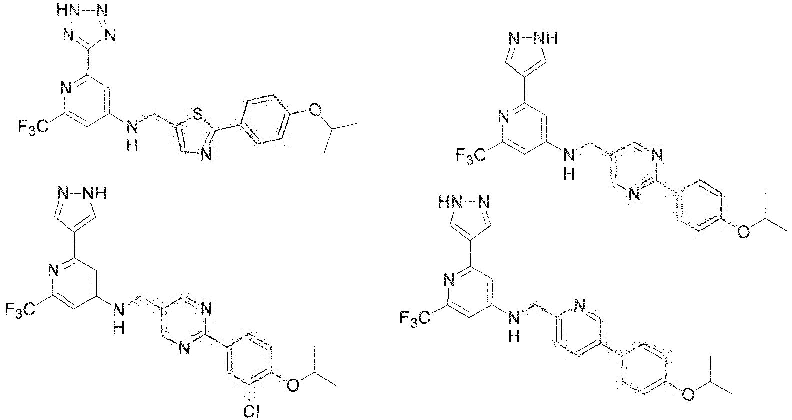

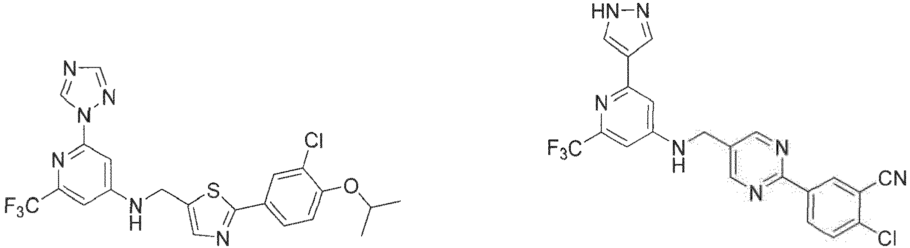

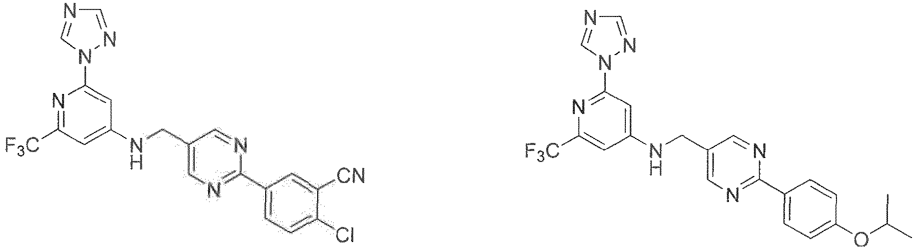

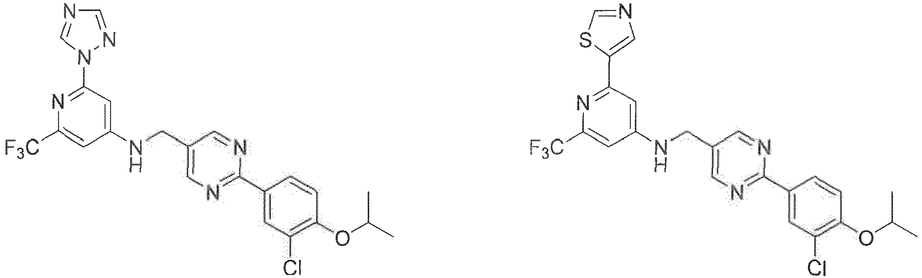

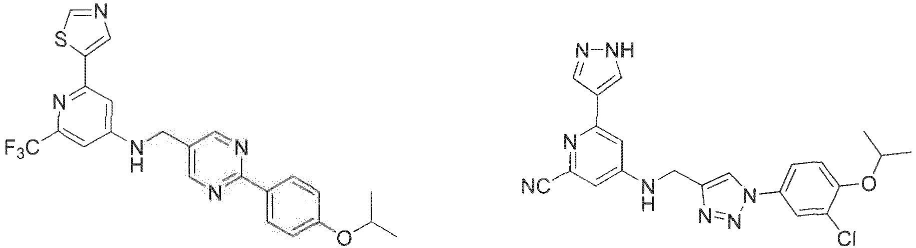

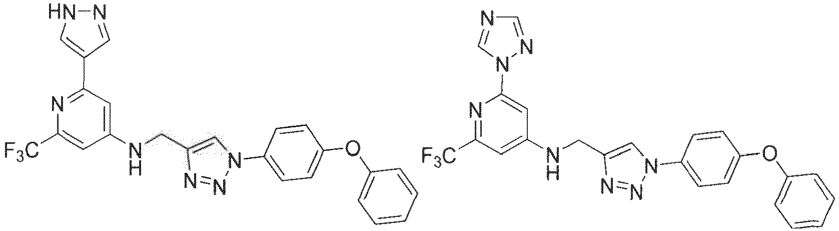

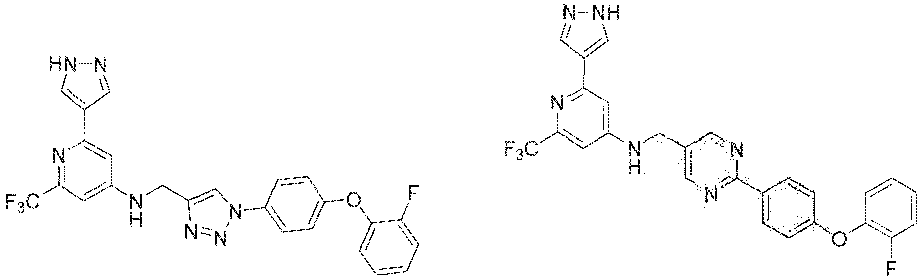

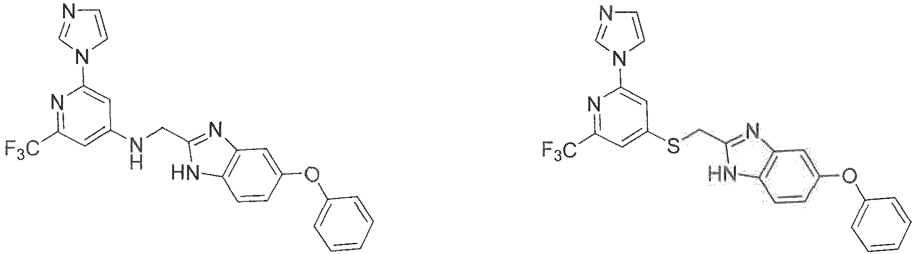

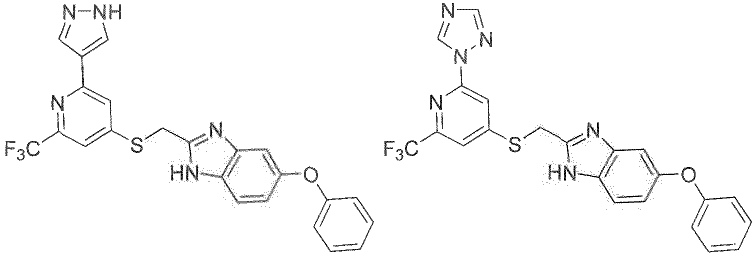

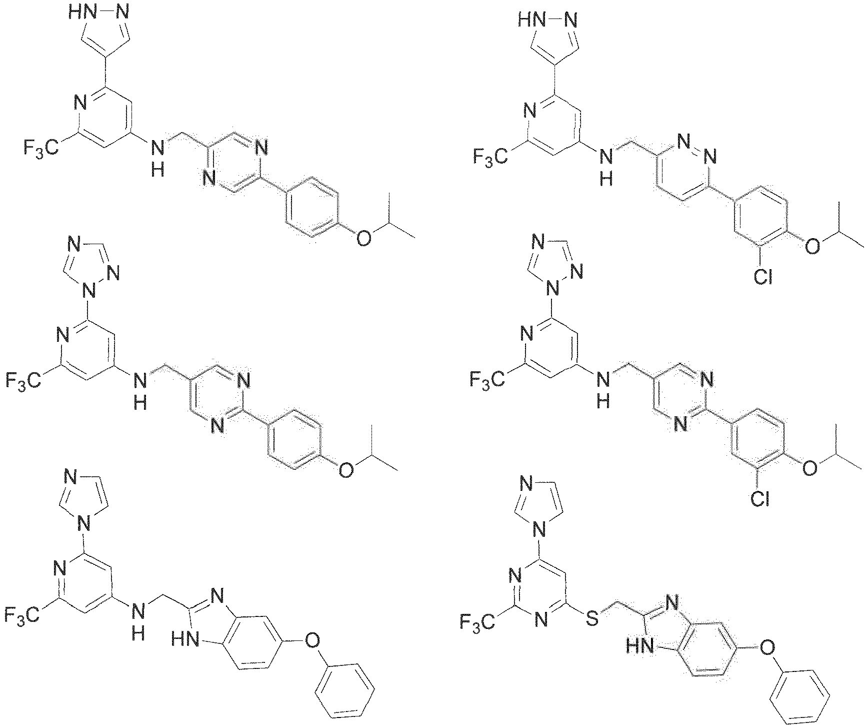

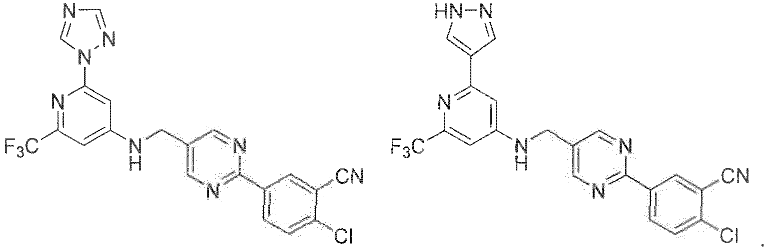

11. A pharmaceutical composition that comprises one or more compound(s) according to any one of claims 1 to 10 and, optionally, at least one carrier substance, excipient and/or adjuvant.

12. A combination preparation comprising at least one compound according to any one of claims 1 to 10, and at least one antibiotic as a further active ingredient.

13. The compound according to any one of claims 1 to 10, the pharmaceutical composition according to claim 11, or the combination preparation of claim 12 for use as a medicament.

14. The compound according to any one of claims 1 to 10, the pharmaceutical composition according to claim 11, or the combination preparation of claim 12 for use in the prophylaxis or treatment of a respiratory condition, including cystic fibrosis (CF), non-cystic-fibrosis bronchiectasis (NCFB), chronic obstructive pulmonary disease (COPD) and primary ciliary dyskinesia.

15. A coating for medicinal devices containing at least one compound according to any one of claims 1 to 10.

## Patentansprüche

1. Verbindung der Formel (I): 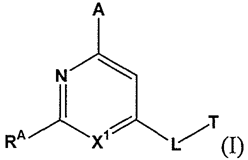 oder ein pharmakologisch akzeptables Salz davon,
wobei
A eine 5- oder 6-gliedrige heteroaromatische Gruppe darstellt, die mindestens ein Stickstoffatom enthält, das optional durch eine oder mehrere, identische oder verschiedene Gruppen, ausgewählt aus einer (C₁-C₃)-Alkylgruppe, CN, einem Halogenatom und OH, substituiert sein kann
X¹ CH oder N darstellt;
R^{A} CF₃, Cl oder CN darstellt;
L eine Gruppe, ist die durch die Formel (L-1) oder (L-2) dargestellt wird: 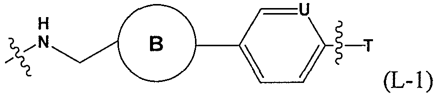
wobei
Ring B 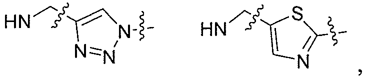 oder ein 6-gliedriger heterocyclischer Ring ist, dargestellt durch die Formel (B-1): 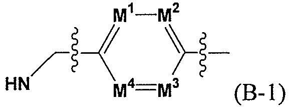
wobei
M¹, M², M³ und M⁴ jeweils unabhängig voneinander CH oder N darstellen; wobei mindestens eines von M¹, M², M³ und M⁴N ist;
wobei der Ring B der zwischen den Wellenlinien gezeigte Ring ist und die Verbindung zu den daran gebundenen Gruppen wie oben gezeigt ist;
U N oder C(R^{U}) darstellt;
R^{U}ein Wasserstoffatom, ein Halogenatom, NR^{U1}R^{U2}, OH, CN, CF₃, CH₂-OH, OCH₃ oder OCF₃ darstellt;
R^{U1} und R^{U2} jeweils unabhängig voneinander ein Wasserstoffatom; oder eine Alkyl-, Alkenyl-, Alkinyl-, Heteroalkyl-, Cycloalkyl-, Heterocycloalkyl-, Alkylcycloalkyl-, Heteroalkylcycloalkyl-, Aryl-, Heteroaryl-, Aralkyl- oder Heteroaralkylgruppe darstellen, wobei alle diese Gruppen optional durch eine oder mehrere, identische oder verschiedene Gruppen, ausgewählt aus einem Halogenatom, OH, =O und NH₂, substituiert sein können; 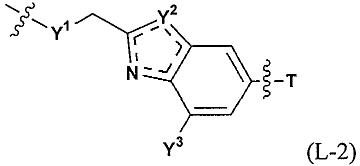
wobei
Y¹ NH oder S ist;
Y² N, NH, O oder S ist;
Y³ ein Wasserstoffatom, F, Cl, OH, CN, (C₁-C₃) -Alkyl, CF₃, CH₂-OH, OCH₃, oder OCF₃ darstellt;
jedes "-----", unabhängig voneinander, eine Einfachbindung oder eine Doppelbindung darstellt, wobei mindestens ein " 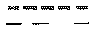 " im Ring der Formel (L-2) eine Doppelbindung ist;
T R oder eine Gruppe -Z-R' ist, wobei
R ein Wasserstoffatom, ein Halogenatom, CN, CF₃, CH₂-OH; NR^{T1} R^{T2}; oder eine Akyl-, Alkenyl, Alkinyl-, Heteroalkyl-, Cycloalkyl-, Heterocycloalkyl-, Alkylcycloalkyl-, Heteroalkylcycloalkyl, Aryl-, Heteroaryl-, Aralkyl- oder Heteroaralkylgruppe darstellt, wobei alle diese Gruppen optional substituiert sein können;
R^{T1} und R^{T2} jeweils unabhängig voneinander ein Wasserstoffatom oder eine (C₁-C₃) -Alkylgruppe darstellen, die durch eine oder mehrere, identische oder verschiedene Gruppe(n), ausgewählt aus einem Halogenatom, OH, =O und NH₂, substituiert sein kann;
Z NH, -O- oder -S- ist; und
R' ein Wasserstoffatom; oder eine Akyl-, Alkenyl-, Alkinyl-, Heteroalkyl-, Cycloalkyl-, Heterocycloalkyl-, Alkylcycloalkyl-, Heteroalkylcycloalkyl-, Aryl-, Heteroaryl-, Aralkyl- oder Heteroaralkylgruppe ist, wobei alle diese Gruppen optional substituiert sein können.

2. Verbindung nach Anspruch 1 oder ein pharmakologisch akzeptables Salz davon, wobei A eine Gruppe ist, die aus den Gruppen ausgewählt ist:

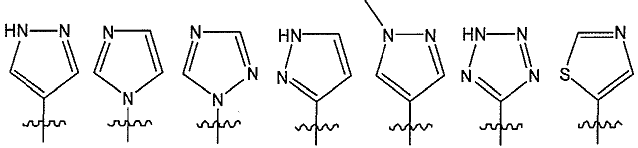

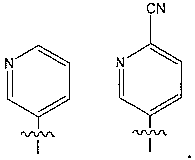

3. Verbindung nach Anspruch 1 oder 2 oder ein pharmakologisch akzeptables Salz davon, wobei L eine Gruppe der Formel (L-1) ist; und
wobei der Ring B 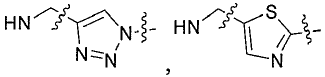 oder
ein 6-gliedriger heterozyklischer Ring der Formel (B-1) ist, der ausgewählt ist aus den Gruppen:

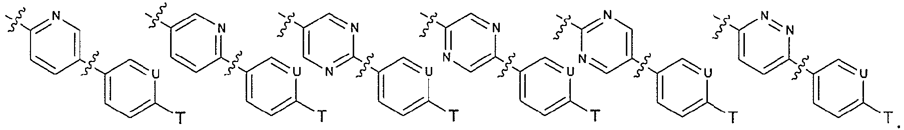

4. Verbindung nach einem der Ansprüche 1 bis 3 oder ein pharmakologisch akzeptables Salz davon, wobei R^{U} ein Wasserstoffatom, Cl, F, CN oder eine aus den Gruppen ausgewählte Gruppe darstellt:

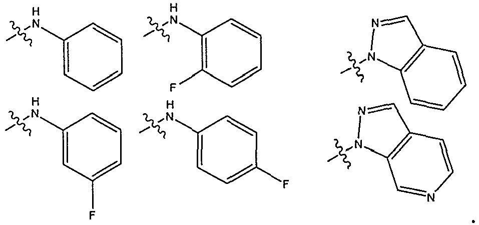

5. Verbindung nach einem der Ansprüche 1 bis 4 oder ein pharmakologisch akzeptables Salz davon, wobei die durch Formel (L-1) dargestellte Gruppe eine der folgenden Gruppen als Teilstruktur enthält:

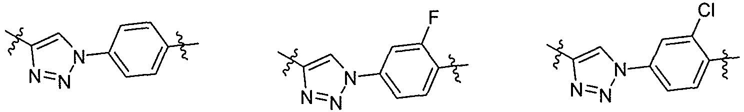

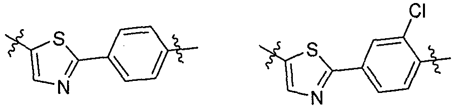

6. Verbindung nach einem der Ansprüche 1 bis 4 oder ein pharmakologisch akzeptables Salz davon, wobei die durch die Formel (L-1) dargestellte Gruppe eine der folgenden Gruppen als Teilstruktur enthält:

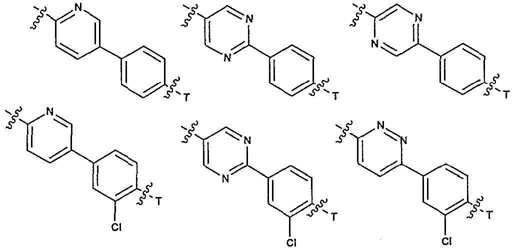

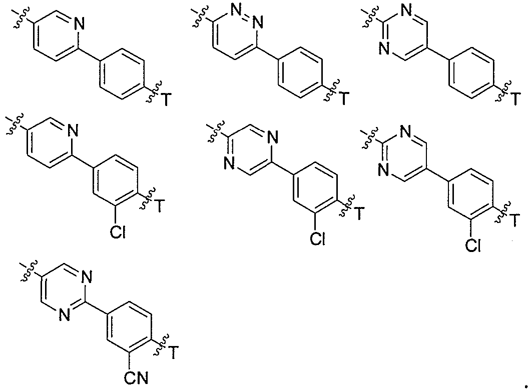

7. Verbindung nach Anspruch 1 oder 2 oder ein pharmakologisch akzeptables Salz davon,
wobei L eine Gruppe der Formel (L-2) ist; und
wobei die durch die Formel (L-2) dargestellte Gruppe eine Gruppe ist, die ausgewählt ist aus:

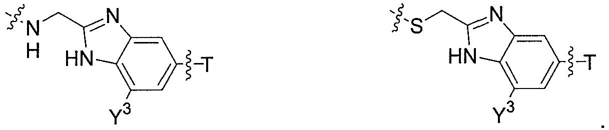

8. Verbindung nach einem der Ansprüche 1 bis 7 oder ein pharmakologisch akzeptables Salz davon, wobei T ein Wasserstoffatom, Cl, F oder eine Gruppe ist, die aus folgenden Gruppen ausgewählt ist:

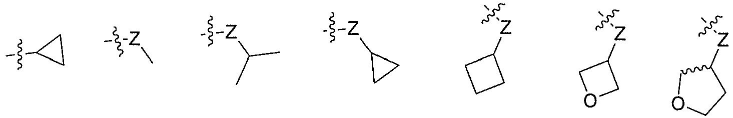

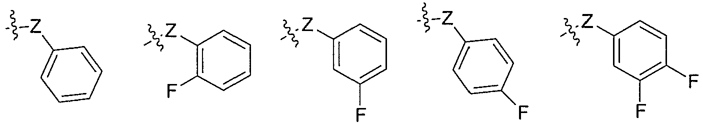

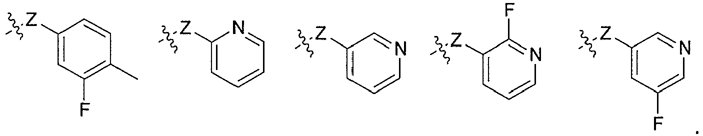

9. Verbindung nach Anspruch 8 oder ein pharmakologisch akzeptables Salz davon, wobei Z NH oder O ist.

10. Verbindung nach einem der Ansprüche 1 bis 9 oder ein pharmakologisch akzeptables Salz davon, wobei die Verbindung ausgewählt ist aus der Gruppe bestehend aus:

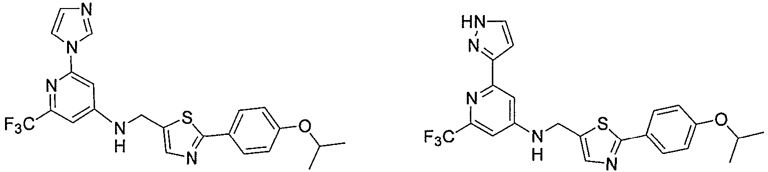

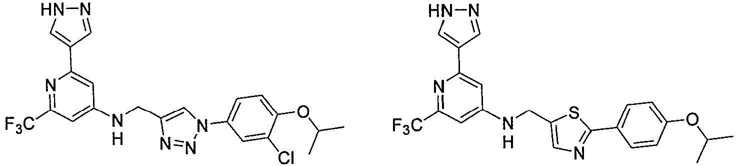

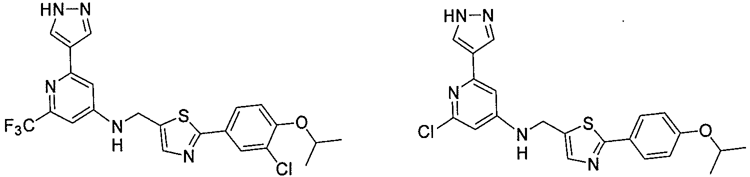

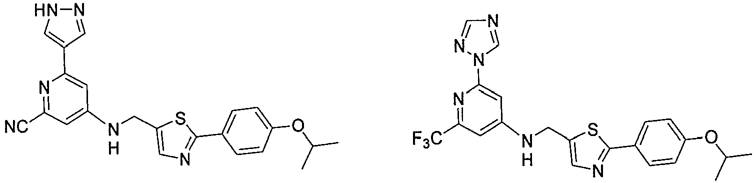

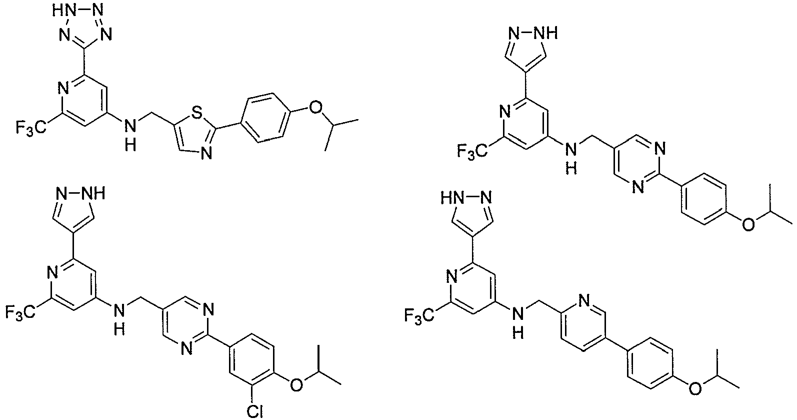

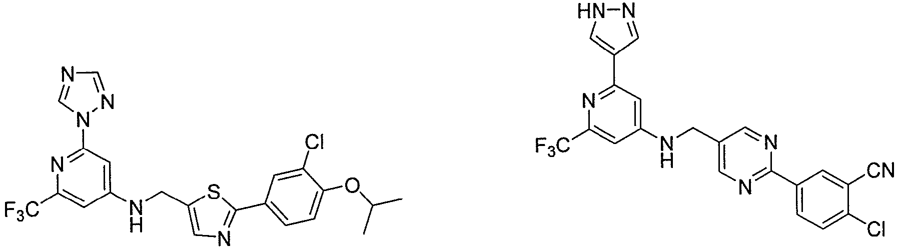

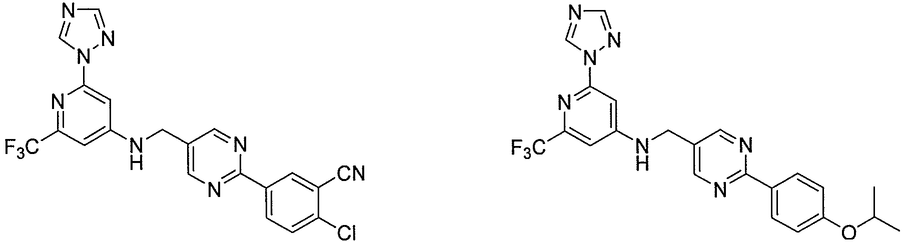

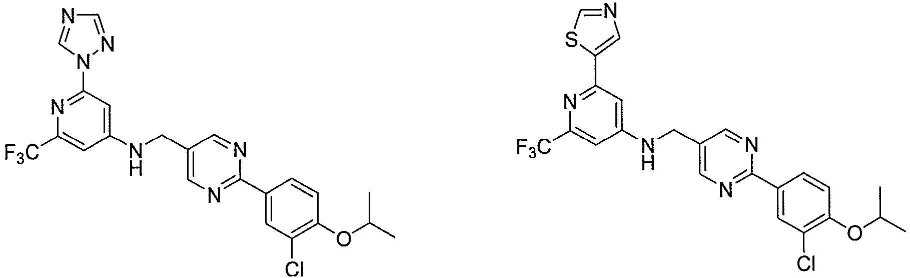

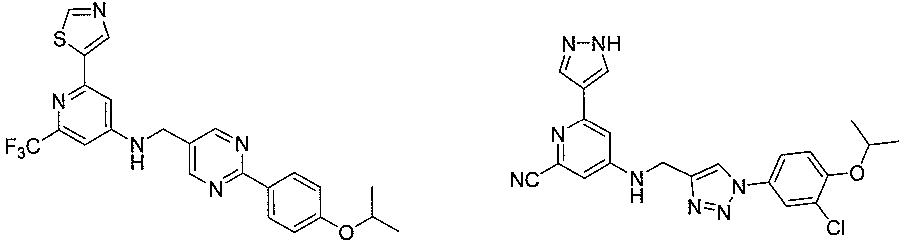

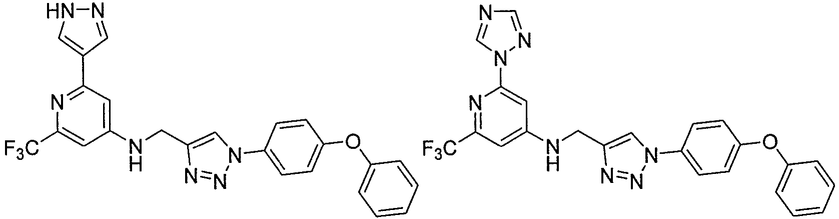

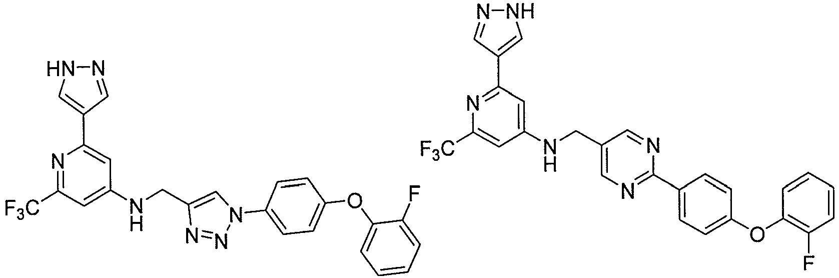

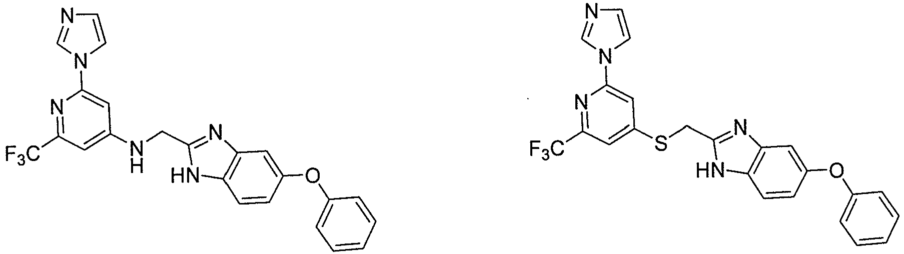

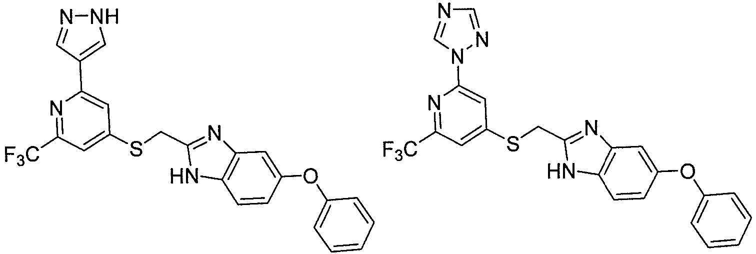

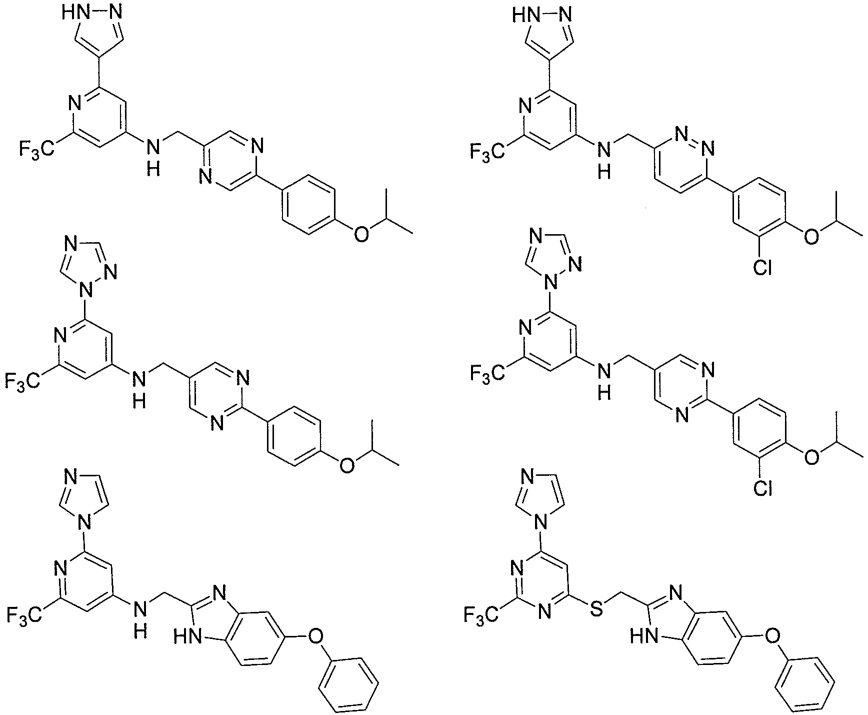

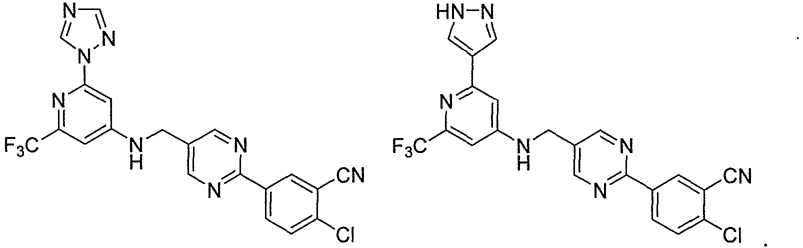

11. Pharmazeutische Zusammensetzung, die eine oder mehrere Verbindung(en) nach einem der Ansprüche 1 bis 10 und gegebenenfalls mindestens eine Trägersubstanz, einen Hilfsstoff und/oder ein Adjuvans enthält.

12. Kombinationspräparat, umfassend mindestens eine Verbindung nach einem der Ansprüche 1 bis 10 und mindestens ein Antibiotikum als weiteren Wirkstoff.

13. Verbindung nach einem der Ansprüche 1 bis 10, die pharmazeutische Zusammensetzung nach Anspruch 11 oder das Kombinationspräparat nach Anspruch 12 zur Verwendung als Arzneimittel.

14. Verbindung nach einem der Ansprüche 1 bis 10, die pharmazeutische Zusammensetzung nach Anspruch 11 oder das Kombinationspräparat nach Anspruch 12 zur Verwendung bei der Prophylaxe oder Behandlung einer Atemwegserkrankung, einschließlich zystischer Fibrose (CF), nicht-zystischer Fibrose-Bronchiektasie (NCFB), chronisch obstruktiver Lungenerkrankung (COPD) und primärer ziliärer Dyskinesie.

15. Beschichtung für medizinische Vorrichtungen, die mindestens eine Verbindung nach einem der Ansprüche 1 bis 10 enthalten.

## Revendications

1. Composé de la formule (I) : 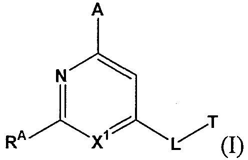 ou un sel pharmacologiquement acceptable de celui-ci,
dans laquelle
A représente un groupe hétéroaromatique à 5 ou 6 chaînons contenant au moins un atome d'azote, qui peut éventuellement être substitué par un ou plusieurs groupes, identiques ou différents, choisis parmi un groupe alkyle (C₁-C₃), CN, un atome d'halogène et OH ;
X¹ représente CH ou N ;
R^{A} représente CF₃, Cl ou CN ;
L est un groupe représenté par la formule (L-1) ou (L-2) : 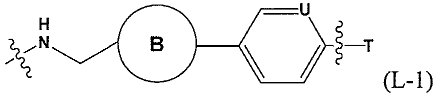
dans laquelle
l'anneau B est 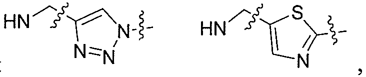 ou un anneau hétérocyclique à 6 membres représenté par la formule (B-1) : 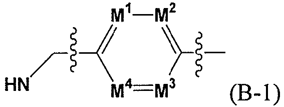
dans laquelle
M¹, M², M³ et M⁴ représentent chacun, indépendamment l'un de l'autre, CH ou N ; au moins un des M¹, M², M³ et M⁴ étant N ;
dans lequel l'anneau B est l'anneau représenté entre les lignes ondulées, et la connectivité avec les groupes qui y sont attachés est telle qu'indiquée ci-dessus;
U représente N ou C(R^{U}) ;
R^{U} représente un atome d'hydrogène, un atome d'halogène, NR^{U1} R^{U2}, OH, CN, CF₃, CH₂-OH, OCH₃, ou OCF₃;
R^{U1} et R^{U2} représentent chacun, indépendamment l'un de l'autre, un atome d'hydrogène ;
ou un groupe akyl, alkenyl, alkynyl, heteroalkyl, cycloalkyl, heterocycloalkyl, alkylcycloalkyl, heteroalkylcycloalkyl, aryl, heteroaryl, aralkyl ou heteroaralkyl, tous ces groupes pouvant éventuellement être substitués par un ou plusieurs groupes, identiques ou différents, choisis parmi un atome d'halogène, OH, =O, et NH₂; 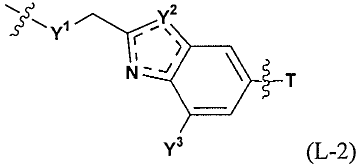
dans lequel
Y¹ est NH ou S ;
Y² est N, NH, O ou S ;
Y³ représente un atome d'hydrogène, F, Cl, OH, CN, (C₁-C₃) alkyle, CF₃, CH₂-OH, OCH₃, ou OCF₃;
chaque " 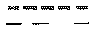 ", indépendamment l'un de l'autre, représente une liaison simple ou une liaison double, où au moins un " 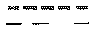 " dans l'anneau de formule (L-2) est une liaison double;
T est R ou un groupe : -Z-R', dans lequel
R représente un atome d'hydrogène, un atome d'halogène, CN, CF₃, CH₂-OH ; NR^{T1}R^{T2}; ou un groupe alkyle, alcényle, alcynyle, hétéroalkyle, cycloalkyle, hétérocycloalkyle, alkylcycloalkyle, hétérocycloalkyle, aryle, hétéroaryle, aralkyle ou hétéroaralkyle, tous ces groupes pouvant éventuellement être substitués;
R^{T1} et R^{T2} représentent chacun, indépendamment l'un de l'autre, un atome d'hydrogène ou un groupe alkyle (C₁-C₃), qui peut être substitué par un ou plusieurs groupes, identiques ou différents, choisis parmi un atome d'halogène, OH, =O et NH₂;
Z est NH, -O- ou -S- ; et
R' est un atome d'hydrogène ou un groupe alkyle, alcényle, alcynyle, hétéroalkyle, cycloalkyle, hétérocycloalkyle, alkylcycloalkyle, hétéroalkylcycloalkyle, aryle, hétéroaryle, aralkyle ou hétéroaralkyle, tous ces groupes pouvant éventuellement être substitués.

2. Composé selon la revendication 1, ou un de ses sels pharmacologiquement acceptables, dans lequel A est un groupe choisi parmi les groupes :

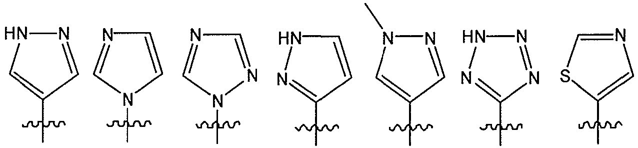

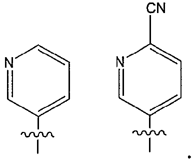

3. Composé selon la revendication 1 ou 2, ou un de ses sels pharmacologiquement acceptables, dans lequel L est un groupe de formule (L-1) ; et
dans lequel le cycle B est 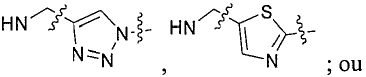
un anneau hétérocyclique à 6 membres représenté par la formule (B-1), qui est choisi parmi les groupes :

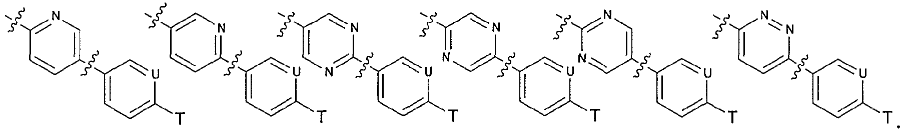

4. Composé selon l'une des revendications 1 à 3, ou son sel pharmacologiquement acceptable, dans lequel R^{U}représente un atome d'hydrogène, Cl, F, CN ; ou un groupe choisi parmi les groupes :

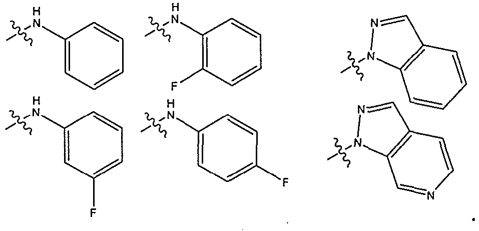

5. Composé selon l'une des revendications 1 à 4, ou son sel pharmacologiquement acceptable, dans lequel le groupe représenté par la formule (L-1) contient l'un des groupes suivants en tant que structure partielle :

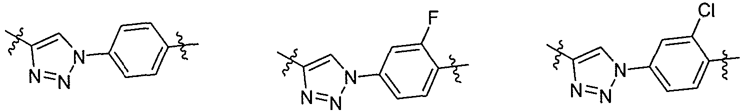

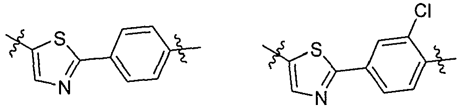

6. Composé selon l'une des revendications 1 à 4, ou son sel pharmacologiquement acceptable, dans lequel le groupe représenté par la formule (L-1) contient l'un des groupes suivants en tant que structure partielle :

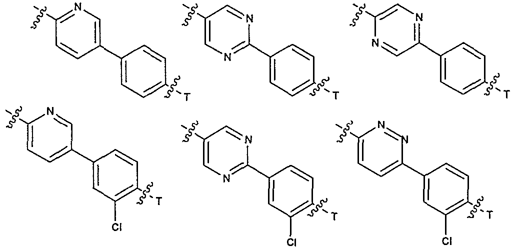

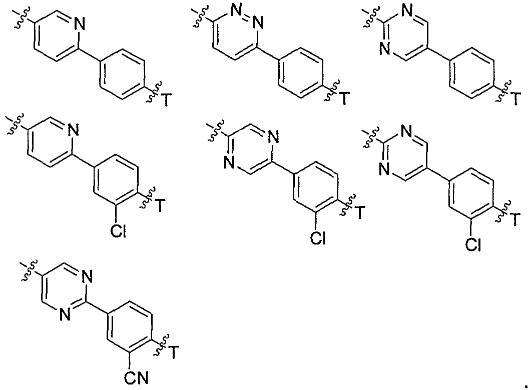

7. Composé selon la revendication 1 ou 2, ou l'un de ses sels pharmacologiquement acceptables,
dans lequel L est un groupe de formule (L-2) ; et
dans lequel le groupe représenté par la formule (L-2) est un groupe choisi parmi :

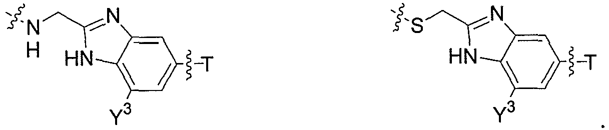

8. Composé selon l'une des revendications 1 à 7, ou un de ses sels pharmacologiquement acceptables, dans lequel T est un atome d'hydrogène, Cl, F ; ou un groupe choisi parmi les groupes :

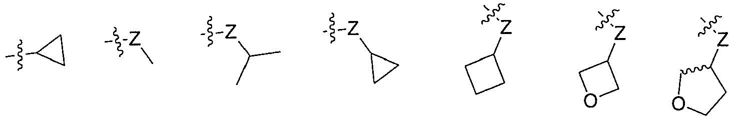

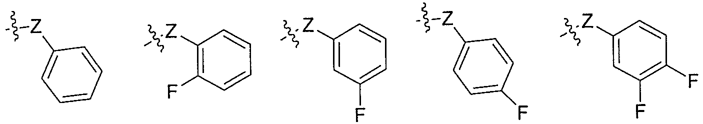

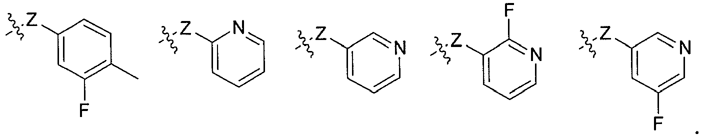

9. Composé selon la revendication 8, ou l'un de ses sels pharmacologiquement acceptables, dans lequel Z est NH ou O.

10. Composé selon l'une des revendications 1 à 9, ou un de ses sels pharmacologiquement acceptables, dans lequel le composé est choisi dans le groupe consistant en :

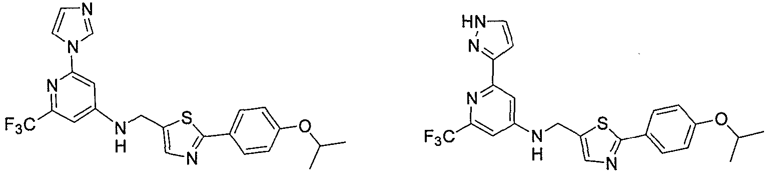

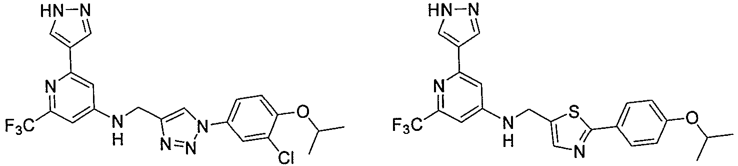

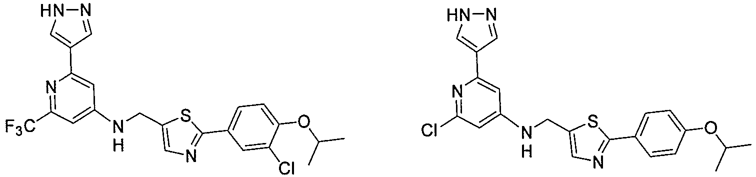

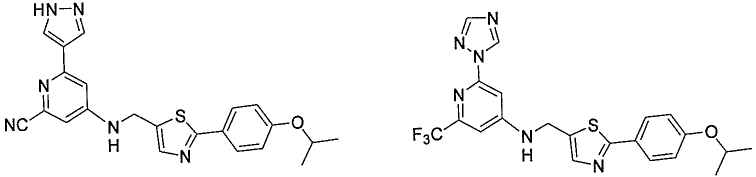

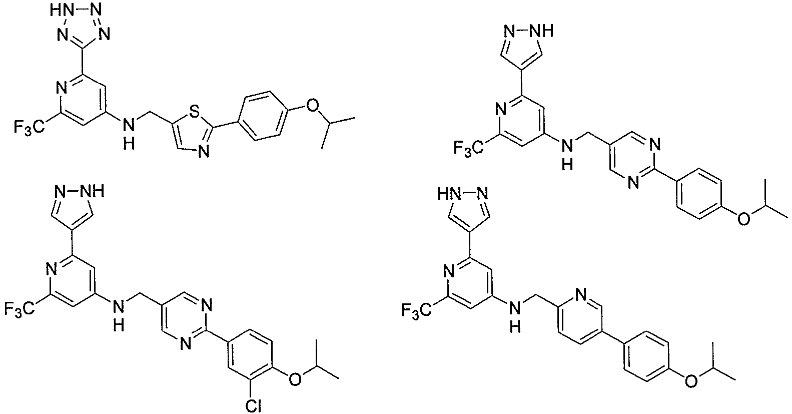

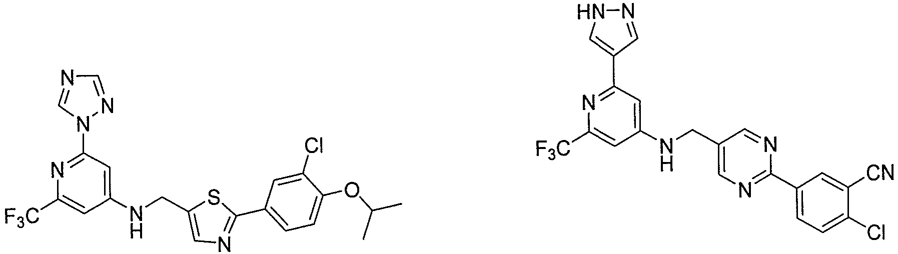

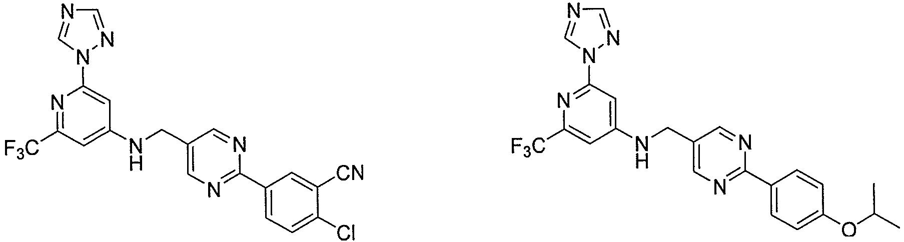

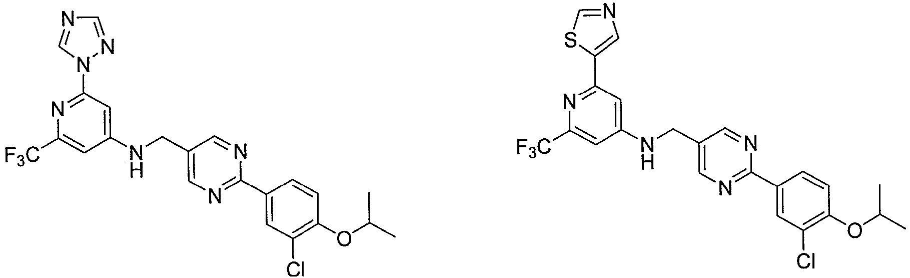

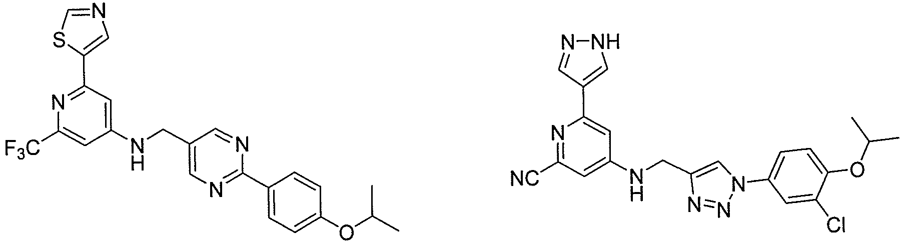

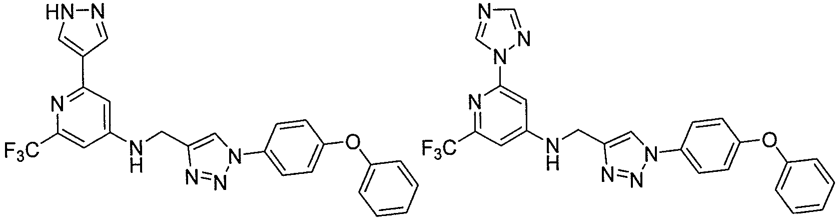

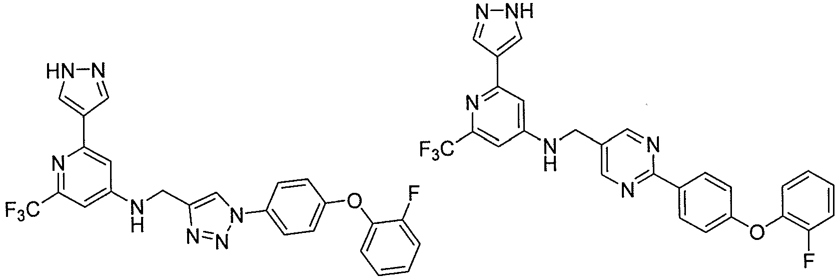

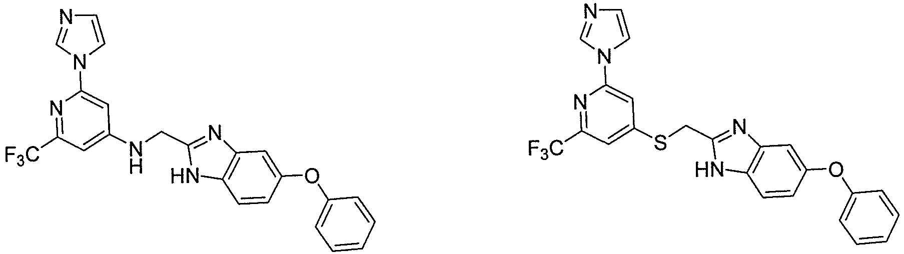

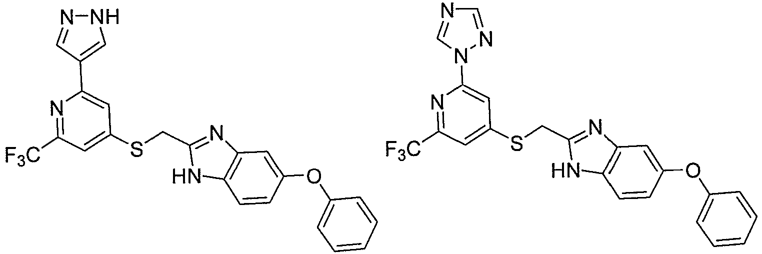

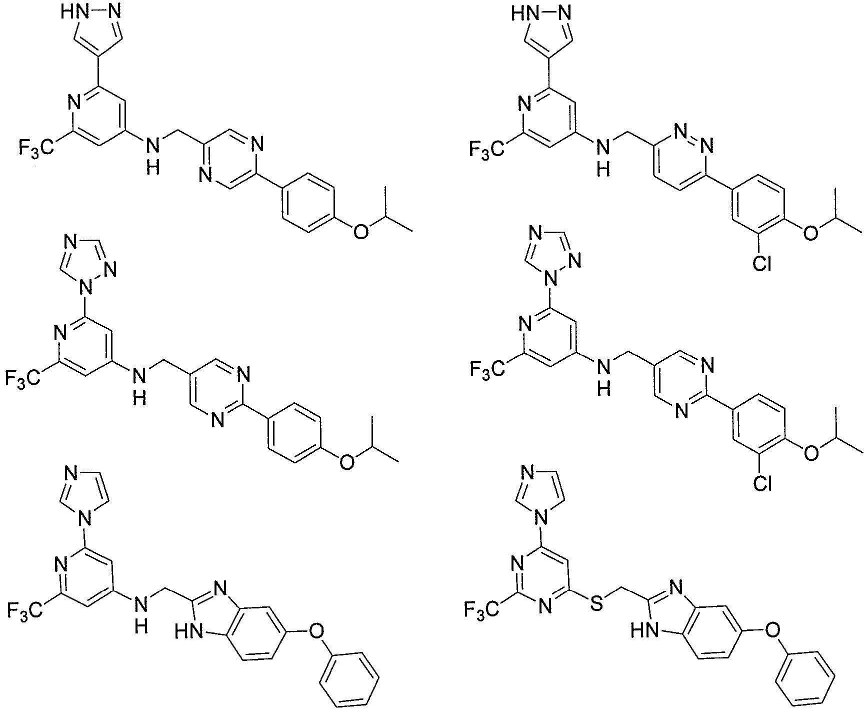

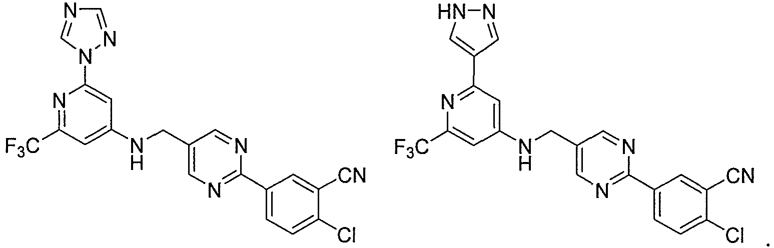

11. Composition pharmaceutique comprenant un ou plusieurs composés selon l'une des revendications 1 à 10 et, éventuellement, au moins une substance porteuse, un excipient et/ou un adjuvant.

12. Préparation combinée comprenant au moins un composé selon l'une des revendications 1 à 10, et au moins un antibiotique en tant qu'ingrédient actif supplémentaire.

13. Composé selon l'une des revendications 1 à 10, la composition pharmaceutique selon la revendication 11, ou la préparation combinée de la revendication 12 pour utilisation comme médicament.

14. Composé selon l'une des revendications 1 à 10, la composition pharmaceutique selon la revendication 11, ou la préparation combinée de la revendication 12 pour l'utilisation dans la prophylaxie ou le traitement d'une condition respiratoire, y compris la fibrose kystique (CF), la bronchiectasie non-cystic fibrosis (NCFB), la maladie pulmonaire obstructive chronique (MPOC) et la dyskinésie ciliaire primaire.

15. Revêtement pour dispositifs médicaux contenant au moins un composé selon l'une des revendications 1 à 10.
